# EUROPEAN PATENT SPECIFICATION

(11) **EP 3 928 100 B1**
(45) Date of publication and mention of the grant of the patent: **22.03.2023**
(21) Application number: 20708581.2
(22) Date of filing: 21.02.2020
(51) Int. Cl.: G01N 33/68

(54) **TREATMENT STRATIFICATION FOR AN EXACERBATION OF INFLAMMATION**
BEHANDLUNGSSTRATIFIKATION FÜR EINE VERSCHLIMMERUNG EINER ENTZÜNDUNG
STRATIFICATION DE TRAITEMENT POUR UNE EXACERBATION D'INFLAMMATION

(30) Priority: 22.02.2019 GB 201902458
(43) Date of publication of application: 29.12.2021
(73) Proprietor: Mologic Limited, Thurleigh, Bedfordshire MK44 2YP (GB)
(72) Inventor: DAVIS, Paul, Thurleigh Bedfordshire MK44 2YP (GB); PAREKH, Gita, Thurleigh Bedfordshire MK44 2YP (GB); DUVOIX, Annelyse, Thurleigh Bedfordshire MK44 2YP (GB)
(74) Representative: Boult Wade Tennant LLP
(86) International application number: PCT/GB2020/050418
(87) International publication number: WO 2020/169987

(56) References cited:
- EP-B1- 2 647 995
- WO-A1-2015/112848
- WO-A1-2015/128681
- WO-A1-2016/185385
- BARTOLOME R. CELLI ET AL: "Inflammatory Biomarkers Improve Clinical Prediction of Mortality in Chronic Obstructive Pulmonary Disease", AMERICAN JOURNAL OF RESPIRATORY AND CRITICAL CARE MEDICINE., vol. 185, no. 10, 15 May 2012 (2012-05-15) , pages 1065-1072, XP055686241, US ISSN: 1073-449X, DOI: 10.1164/rccm.201110-1792OC
- MONA BAFADHEL ET AL: "Acute Exacerbations of Chronic Obstructive Pulmonary Disease", AMERICAN JOURNAL OF RESPIRATORY AND CRITICAL CARE MEDICINE, vol. 184, no. 6, 15 September 2011 (2011-09-15), pages 662-671, XP055205020, ISSN: 1073-449X, DOI: 10.1164/rccm.201104-0597OC

## Description

### FIELD OF THE INVENTION

The present invention relates to the identification of markers in a blood sample that correlate with eosinophil and/or neutrophil levels/activity. Thus, the markers can be used to select the most appropriate treatment for a patient suffering from an exacerbation of inflammation, more specifically pulmonary exacerbations, based upon measuring the levels of said markers.

### BACKGROUND TO THE INVENTION

There are a number of different disorders of the respiratory tract, many of which have an inflammatory component. Examples included chronic obstructive pulmonary disease (COPD), cystic fibrosis (CF) and asthma.

The chronic infection and inflammation of lung disease can cause a progressive decline of lung function resulting in daily symptoms such as cough and sputum production. There are also intermittent episodes of an acute worsening of symptoms from their usual stable state, which is beyond normal day-to-day variations, and which requires additional treatment (Rodriguez-Roisin R., Chest, 2000, 117(5 Suppl 2):398S-401S). These are referred to as pulmonary exacerbations. Pulmonary exacerbations (PEx) are a major cause of morbidity, mortality and hospital admission.

Using COPD as an illustrative example, a recognized criterion used to classify PEx according to symptoms is the Anthonisen standard (Anthonisen et al., Ann Intern Med, 1987, 106:196-204). Anthonisen *et al.* divided exacerbations into three types: Type 1 exacerbations involve increased dyspnea, sputum volume, and sputum purulence; Type 2 involve any two of the latter symptoms; and Type 3 just involves one of those symptoms combined with cough, wheeze, or symptoms of an upper respiratory tract infection. It has been shown that a single exacerbation (the first) may result in significant increase in the rate of decline in lung function.

Thus, whilst there are a number of ways in which a PEx can be identified in practice by a clinician based on the observed symptoms, common to all of these is the fact that a pulmonary exacerbation is considered to be an acute worsening of symptoms in the context of the overall disease. It is thus accepted in the art that a PEx is an episode distinct from the progressive decline of lung function caused by chronic infection and inflammation of lung disease. When a PEx occurs, the clinician will typically intervene therapeutically in order to combat the acute worsening of symptoms. Generally, the clinician can recommend administration of antibiotics and/or corticosteroids. However, often the clinician will not know the underlying cause of the PEx. As a consequence, current guidelines advocate the use of both oral corticosteroids and antibiotics in combination to treat a PEx in a "shotgun-approach" to treating the acute worsening of symptoms.

WO 2015/128681 A1 describes methods for monitoring inflammation status of a subject comprising determining levels of at least one neutrophil activation marker, or at least three markers, in urine samples taken from the subject at multiple time points.

WO 2015/112848 A1 describes methods of identifying, predicting and treating subjects at risk for exacerbation or the presence of a respiratory disease, by detecting expression levels of one or more proteins associated with the respiratory disease.

WO 2016/185385 A1 describes compositions and methods for diagnosing acute exacerbations of chronic obstructive pulmonary disease.

EP 2647995 B1 describes methods for assessing the severity of chronic obstructive pulmonary disease.

### DESCRIPTION OF THE INVENTION

The clinical response to treatment varies considerably and is associated with significant side effects; the inability to target therapy means some patients are inappropriately treated placing a vulnerable population at further risk i.e. the elderly population. The benefit of antibiotics in mild to moderate PEx remains controversial and their overuse can contribute to the development of bacterial resistance. Systemic corticosteroids bear the risk of adverse side effects (hyperglycaemia, increased risk of diabetes and cardiovascular disease), especially in patients with co-morbidities. Furthermore, in some patients oral corticosteroid therapy is associated with increased treatment failures (defined as retreatment, hospitalisation, or death within 30 days). This has led to strategies to reduce the duration of oral corticosteroid treatment. A recent review concluded that current guidelines are of little help in identifying which PEx patients might benefit from treatment with corticosteroids and antibiotics in a primary care setting.

The present inventors have now developed a solution to this problem. More specifically, the inventors have discovered markers in the blood the levels of which correlate with eosinophil levels and/or neutrophil levels. Measuring the levels of these markers can indicate whether the patient has high levels of eosinophils and/or neutrophils in the blood as a consequence of experiencing a pulmonary exacerbation. Moreover, instead of enumerating eosinophils or neutrophils in body fluid samples, the invention involves the determination of marker levels. Eosinophil counts would only provide information on the numbers of eosinophil cells, regardless of whether the cells were active and degranulating or in a pre-activation state. The analysis of biomarkers according to the invention provides information on eosinophil activity. Without wishing to be bound by theory, blood biomarker levels provide an insight into the activity of those eosinophils that have infiltrated into the lung tissues, because the biomarker molecules such eosinophils produce locally may spill over into the blood. Importantly, the inventors have realised that, by measuring the levels of at least one marker of eosinophil levels and at least one marker of neutrophil levels in combination, treatment with corticosteroids (due to high eosinophil levels), antibiotics (due to high neutrophil levels) or both (due to high eosinophil levels and high neutrophil levels) can be appropriately determined. Importantly, when the levels of eosinophil are high and the levels of neutrophil are low, treatment with corticosteroids is selected, and preferably treatment with antibiotics is not selected. Similarly, when the levels of neutrophils are high and the levels of eosinophils are low, treatment with antibiotics is selected, and preferably treatment with corticosteroids is not selected.

The present invention is defined in the appended claims.

The invention provides a method for selecting a treatment (typically the initial treatment in response to the onset of a PEx) to be administered to a patient suffering from an exacerbation of inflammation, the method comprising determining the levels of at least 3 markers of eosinophil levels and at least 3 markers of neutrophil levels in a blood sample taken from the patient suffering from an exacerbation of inflammation wherein:
(i) perturbed levels of the at least 3 markers of eosinophil levels and no perturbation in the levels of the at least 3 markers of neutrophil levels result in selection of corticosteroids to be administered as the treatment for the exacerbation of inflammation;
(ii) perturbed levels of the at least 3 markers of neutrophil levels and no perturbation in the levels of the at least 3 markers of eosinophil levels result in selection of antibiotics to be administered as the treatment for the exacerbation of inflammation ; or
(iii) perturbed levels of the at least 3 markers of eosinophil levels and the at least 3 markers of neutrophil levels result in selection of corticosteroids and antibiotics to be co-administered as the treatment for the exacerbation of inflammation
wherein determining the levels of the at least 3 markers of eosinophil levels comprises determining the levels of at least Eosinophil-derived neurotoxin (EDN), Myeloperoxidase (MPO) and Eosinophil cationic protein (RNASE3);
and wherein determining the levels of the at least 3 markers of neutrophil levels comprises determining the levels of at least (i) Matrix metallopeptidase 9 (MMP9) and Eosinophil-derived neurotoxin (EDN); and (ii) at least one of leukotriene B4 (LTB4), C-reactive protein (CRP), Soluble urokinase-type plasminogen activator receptor (SuPAR), and/or Alpha-1-antitrypsin (A1AT), preferably LTB4.

The method is preferably implemented in a system or test kit for the primary care setting (i.e. to be used by the clinician or nurse).

Accordingly, the invention also provides a system or test kit for selecting a treatment to be administered to a patient suffering from an exacerbation of inflammation, comprising:
a. one or more testing devices for determining the levels of at least 3 markers of eosinophil levels and at least 3 markers of neutrophil levels in a blood sample taken from the patient suffering from an exacerbation of inflammation;
b. a processor; and
c. a storage medium comprising a computer application that, when executed by the processor, is configured to:
   i. Access and/or calculate the determined levels of the at least 3 markers of eosinophil levels and the at least 3 markers of neutrophil levels in a blood sample on the one or more testing devices;
   ii. Calculate whether there is a perturbed level of the at least one marker of eosinophil levels and the at least 3 markers of neutrophil levels in the blood sample; and
   iii. Output from the processor the treatment to be administered to the patient suffering from an exacerbation of inflammation, wherein:
      perturbed levels of the at least 3 markers of eosinophil levels and no perturbation in the levels of the at least 3 markers of neutrophil levels result in selection of corticosteroids to be administered as the treatment for the exacerbation of inflammation; or
      perturbed levels of the at least 3 markers of neutrophil levels and no perturbation in the levels of the at least 3 markers of eosinophil levels result in selection of antibiotics to be administered as the treatment for the exacerbation of inflammation; or
      perturbed levels of the at least 3 markers of eosinophil levels and the at least 3 markers of neutrophil levels result in selection of corticosteroids and antibiotics to be co-administered as the treatment for the exacerbation of inflammation
      wherein the at least 3 markers of eosinophil levels comprise at least EDN, MPO and RNASE3;
   and wherein the at least 3 markers of neutrophil levels comprise at least (i) MMP9 and EDN; and (ii) at least one of leukotriene B4 (LTB4), C-reactive protein (CRP), Soluble urokinase-type plasminogen activator receptor (SuPAR), and/or Alpha-1-antitrypsin (A1AT), preferably LTB4.

The invention also relates to a corresponding computer application for use in the system or test kit.

The exacerbation of inflammation is preferably an exacerbation of lung inflammation. In particular, the exacerbation of inflammation may be a pulmonary exacerbation (PEx). The exacerbation of inflammation (e.g. a PEx) may be diagnosed based on the symptoms the subject presents (e.g. shortness of breath, increased wheeze, increased pulse rate, dyspnoea, increased sputum purulence, increased sputum colour, sore throat, increased cough, cold, fever and/or Forced Expiratory Volume in one second (FEV₁)). For example, using symptoms to diagnose and classify a PEx in COPD patients has been codified in Rodriguez-Roisin R., Chest, 2000, 117(5 Suppl 2):398S-401S and Anthonisen et al., Ann Intern Med, 1987, 106:196-204.

Alternatively, the exacerbation of inflammation may be diagnosed before symptoms begin to present visibly using markers present in urine samples taking from the subject at multiple time points, as described in WO2015/028681.

In preferred embodiments, the exacerbation of inflammation is diagnosed in this way using Headstart^{®} (Mologic). This is advantageous since the exacerbation can thus be treated earlier, limiting the effect and potential consequences of the symptoms that would otherwise develop.

The subject is a mammalian subject, typically a human. Typically, the subject is suffering from a respiratory disorder. More specifically, the respiratory disorder may be chronic obstructive pulmonary disease (COPD). The inventors have accumulated data showing the effectiveness of this approach in this specific disease condition. COPD represents a collection of lung diseases including chronic bronchitis, emphysema and chronic obstructive airways disease and thus the invention is applicable to any of these lung diseases. The invention may also be applicable to monitoring of cystic fibrosis (CF) and asthma.

It should be noted that the invention is performed *in vitro* based upon isolated blood samples, e.g. a provided blood sample. The blood sample may be a serum or plasma sample. The methods of the invention may include steps of obtaining a blood sample for testing. Similarly, the systems and test kits may include suitable vessels for receiving a blood sample. Those vessels may be specifically adapted for blood collection and may be different depending upon the gender of the subject. The container may be coloured to protect any light sensitive analytes.

The phrase "marker of eosinophil levels" means a molecule found in the blood for which levels thereof correlate (positively or negatively or exhibit a more complex pattern depending on the marker) with eosinophil levels in the blood. That is to say, the marker is distinct from eosinophils themselves. Typically, the marker is a protein or peptide. Advantageously, the markers of eosinophil levels described herein can be conveniently detected using, for instance, labelled antibodies as further described herein. Thus, the levels of the at least one marker of eosinophil levels reflect the level of eosinophils in the blood. In this regard, the inventors have determined advantageous combinations of markers that are particularly strong reflectors of the level or activity of eosinophils. This conveniently avoids the need to otherwise determine an eosinophil count which is technically more difficult to do and not well-suited for determination using a point-of-care diagnostic test.

Similarly, the phrase "marker of neutrophil levels" means a molecule found in the blood for which levels thereof correlate (positively or negatively or exhibit a more complex pattern depending on the marker) with neutrophil levels in the blood. That is to say, the marker is distinct from neutrophils themselves. Typically, the marker is a protein or peptide. Advantageously, the markers of neutrophil levels described herein can be conveniently detected using, for instance, labelled antibodies as further described herein. Thus, the levels of the at least one marker of neutrophil levels reflect the level of neutrophils in the blood. In this regard, the inventors have determined advantageous combinations of markers that are particularly strong reflectors of the level or activity of neutrophils. This conveniently avoids the need to otherwise determine a neutrophil count which is technically more difficult to do and not well-suited for determination using a point-of-care diagnostic test.

Whenever reference is made herein to determining the levels of a marker of eosinophil levels, it should be understood that the determination of at least 3 markers of eosinophil levels is required, wherein the markers of eosinophil levels are at least Eosinophil-derived neurotoxin (EDN), Myeloperoxidase (MPO) and Eosinophil cationic protein (RNASE3). The at least 3 markers of eosinophil levels may further include an additional marker selected from: Human neutrophil elastase (HNE), Soluble urokinase-type plasminogen activator receptor (SuPAR), Calprotectin, and/or Major Basic Protein (MBP).

EDN (also known as RNase2; UniProt ID: P10153) is a protein belonging to the ribonuclease A (RNase A) superfamily, which has been found to have antiviral activity *in vitro.* It is produced in the eosinophil granulocytes. It is closely related to the eosinophil cationic protein (RNASE3).

MBP (UniProt ID: P13727) is the predominant constituent of the crystalline core of the eosinophil granule. This protein may be involved in antiparasitic defence mechanisms as a cytotoxin and helminthotoxin, and in immune hypersensitivity reactions. MBP also causes the release of histamine from mast cells and basophils, and activates neutrophils and alveolar macrophages.

RNASE3 (sometimes referred to as RNase3; UniProt ID: P12724) is a protein belonging to the ribonuclease A (RNase A) superfamily. RNASE3 is released during degranulation of eosinophils. This protein is related to inflammation and asthma. It possesses neurotoxic, helmintho-toxic, and ribonucleo-lytic activities and is localised to the granule matrix of the eosinophil.

As demonstrated in the Examples section, the inventors have found that the levels of EDN and MBP each positively correlate with levels of eosinophils in the blood. Thus, increased levels of EDN and/or MBP in a blood sample indicate increased levels of eosinophils and, therefore, that corticosteroids should be administered as the (initial) treatment for the exacerbation of inflammation.

It will be readily apparent that the phrase "at least one" as used throughout this specification means one or more. Thus, it encompasses one, two, three, four, five, six, seven, eight, nine, ten or more, and so on. In the methods of the invention, where the determination of markers of eosinophil levels is required, the determination of at least 3 markers of eosinophil levels is required; and where the determination of markers of neutrophil levels is required, the determination of at least 3 markers of neutrophil levels is required.

Furthermore, "at least 3 markers" as used in the method means three or more. Thus, it encompasses three, four, five, six, seven, eight, nine, ten or more markers, and so on. It will now be readily apparent to the skilled person that the method may comprise determining the levels of four, five, six, seven, eight, nine, ten or more markers of eosinophil levels and/or four, five, six, seven, eight, nine, ten or more markers of neutrophil levels. The number of markers of eosinophil levels and neutrophil levels does not need to be the same. For example, the method may comprise determining the levels of four markers of eosinophil levels and five markers of neutrophil levels.

In any embodiment, the "at least [number] markers" is preferably about or exactly that number of markers. For example, the "at least 3 markers" is preferably about or exactly 3 markers. In any embodiment, the determined markers may comprise or consist of the recited markers.

Preferably, no more than 20, 15, 10, 9, 8, 7, 6 or 5 markers of eosinophil levels and/or no more than 20, 15, 10, 9, 8, 7, 6 or 5 markers of neutrophil levels are determined. The determination of at least, about or exactly 5 markers of eosinophil levels and/or at least, about or exactly 5 markers of neutrophil levels is preferred.

Preferably, the at least 3 markers of eosinophil levels are Eosinophil-derived neurotoxin (EDN), Myeloperoxidase (MPO) and Eosinophil cationic protein (RNASE3); or are selected from Eosinophil-derived neurotoxin (EDN), Myeloperoxidase (MPO), Eosinophil cationic protein (RNASE3), Human neutrophil elastase (HNE), Soluble urokinase-type plasminogen activator receptor (SuPAR), Calprotectin, and/or Major Basic Protein (MBP).

Whenever reference is made herein to determining the levels of a marker of neutrophil levels, it should be understood that the determination of at least 3 markers of neutrophil levels is required, wherein determining the levels of the at least 3 markers of neutrophil levels comprises determining the levels of at least (i) Matrix metallopeptidase 9 (MMP9) and Eosinophil-derived neurotoxin (EDN); and (ii) at least one of leukotriene B4 (LTB4), C-reactive protein (CRP), Soluble urokinase-type plasminogen activator receptor (SuPAR), and/or Alpha-1-antitrypsin (A1AT), preferably LTB4. The at least 3 markers of neutrophil levels may further include an additional marker selected from: Calprotectin, C-reactive protein (CRP), Alpha-1-antitrypsin (A1AT), MBP, myeloperoxidase (MPO), Interleukin-8 (IL-8), Interleukin-6 (IL-6) and Interleukin-1β (IL-1β); or MMP9, EDN, LTB4, CRP, SuPAR and/or A1AT.

Calprotectin is a complex of the mammalian proteins S100A8 (UniProt ID: P05109) and S100A9 (UniProt ID: P06702). In the presence of calcium, calprotectin is capable of sequestering the transition metals manganese and zinc which gives the complex antimicrobial properties. Calprotectin comprises as much as 60% of the soluble protein content of the cytosol of a neutrophil, and it is secreted during inflammation.

CRP (UniProt ID: P02741) is a pentameric protein found in plasma, whose levels rise in response to inflammation. It is an acute-phase protein of hepatic origin that increases following interleukin-6 secretion by macrophages and T cells.

A1AT (UniProt ID: P01009) is a protein belonging to the serpin superfamily. It is encoded in humans by the SERPINA1 gene. A protease inhibitor, it is also known as alpha1-proteinase inhibitor (A1PI) or alpha1-antiproteinase (A1AP) because it inhibits various proteases (not just trypsin). In older biomedical literature it was sometimes called serum trypsin inhibitor (STI, dated terminology), because its capability as a trypsin inhibitor was a salient feature of its early study. As a type of enzyme inhibitor, it protects tissues from enzymes of inflammatory cells, especially neutrophil elastase.

MPO (UniProt ID: P05164) is a peroxidase enzyme that is most abundantly expressed in neutrophil granulocytes, and produces hypochlorous acid that kills bacteria and other pathogens through cytotoxicity.

IL-8 (also known as chemokine (C-X-C motif) ligand 8, CXCL8; UniProt ID: P10145) is a chemokine produced by macrophages and other cell types such as epithelial cells, airway smooth muscle cells and endothelial cells. In humans, the interleukin-8 protein is encoded by the CXCL8 gene. IL-8 is initially produced as a precursor peptide of 99 amino acids which then undergoes cleavage to create several active IL-8 isoforms. In culture, a 72 amino acid peptide is the major form secreted by macrophages.

IL-6 (UniProt ID: P05231) acts as both a pro-inflammatory cytokine and an anti-inflammatory myokine. In humans, it is encoded by the IL6 gene. In addition, osteoblasts secrete IL-6 to stimulate osteoclast formation. Smooth muscle cells in the tunica media of many blood vessels also produce IL-6 as a pro-inflammatory cytokine. IL-6's role as an anti-inflammatory myokine is mediated through its inhibitory effects on TNF-alpha and IL-1, and activation of IL-1ra and IL-10.

IL-1β (also known as leukocytic pyrogen, leukocytic endogenous mediator, mononuclear cell factor, lymphocyte activating factor and other names; UniProt ID: 01584) is a cytokine protein that in humans is encoded by the *IL1B* gene. IL-1β precursor is cleaved by cytosolic caspase 1 (interleukin 1 beta convertase) to form mature IL-1β. This cytokine is produced by activated macrophages as a proprotein, which is proteolytically processed to its active form by caspase 1 (CASP1/ICE). This cytokine is an important mediator of the inflammatory response, and is involved in a variety of cellular activities, including cell proliferation, differentiation, and apoptosis.

As demonstrated in the Examples section, the inventors have found that levels of Calprotectin positively correlate with levels of neutrophils in the blood, whilst levels of MBP negatively correlate with levels of neutrophils in the blood. Thus, increased levels of Calprotectin and/or decreased levels of MBP in a blood sample indicate increased levels of neutrophils and, therefore, that antibiotics should be administered as the (initial) treatment for the exacerbation of inflammation.

The inventors have also found that the levels of CRP positively correlate with neutrophil levels during an exacerbation. Thus, increased levels of CRP in a blood sample taken from a patient suffering from an exacerbation of inflammation indicate increased levels of neutrophils and, therefore, that antibiotics should be administered as the (initial) treatment for the exacerbation of inflammation.

It will be noted that MBP is stated as a marker of eosinophil levels and as a marker of neutrophil levels. It is important to note in this context that the levels of MBP positively correlate with eosinophil levels, whilst the levels of MBP negatively correlate with neutrophil levels. Thus, MBP can be used to distinguish the levels of one cell type from the other.

The at least one marker of neutrophil levels may comprise all of MBP, Calprotectin and A1AT. The at least one marker of neutrophil levels may comprise all of Calprotectin, IL-8, IL-6, CRP, MPO and IL-1β.

The inventors have also identified a further set of markers the levels of which correlate (positively or negatively or exhibit a more complex pattern depending on the marker) with both eosinophil levels and neutrophil levels. Thus, whilst independently these markers cannot distinguish between eosinophil levels and neutrophil levels, the inventors have found that their combination with at least one marker of eosinophil levels and/or at least one marker of neutrophil levels increases the predictive power in relation to correctly identifying an increase in eosinophil levels and/or neutrophil levels in a blood sample (as appropriate depending on the specific marker combination employed). These markers are referred to herein as "supporting markers of eosinophil and neutrophil levels".

Thus, the method for selecting a treatment to be administered to a patient suffering from an exacerbation of inflammation may further comprise at least one supporting marker of eosinophil and neutrophil levels wherein perturbed levels of the at least one supporting marker:
(i) in combination with perturbed levels of the at least one marker of eosinophil levels and no perturbation in the levels of the at least one marker of neutrophil levels result in selection of corticosteroids to be administered as the treatment for the exacerbation of inflammation;
(ii) in combination with perturbed levels of the at least one marker of neutrophil levels and no perturbation in the levels of the at least one marker of eosinophil levels result in selection of antibiotics to be administered as the treatment for the exacerbation of inflammation; or
(iii) in combination with perturbed levels of the at least one marker of eosinophil levels and the at least one marker of neutrophil levels result in selection of corticosteroids and antibiotics to be co-administered as the treatment for the exacerbation of inflammation.

Similarly, the system or test kit for selecting a treatment to be administered to a patient suffering from an exacerbation of inflammation may further comprise determining the levels of at least one supporting marker of eosinophil and neutrophil levels in the sample, wherein:
i. the computer application, when executed by the processor, is configured to access and/or calculate the determined levels of the at least one supporting marker of eosinophil and neutrophil levels in the blood sample on the one or more testing devices;
ii. Calculate whether there is a perturbed level of the at least one supporting marker of eosinophil and neutrophil levels in the blood sample; and
iii. Output from the processor the treatment to be administered to the patient suffering from an exacerbation of inflammation, wherein perturbed levels of the at least one supporting marker:
   (a) in combination with perturbed levels of the at least one marker of eosinophil levels and no perturbation in the levels of the at least one marker of neutrophil levels result in selection of corticosteroids to be administered as the treatment for the exacerbation of inflammation;
   (b) in combination with perturbed levels of the at least one marker of neutrophil levels and no perturbation in the levels of the at least one marker of eosinophil levels result in selection of antibiotics to be administered as the treatment for the exacerbation of inflammation; or
   (c) in combination with perturbed levels of the at least one marker of eosinophil levels and the at least one marker of neutrophil levels result in selection of corticosteroids and antibiotics to be co-administered as the treatment for the exacerbation of inflammation.

The term "supporting marker" is used herein to indicate that the marker should not be analysed on its own. However, , at least 3 markers (which may include supporting markers) are analysed to determine the levels of eosinophils, meaning that the method does not rely on the analysis of a single marker. Accordingly, when combinations of markers are used, the distinction between a "marker" and a "supporting marker" is not required and for simplicity, the term "marker" is applied to all markers, including those otherwise denoted as "supporting markers". Thus, any reference herein to a "supporting marker" should be understood to mean a "marker" when the method requires the analysis of at least 3 markers. In other words, a "supporting" marker is simply a "further" marker and as this is implicit when more than 1 marker is used, the term "supporting" is no longer required.

Typically, the levels of the at least one supporting marker of eosinophil and neutrophil levels are determined using the same one or more testing devices used to determine the levels of the at least one marker of eosinophil levels and the at least one marker of neutrophil levels. However, it is possible that levels of the at least one supporting marker of eosinophil and neutrophil levels are determined using an additional one or more testing devices.

The at least one (supporting) marker of eosinophil and neutrophil levels may be selected from: Matrix metallopeptidase 9 (MMP9), Human neutrophil elastase (HNE), and neutrophil gelatinase-associated lipocalin (NGAL).

MMP9 (UniProt ID: P14780) is a matrixin, a class of enzymes that belong to the zinc-metalloproteinases family involved in the degradation of the extracellular matrix. In humans, MMP9 is expressed as propeptide which is activated when cleaved by extracellular proteinases. MMP9 plays several important functions within neutrophil action, such as degrading extracellular matrix, activation of IL-1β, and cleavage of several chemokines.

HNE (UniProt ID: P08246) is a serine proteinase in the same family as chymotrypsin and has broad substrate specificity. Secreted by neutrophils and macrophages during inflammation, it destroys bacteria and host tissue. It also localizes to Neutrophil extracellular traps (NETs), via its high affinity for DNA, an unusual property for serine proteases.

NGAL (also known as Lipocalin-2; UniProt ID: P80188) is a protein that in humans is encoded by the LCN2 gene. NGAL is involved in innate immunity by sequestring iron that in turn limits bacterial growth. It is expressed in neutrophils and in low levels in the kidney, prostate, and epithelia of the respiratory and alimentary tracts.

As demonstrated in the Examples section, the inventors have found that levels of MMP9 positively correlate with both eosinophil levels and neutrophil levels, whilst levels of HNE negatively correlate with both eosinophil levels and neutrophil levels.

Thus, increased levels of MMP9 and/or decreased levels of HNE can be used, in combination with perturbed levels of at least one neutrophil and eosinophil marker, to indicate increased levels neutrophils and eosinophils respectively.

As shown in Example 5, using a large number of blood samples from patients suffering from an exacerbation, the inventors subsequently confirmed that during an exacerbation, levels of MMP9 positively correlate with neutrophil levels and may be used as a marker of neutrophil levels, particularly when used in combination with at least EDN.

Moreover, as shown in Example 5, using a large number of blood samples from patients suffering from an exacerbation, the inventors subsequently determined that HNE may be used in combination with other markers to determine eosinophil levels.

Thus, preferably the methods and systems and test kits described herein for selecting a treatment to be administered to a patient suffering from an exacerbation of inflammation may comprise determining the levels of at least three markers, in any combination of markers, provided that the at least three markers comprise at least one marker of eosinophil levels and at least one marker of neutrophil levels. Thus, using three markers as an illustrative example, the following combinations are disclosed:
(i) one marker of eosinophil levels, one marker of neutrophil levels and one supporting marker of eosinophil and neutrophil levels;
(ii) one marker of eosinophil levels and two markers of neutrophil levels;
(iii) two markers of eosinophil levels and one marker of neutrophil levels.

Furthermore, "at least three markers" as used in this specification means three or more. Thus, it encompasses three, four, five, six, seven, eight, nine, ten or more markers, and so on. It will now be readily apparent to the skilled person that the four, five, six, seven, eight, nine, ten or more markers, and so on, may be in any combination of markers (i.e. combinations made up of markers of eosinophil levels, markers of neutrophil levels and supporting markers of eosinophil and neutrophil levels), provided that the combination of markers comprises at least one marker of eosinophil levels and at least one marker of neutrophil levels. However, when it is specified that the combination of markers must comprise at least 3 markers of eosinophil levels and at least 3 markers of neutrophil levels, in which case the combination must comprise at least 6 markers, of which at least 3 must be markers of eosinophil levels and at least 3 must be markers of neutrophil levels. Where a marker is a marker of eosinophil levels and a marker of neutrophil levels, for example EDN, it may be included in both sets such that the combination of 6 markers only includes 5 different markers

As described in the Examples section, the inventors have identified marker combinations with particularly good performance in relation to correlation with eosinophil or neutrophil levels. Thus, particular markers that may be included in the marker combinations useful in the invention may comprise:
(i) EDN, MMP9, HNE, NGAL and MBP;
(ii) MMP9, CRP and/or NGAL;
(iii) MMP9, Calprotectin, HNE and CRP; and/or
(iv) A1AT and/or NGAL.

Specific markers that may be included in the marker combinations useful in the invention include:
- EDN, MMP9, HNE, NGAL and MBP
- Calprotectin, MBP, MMP9, CRP and NGAL
- MBP, Calprotectin and A1AT
- MBP, Calprotectin, A1AT, MMP9 and CRP
- MBP, Calprotectin, A1AT, MMP9, CRP and NGAL
- MMP9, Calprotectin, HNE, CRP and A1AT
- MMP9, Calprotectin, HNE and CRP
- MMP9, Calprotectin, HNE, CRP, A1AT and NGAL
- Calprotectin, MMP9, IL-8, IL-6, NGAL, CRP, MPO and IL-1β

In the methods, systems and test kits described above, the levels of at least one marker of eosinophil levels and the levels of at least one marker of neutrophil levels are determined. For example, the at least one marker of eosinophil levels comprises EDN and the at least one marker of neutrophil levels comprises Calprotectin and/or CRP. Thus, increased levels of EDN (as a marker of eosinophil levels) in combination with increased levels of Calprotectin and/or CRP (as markers of neutrophil levels) result in selection of corticosteroids and antibiotics to be co-administered as the treatment for the exacerbation of inflammation.

As demonstrated in the Examples section, through extensive analysis of blood samples from patients suffering from a Px, the inventors have determined that whilst certain individual markers may correlate (positively, negatively, or via a more complex pattern) with eosinophil or neutrophil levels, certain specific combinations are a superior indicator of eosinophil or neutrophil levels during exacerbations. These combinations are a more reliable, sensitive and specific indicator of such levels.

Thus, the inventors have found that the levels of at least 3 markers of eosinophil levels and at least 3 markers of neutrophil levels in a blood sample from the patient suffering from an exacerbation of inflammation of a respiratory condition can advantageously be used to determine the type of treatment that needs to be administered to a patient.

Thus, the invention provides a method for selecting a treatment to be administered to a patient suffering from an exacerbation of inflammation of a respiratory condition, the method comprising determining the levels of at least 3 markers of eosinophil levels and at least 3 markers of neutrophil levels in a blood sample taken from the patient suffering from an exacerbation of inflammation of a respiratory condition wherein:
(i) perturbed levels of the at least 3 markers of eosinophil levels and no perturbation in the levels of the at least 3 markers of neutrophil levels result in selection of corticosteroids to be administered as the treatment for the exacerbation of inflammation;
(ii) perturbed levels of the at least 3 markers of neutrophil levels and no perturbation in the levels of the at least 3 markers of eosinophil levels result in selection of antibiotics to be administered as the treatment for the exacerbation of inflammation; or
(iii) perturbed levels of the at least 3 markers of eosinophil levels and the at least 3 markers of neutrophil levels result in selection of corticosteroids and antibiotics to be co-administered as the treatment for the exacerbation of inflammation
wherein determining the levels of the at least 3 markers of eosinophil levels comprises determining the levels of at least Eosinophil-derived neurotoxin (EDN), Myeloperoxidase (MPO) and Eosinophil cationic protein (RNASE3);
and wherein determining the levels of the at least 3 markers of neutrophil levels comprises determining the levels of at least (i) Matrix metallopeptidase 9 (MMP9) and Eosinophil-derived neurotoxin (EDN); and (ii) at least one of leukotriene B4 (LTB4), C-reactive protein (CRP), Soluble urokinase-type plasminogen activator receptor (SuPAR), and/or Alpha-1-antitrypsin (A1AT), preferably LTB4.

LTB4 is a potent chemoattractant of neutrophils that promotes polymorphonuclear (PMN) cell migration, blocks PMN apoptosis, and induces neutrophil granule release in conjunction with reactive oxygen species generation. LTB4 is produced by the metabolism of arachidonic acid released during inflammatory response. LTB4 activates macrophage phagocytosis and drives mononuclear pro-inflammatory cytokine release.

SuPAR is released from the membrane bound plasminogen activator and is positively correlated with the activation of immune system. SuPAR is expressed by endothelial cells, macrophages, monocytes, neutrophils, lymphocytes and fibroblasts.

Importantly, when the levels of the at least 1, e.g. 3, marker(s) of eosinophil levels is/are perturbed and the levels of at least 1, e.g. 3, marker(s)of neutrophil levels is/are not perturbed, treatment with corticosteroids is selected, , and preferably treatment with antibiotics is not selected. Similarly, when the levels at least 1, e.g. 3, marker(s)of neutrophil levels is/are perturbed and the levels of the at least 1, e.g. 3, marker(s)of eosinophil levels is/are not perturbed, treatment with antibiotics is selected, , and preferably treatment with corticosteroids is not selected.

The method may include a further marker of eosinophil levels such as HNE, SuPAR, and/or Calprotectin.

The method may include a further marker of neutrophil levels such as A1AT, SuPAR, CRP, and/or MBP.

The method may comprise determining the levels of at least, about or exactly 5 markers of eosinophil levels and at least, about or exactly 5 markers of neutrophil levels in the blood sample.

In this method, the markers of eosinophil levels are preferably EDN, RNASE3, SuPAR, HNE and MPO; and the markers of neutrophil levels are preferably MMP9, EDN, LTB4 and A1AT; and either SuPAR or CRP.

As demonstrated in the Examples section, the inventors have found that levels of MMP9, MMP8, MPO, PCT, CRP, HNE, NGAL and Calprotectin positively correlate with neutrophil levels, whilst levels of LTB4 negatively correlate with neutrophil levels. In addition, the inventors have found that levels of EDN, RNASE3, Lactoferrin, IgE and MBP positively correlate with eosinophil levels, whilst levels of CRP, MPO and PCT negatively correlate with eosinophil levels. The negative correlation is determined by AUC values below 0.5, whereas the positive correlation is determined by AUC values above 0.5.

Other markers have a more complex correlation pattern. For example, a marker may have perturbed high levels if eosinophil levels are high, without displaying a linear correlation. The inventors have determined that combinations of markers are more reliable, sensitive and specific indicators of eosinophil or neutrophil levels respectively. Thus, preferably, a combination of markers is analysed.

It is also possible that the markers of eosinophil levels described herein may be used separately from the markers of neutrophil levels in order to determine if corticosteroids would be an appropriate (initial) treatment for an exacerbation of inflammation. This method may whether the exacerbation is eosinophil-driven. Thus, the invention also provides a method for selecting corticosteroids to be administered as a treatment to a patient suffering from an exacerbation of inflammation of a respiratory condition, the method comprising determining the levels of at least 3 markers of eosinophil levels in a blood sample taken from the patient suffering from an exacerbation of inflammation of a respiratory condition wherein perturbed levels of the at least 3 markers of eosinophil levels results in selection of corticosteroids to be administered as the treatment for the exacerbation of inflammation wherein determining the levels of the at least 3 markers of eosinophil levels comprises determining the levels of EDN, MPO and RNASE3; optionally wherein determining the levels of the at least 3 markers of eosinophil levels further comprises determining the levels of HNE, SuPAR, and/or Calprotectin, preferably HNE and SuPAR.

In analogous fashion, the invention provides a system or test kit for selecting corticosteroids to be administered as a treatment to a patient suffering from an exacerbation of inflammation of a respiratory condition, comprising:
a. one or more testing devices for determining the levels of at least 3 markers of eosinophil levels in a blood sample taken from the patient suffering from an exacerbation of inflammation of a respiratory condition;
b. a processor; and
c. a storage medium comprising a computer application that, when executed by the processor, is configured to:
   i. Access and/or calculate the determined levels of the at least one marker of eosinophil levels in a blood sample on the one or more testing devices;
   ii. Calculate whether there is a perturbed level of the at least one marker of eosinophil levels in the blood sample; and
   iii. Output from the processor that corticosteroids are selected to be administered as the treatment for the exacerbation of inflammation if there is a perturbed level of the at least one marker of eosinophil levels in the blood sample,
   wherein the at least 3 markers of eosinophil levels comprise at least Eosinophil-derived neurotoxin (EDN), Myeloperoxidase (MPO) and Eosinophil cationic protein (RNASE3), and optionally the levels of HNE, SuPAR and/or Calprotecin are also determined.

As demonstrated in the Examples section, the inventors have found that the levels of EDN and MBP each positively correlate with levels of eosinophils in the blood. Thus, increased levels of EDN and/or MBP in a blood sample indicate increased levels of eosinophils and, therefore, that corticosteroids should be administered as the (initial) treatment for the exacerbation of inflammation. The at least one marker of eosinophil levels may comprise both EDN and MBP. Similarly, this may further include RNASE3.

The method for selecting corticosteroids to be administered as a treatment to a patient suffering from an exacerbation of inflammation may further comprise at least one supporting marker of eosinophil levels wherein perturbed levels of the at least one supporting marker in combination with perturbed levels of the at least one marker of eosinophil levels result in selection of corticosteroids to be administered as the treatment for the exacerbation of inflammation.

Similarly, the system or test kit for selecting corticosteroids to be administered as a treatment to a patient suffering from an exacerbation of inflammation may further comprise determining the levels of at least one supporting marker of eosinophil and neutrophil levels in the sample, wherein:
i. the computer application, when executed by the processor, is configured to access and/or calculate the determined levels of the at least one supporting marker of eosinophil levels in the blood sample on the one or more testing devices;
ii. Calculate whether there is a perturbed level of the at least one supporting marker of eosinophil levels in the blood sample; and
iii. Output from the processor the treatment to be administered to the patient suffering from an exacerbation of inflammation, wherein perturbed levels of the at least one supporting marker in combination with perturbed levels of the at least one marker of eosinophil levels result in selection of corticosteroids to be administered as the treatment for the exacerbation of inflammation.

Typically, the levels of the at least one supporting marker of eosinophil levels are determined using the same one or more testing devices used to determine the levels of the at least one marker of eosinophil levels. However, it is possible that levels of the at least one supporting marker of eosinophil levels are determined using an additional one or more testing devices.

For the avoidance of doubt, the at least one supporting marker of eosinophil levels correspond to the at least one supporting marker of eosinophil and neutrophil levels described elsewhere herein. Thus, the at least one supporting marker of eosinophil levels may be selected from MMP9, HNE and NGAL.

As demonstrated in the Examples section, the inventors have found that levels of MMP9 may positively correlate with eosinophil levels, whilst levels of HNE negatively correlate with eosinophil levels. Thus, increased levels of MMP9 and/or decreased levels of HNE can be used, in combination with perturbed levels of at least one eosinophil marker, to indicate increased levels eosinophils respectively.

Thus, preferably the methods and systems and test kits described herein for selecting corticosteroids to be administered as a treatment to a patient suffering from an exacerbation of inflammation may comprise determining the levels of at least three markers, in any combination of markers of eosinophil levels and supporting markers of eosinophil levels, provided that the at least three markers comprise at least one marker of eosinophil levels. Thus, using three markers as an illustrative example, the following combinations are encompassed:
(i) three markers of eosinophil levels;
(ii) two markers of eosinophil levels and one supporting markers of eosinophil levels;
(iii) one marker of eosinophil levels and two supporting markers of eosinophil levels.

Furthermore, "at least three markers" as used in this specification means three or more. Thus, it encompasses three, four, five, six, seven, eight, nine, ten or more markers, and so on. It will now be readily apparent to the skilled person that the four, five, six, seven, eight, nine, ten or more markers, and so on, in the context of methods and systems and test kits described herein for selecting corticosteroids to be administered as a treatment to a patient suffering from an exacerbation of inflammation may be in any combination of markers (i.e. combinations made up of markers of eosinophil levels and supporting markers of eosinophil levels), provided that the combination of markers comprises at least one marker of eosinophil levels.

As described in the Examples section, the inventors have identified that the combination of EDN, MMP9, HNE, NGAL and MBP gave particularly good performance in relation to correlation with eosinophil levels. Thus, a particular marker combination useful in the methods and systems and test kits described herein for selecting corticosteroids to be administered as a treatment to a patient suffering from an exacerbation of inflammation may comprise or consist of EDN, MMP9, HNE, NGAL and MBP.

As described in the Examples section, the inventors have further identified that a particularly effective combination of markers of eosinophil levels during a Px exacerbation includes at least 3 markers selected from EDN, MPO, RNAse3, HNE, SuPAR and/or Calprotectin; preferably EDN, MPO and RNASE3 and optionally one or more further markers selected from HNE, SuPAR, and/or Calprotectin, preferably HNE and SuPAR. This represents an especially useful combination for any of the methods and systems and test kits described herein for determining eosinophil levels/activity and optionally selecting corticosteroids to be administered as a treatment to a patient suffering from an exacerbation of inflammation. Thus, this combination is also particularly useful in the methods, systems and kits in which at least one neutrophil marker is also determined.

It is also possible that the markers of neutrophil levels described herein may be used separately from the markers of eosinophil levels in order to determine if antibiotics would be an appropriate (initial) treatment for an exacerbation of inflammation. Thus, the invention also provides a method for selecting antibiotics to be administered as a treatment to a patient suffering from an exacerbation of inflammation of a respiratory condition, the method comprising determining the levels of at least 3 markers of neutrophil levels in a blood sample taken from the patient suffering from an exacerbation of inflammation of a respiratory condition wherein perturbed levels of at least 3 markers of neutrophil levels results in selection of antibiotics to be administered as the treatment for the exacerbation of inflammation,
wherein determining the levels of the at least 3 markers of neutrophil levels comprises determining the levels of at least (i) Matrix metallopeptidase 9 (MMP9) and Eosinophil-derived neurotoxin (EDN); and (ii) at least one of leukotriene B4 (LTB4), C-reactive protein (CRP), Soluble urokinase-type plasminogen activator receptor (SuPAR), and/or Alpha-1-antitrypsin (A1AT), preferably LTB4, optionally at least 2 or 3 of these markers.

In analogous fashion, the invention also provides a system or test kit for selecting antibiotics to be administered as a treatment to a patient suffering from an exacerbation of inflammation of a respiratory condition, comprising:
a. one or more testing devices for determining the levels of at least 3 markers of neutrophil levels in a blood sample taken from the patient suffering from an exacerbation of inflammation of a respiratory condition;
b. a processor; and
c. a storage medium comprising a computer application that, when executed by the processor, is configured to:
   i. Access and/or calculate the determined levels of the at least one marker of neutrophil levels in a blood sample on the one or more testing devices;
   ii. Calculate whether there is a perturbed level of the at least one marker of neutrophil levels in the blood sample; and
   iii. Output from the processor that antibiotics are selected to be administered as the treatment for the exacerbation of inflammation if there is a perturbed level of the at least one marker of neutrophil levels in the blood sample;
   wherein the at least 3 markers of neutrophil levels comprise at least (i) MMP9 and EDN; and (ii) at least one of leukotriene B4 (LTB4), C-reactive protein (CRP), Soluble urokinase-type plasminogen activator receptor (SuPAR), and/or Alpha-1-antitrypsin (A1AT), preferably LTB4.

As demonstrated in the Examples section, the inventors have found that levels of Calprotectin positively correlate with levels of neutrophils in the blood, whilst levels of MBP negatively correlate with levels of neutrophils in the blood. Thus, increased levels of Calprotectin and/or decreased levels of MBP in a blood sample indicate increased levels of neutrophils and, therefore, that antibiotics should be administered as the (initial) treatment for the exacerbation of inflammation.

The inventors have also found that the levels of CRP positively correlate with neutrophil levels during an exacerbation. Thus, increased levels of CRP in a blood sample taken from a patient suffering from an exacerbation of inflammation indicate increased levels of neutrophils and, therefore, that antibiotics should be administered as the (initial) treatment for the exacerbation of inflammation.

The at least one marker of neutrophil levels may comprise all of MBP, Calprotectin and A1AT. The at least one marker of neutrophil levels may comprise all of Calprotectin, IL-8, IL-6, CRP, MPO and IL-1β.

The method for selecting antibiotics to be administered as a treatment to a patient suffering from an exacerbation of inflammation may further comprise at least one supporting marker of neutrophil levels wherein perturbed levels of the at least one supporting marker in combination with perturbed levels of the at least one marker of neutrophil levels result in selection of antibiotics to be administered as the treatment for the exacerbation of inflammation.

Similarly, the system or test kit for selecting antibiotics to be administered as a treatment to a patient suffering from an exacerbation of inflammation may further comprise determining the levels of at least one supporting marker of neutrophil levels in the sample, wherein:
i. the computer application, when executed by the processor, is configured to access and/or calculate the determined levels of the at least one supporting marker of neutrophil levels in the blood sample on the one or more testing devices;
ii. Calculate whether there is a perturbed level of the at least one supporting marker of neutrophil levels in the blood sample; and
iii. Output from the processor the treatment to be administered to the patient suffering from an exacerbation of inflammation, wherein perturbed levels of the at least one supporting marker in combination with perturbed levels of the at least one marker of neutrophil levels result in selection of antibiotics to be administered as the treatment for the exacerbation of inflammation.

Typically, the levels of the at least one supporting marker of neutrophil levels are determined using the same one or more testing devices used to determine the levels of the at least one marker of neutrophil levels. However, it is possible that levels of the at least one supporting marker of neutrophil levels are determined using an additional one or more testing devices.

For the avoidance of doubt, the at least one supporting marker of neutrophil levels correspond to the at least one supporting marker of eosinophil and neutrophil levels described elsewhere herein. Thus, the at least one supporting marker of neutrophil levels may be selected from MMP9, HNE and NGAL.

As demonstrated in the Examples section, the inventors have found that levels of MMP9 positively correlate with neutrophil levels, whilst levels of HNE negatively correlate with neutrophil levels. Thus, increased levels of MMP9 and/or decreased levels of HNE can be used, in combination with perturbed levels of at least one neutrophil marker, to indicate increased levels neutrophils respectively, although further data from Example 5 suggest that during a Px, HNE levels may in fact increase with increasing neutrophil levels

As described in the Examples section, the inventors have further identified that a particularly effective combination of markers of neutrophil levels during a Px exacerbation includes at least 3 markers selected from MMP9, EDN, LTB4, CRP, SuPAR and/or A1AT; preferably at least (i) Matrix metallopeptidase 9 (MMP9) and Eosinophil-derived neurotoxin (EDN); and (ii) at least one of leukotriene B4 (LTB4), C-reactive protein (CRP), Soluble urokinase-type plasminogen activator receptor (SuPAR), and/or Alpha-1-antitrypsin (A1AT), preferably LTB4, so this represents an especially useful combination for the methods and systems and test kits described herein for determining neutrophil levels/activity and optionally selecting antibiotics to be administered as a treatment to a patient suffering from an exacerbation of inflammation. Thus, this combination is also particularly useful in the methods, systems and kits in which at least one eosinophil marker is also determined.

Thus, preferably the methods and systems and test kits described herein for selecting antibiotics to be administered as a treatment to a patient suffering from an exacerbation of inflammation may comprise determining the levels of at least three markers, in any combination of markers of neutrophil levels and supporting markers of neutrophil levels, provided that the at least three markers comprise at least one marker of neutrophil levels. Thus, using three markers as an illustrative example, the following combinations are disclosed:
(i) three markers of neutrophil levels;
(ii) two markers of neutrophil levels and one supporting markers of neutrophil levels;
(iii) one marker of neutrophil levels and two supporting markers of neutrophil levels.

Furthermore, "at least three markers" as used in this specification means three or more. Thus, it encompasses three, four, five, six, seven, eight, nine, ten or more markers, and so on. It will now be readily apparent to the skilled person that the four, five, six, seven, eight, nine, ten or more markers, and so on, in the context of methods and systems and test kits described herein for selecting antibiotics to be administered as a treatment to a patient suffering from an exacerbation of inflammation may be in any combination of markers (i.e. combinations made up of markers of neutrophil levels and supporting markers of neutrophil levels), provided that the combination of markers comprises at least one marker of neutrophil levels.

As described in the Examples section, the inventors have identified marker combinations with particularly good performance in relation to correlation with neutrophil levels. Thus, particular marker combinations useful for selecting antibiotics to be administered as a treatment to a patient suffering from an exacerbation of inflammation may comprise:
(i) MBP, Calprotectin and A1AT
(ii) MMP9, CRP and/or NGAL;
(iii) MMP9, Calprotectin, HNE and CRP; and/or
(iv) A1AT and/or NGAL.

Specific marker combinations useful for selecting antibiotics to be administered as a treatment to a patient suffering from an exacerbation of inflammation include:
- Calprotectin, MBP, MMP9, CRP and NGAL
- MBP, Calprotectin and A1AT
- MBP, Calprotectin, A1AT, MMP9 and CRP
- MBP, Calprotectin, A1AT, MMP9, CRP and NGAL
- MMP9, Calprotectin, HNE, CRP and A1AT
- MMP9, Calprotectin, HNE and CRP
- MMP9, Calprotectin, HNE, CRP, A1AT and NGAL
- Calprotectin, MMP9, IL-8, IL-6, NGAL, CRP, MPO and IL-1β

Other markers which may be useful as further markers in addition to those required by the invention, according to all of the methods, systems and test kits described herein, include: Periostin, active MMP (composite activity of MMP2, MMP8, MMP9, MMP12, MMP13 and MMP7), Fibrinogen, Secretory Leukocyte Protease Inhibitor (SLPI), N-formylmethionine-leucyl-phenylalanine (fMLP), Desmosine (whole or fragments), Club cell-16 (CC16), Tissue Inhibitor of Metalloproteinase-1 (TIMP1), Tissue Inhibitor of Metalloproteinase-2 (TIMP2), Chitinase-3-like-1 protein (CHI3L1), N-acetyl-proline-glycine-proline (AcPGP), beta-2-microglobulin (B2M), Cystatin C, Matrix Metalloproteinase 8 (MMP8), Retinol Binding Protein-4 (RBP4), Human Serum Albumin (HSA), Large Elastin Fragments (LEF), Siglec 8 and Soluble Receptor for Advanced Glycation End Products (sRAGE).

Typically, the methods, systems and test kits described herein are designed to be used at the onset of an exacerbation of inflammation so that the most appropriate initial treatment can be administered to the patient. Specifically, when an appropriate initial treatment (such as corticosteroids) is selected, preferably the other treatments (such as antibiotics) are not selected as initial treatments. However, the methods described above can be performed again after a treatment has been selected and administered to the patient in order to determine whether or not the treatment has been effective. Thus, the invention also provides a method for selecting and monitoring treatment of a patient suffering from an exacerbation of inflammation of a respiratory condition, the method comprising:
(i) selecting a treatment to be administered to the patient using a method as defined above; and
(ii) with respect to the at least 3 markers for which levels were perturbed when determining the treatment to be administered of step (i), determining the levels of said at least 3 markers in a further blood sample taken from the patient at a later time point wherein:
   (a) perturbed levels of the at least 3 markers in the further sample indicate that the treatment should continue or be altered; or
   (b) a return to non-perturbed levels of the at least 3 markers in the further sample indicate or predict successful treatment of the exacerbation of inflammation.

Thus, if perturbed levels of the at least 3 markers continue to be detected after a first treatment has been administered, this may indicate that the same treatment should continue to be administered, or, treatment should be altered (e.g. the dosage adjusted, level of intervention altered, or changed to a different therapeutic agent). This may depend on the length of time that has passed since the previous treatment was administered: a short passage of time may indicate that the same treatment is continued but further monitored, whilst a longer passage of time (e.g. towards the end or after the expected therapeutic window has expired) may indicate that the treatment should be altered. Similarly, if the levels of the at least 3 markers are further perturbed after administration of the previous treatment this may indicate that the treatment is ineffective and the exacerbation of inflammation is getting worse. This would indicate that treatment should be changed to a different therapeutic agent. A "non-perturbed level" means a level of the marker considered stable based on a threshold or baseline or a population level as defined herein.

The methods and systems and kits of the invention are useful in determining eosinophil levels or activity, and/or neutrophil levels or activity. Lung exacerbations can be classified as neutrophil driven and eosinophil driven ones and there was a clear unmet need for tests that allow patients to be diagnosed or stratified as having a neutrophil or eosinophil-driven exacerbation. Such a diagnosis or stratification may subsequently guide clinicians in their choice of intervention. Suitable analysis methods, systems and kits are now provided as described herein.

Accordingly, disclosed is a method of analysis, the method comprising determining (detecting or measuring) the levels of at least 3 markers of eosinophil levels and/or at least 3 markers of neutrophil levels in a blood sample taken from the patient suffering from an exacerbation of inflammation of a respiratory condition
wherein
determining the levels of the at least 3 markers of eosinophil levels comprises determining the levels of at least 3 markers selected from EDN, MPO, RNAse3, HNE, SuPAR and/or Calprotectin; preferably EDN, MPO and RNASE3 and optionally one or more further markers selected from HNE, SuPAR, and/or Calprotectin, preferably HNE and SuPAR; and/or
wherein determining the levels of the at least 3 markers of neutrophil levels comprises determining the levels of at least 3 markers selected from MMP9, EDN, LTB4, CRP, SuPAR and/or A1AT; preferably at least (i) Matrix metallopeptidase 9 (MMP9) and Eosinophil-derived neurotoxin (EDN); and (ii) at least one of leukotriene B4 (LTB4), C-reactive protein (CRP), Soluble urokinase-type plasminogen activator receptor (SuPAR), and/or Alpha-1-antitrypsin (A1AT), preferably LTB4.

This method may be a method of diagnosis or a method of stratification and may include a step of diagnosing or stratifying a patient as having an neutrophil or eosinophil-driven exacerbation based on the results of the analysis.

There are various known techniques by which marker levels may be measured. Thus, by marker levels is meant the level of expression and/or activity and/or amount and/or concentration of the marker. Expression levels of the markers may be measured in blood. Expression levels may correlate with activity and can thus be used as a surrogate of activity. Expression levels may be measured at the level of protein or mRNA according to any suitable method. Protein modifications, such as glycosylation may also be relevant and can be measured by any suitable method. Many such methods are well known in the art and include use of mass spectrometry (e.g. MALDI-TOF mass spectrometry). MicroRNAs may also be measured in blood samples as post-transcriptional regulators of gene expression. A platform such as that offered by Exiqon may be utilised to provide high-throughput microRNA profiling. Such platforms may be array and/or PCR based.

The expression level and/or amount and/or concentration of a marker (e.g. a protein) may rely upon a binding reagent such as an antibody or aptamer that binds specifically to the marker of interest (e.g. protein). The antibody may be of monoclonal or polyclonal origin. Fragments and derivative antibodies may also be utilised, to include without limitation Fab fragments, ScFv, single domain antibodies, nanoantibodies, heavy chain antibodies, aptamers etc. which retain specific binding function and these are included in the definition of "antibody". Such antibodies are useful in the methods, systems and test kits of the invention. They may be used to measure the level of a particular marker (e.g. protein, or in some instances one or more specific isoforms of a protein. The skilled person is well able to identify epitopes that permit specific isoforms to be discriminated from one another).

Methods for generating specific antibodies are known to those skilled in the art. Antibodies may be of human or non-human origin (e.g. rodent, such as rat or mouse) and be humanized etc. according to known techniques (Jones et al., Nature (1986) May 29-Jun. 4;321(6069):522-5; Roguska et al., Protein Engineering, 1996, 9(10):895-904; and Studnicka et al., Humanizing Mouse Antibody Frameworks While Preserving 3-D Structure. Protein Engineering, 1994, Vol.7, pg 805).

In certain embodiments the expression level and/or amount and/or concentration of a marker is determined using an antibody or aptamer conjugated to a label. By label is meant a component that permits detection, directly or indirectly. For example, the label may be an enzyme, optionally a peroxidase, or a fluorophore. Gold labels may be utilised, e.g. in the form of colloidal gold.

A label is an example of a detection agent. By detection agent is meant an agent that may be used to assist in the detection of the antibody-marker (e.g. protein) complex. Where the antibody is conjugated to an enzyme the detection agent may comprise a chemical composition such that the enzyme catalyses a chemical reaction to produce a detectable product. The products of reactions catalysed by appropriate enzymes can be, without limitation, fluorescent, luminescent, or radioactive or they may absorb or reflect visible or ultraviolet light. Examples of detectors suitable for detecting such detectable labels include, without limitation, x-ray film, radioactivity counters, scintillation counters, spectrophotometers, colorimeters, fluorometers, luminometers, photodetectors and densitometers. In certain embodiments the detection agent may comprise a secondary antibody. The expression level is then determined using an unlabelled primary antibody that binds to the target protein and a secondary antibody conjugated to a label, wherein the secondary antibody binds to the primary antibody.

Additional techniques for determining expression level at the level of protein and/or the amount and/or concentration of a marker include, for example, Western blot, immunoprecipitation, immunocytochemistry, mass spectrometry, ELISA and others (see ImmunoAssay: A Practical Guide, edited by Brian Law, published by Taylor & Francis, Ltd., 2005 edition). To improve specificity and sensitivity of an assay method based on immunoreactivity, monoclonal antibodies are often used because of their specific epitope recognition. Polyclonal antibodies have also been successfully used in various immunoassays because of their increased affinity for the target as compared to monoclonal antibodies. Levels of protein may be detected using a lateral flow assay in some embodiments (discussed in further detail herein).

Measuring mRNA in a biological sample may be used as a surrogate for detection of the level of the corresponding protein in the blood sample. Thus, the expression level of any of the relevant markers described herein can also be detected by detecting the appropriate RNA.

Accordingly, in specific embodiments the expression level is determined by microarray, northern blotting, or nucleic acid amplification. Nucleic acid amplification includes PCR and all variants thereof such as real-time and end point methods and qPCR. Other nucleic acid amplification techniques are well known in the art, and include methods such as NASBA, 3SR and Transcription Mediated Amplification (TMA). Other suitable amplification methods include the ligase chain reaction (LCR), selective amplification of target polynucleotide sequences (US Patent No. 6,410,276), consensus sequence primed polymerase chain reaction (US Patent No 4,437,975), arbitrarily primed polymerase chain reaction (WO 90/06995), invader technology, strand displacement technology, recombinase polymerase amplification (RPA), nicking enzyme amplification reaction (NEAR) and nick displacement amplification (WO 2004/067726). This list is not intended to be exhaustive; any nucleic acid amplification technique may be used provided the appropriate nucleic acid product is specifically amplified. Design of suitable primers and/or probes is within the capability of one skilled in the art. Various primer design tools are freely available to assist in this process such as the NCBI Primer-BLAST tool. Primers and/or probes may be at least 15, 16, 17, 18, 19, 20, 21, 22, 23, 24 or 25 (or more) nucleotides in length. mRNA expression levels may be measured by reverse transcription quantitative polymerase chain reaction (RT-PCR followed with qPCR). RT-PCR is used to create a cDNA from the mRNA. The cDNA may be used in a qPCR assay to produce fluorescence as the DNA amplification process progresses. By comparison to a standard curve, qPCR can produce an absolute measurement such as number of copies of mRNA per cell. Northern blots, microarrays, Invader assays, and RT-PCR combined with capillary electrophoresis have all been used to measure expression levels of mRNA in a sample. See Gene Expression Profiling: Methods and Protocols, Richard A. Shimkets, editor, Humana Press, 2004.

RNA expression may be determined by hybridization of RNA to a set of probes. The probes may be arranged in an array. Microarray platforms include those manufactured by companies such as Affymetrix, Illumina and Agilent. RNA expression may also be measured using next generation sequencing methods, such as RNA-seq.

Similarly, activity of an effector molecule, such as enzymatic activity, may be measured in the blood sample. Enzymatic activity may be measured for example by detecting processing of a substrate, which may be labelled, in the sample. For example, the assay may be a fluorogenic substrate assay. Enzyme activity may be detected using a suitable lateral flow assay. Examples of suitable assay formats include the assays set forth in International Patent Applications WO2009/024805, WO2009/063208, WO2007/128980, WO2007/096642, WO2007/096637, WO2013/156794 and WO2013/156795.

In specific embodiments, protease activity is determined by measuring cleavage of a peptide substrate. For example, the assay may be a fluorogenic substrate assay. In certain embodiments, protease activity is determined by a method comprising:
a. bringing an indicator molecule into contact with the test sample, said indicator molecule comprising
   i. a cleavage region comprising at least one cleavage site, which can be cleaved by said protease if present; and
   ii. a capture site;
   wherein cleavage of the at least one cleavage site produces a novel binding site;
b. adding to the test sample binding molecules capable of binding to the novel binding site, wherein the binding molecules are incapable of binding to the indicator molecule unless and until cleavage has occurred;
c. capturing the part of the indicator molecule containing the novel binding site at a capture zone through binding of capture molecules in the capture zone to the capture site; and
d. detecting cleavage of the at least one cleavage site by determining binding of the binding molecules to the novel binding site of the indicator molecule captured in the capture zone.

This assay may be referred to herein as the "ultimate ELTABA" assay.

Thus, the invention may incorporate an enzyme detection device for detecting the presence in a test sample of cleavage activity of an enzyme capable of cleaving a substrate, the device comprising:
(i) an indicator molecule for adding to the test sample, said indicator molecule comprising
   (a) a cleavage region comprising at least one cleavage site, which can be cleaved by said enzyme if said enzyme cleavage activity is present; and
   (b) a capture site;
   wherein cleavage of the at least one cleavage site produces a novel binding site;
(ii) a capture zone to receive the test sample, wherein the capture zone comprises capture molecules capable of binding to the capture site of the indicator molecule in order to immobilise the indicator molecule including the novel binding site; and
(iii) binding molecules capable of binding to the novel binding site, wherein the binding molecules are incapable of binding to the indicator molecule unless and until cleavage has occurred.

Similarly, the invention may incorporate an enzyme detection device for detecting the presence in a test sample of cleavage activity of an enzyme capable of cleaving a substrate, the device comprising:
(i) an indicator molecule for adding to the test sample, said indicator molecule comprising
   (a) a cleavage region comprising at least one cleavage site, which can be cleaved by said enzyme if said enzyme cleavage activity is present; and
   (b) a capture site;
   wherein cleavage of the at least one cleavage site produces at least two parts of the cleavage region, at least one part of which remains connected to the capture site;
(ii) a capture zone to receive the test sample, wherein the capture zone comprises capture molecules capable of binding to the capture site of the indicator molecule; and
(iii) binding molecules capable of binding to the part of the indicator molecule containing the at least one part of the cleavage region connected to the capture site, wherein the binding molecules are incapable of binding to the indicator molecule unless and until cleavage has occurred.

The two parts of the cleavage region are thus separated from one another at the site of cleavage. The cleavage event at the site of the cleavage produces the novel binding site. These devices may be included as one or more testing devices in the systems and test kits of the invention.

The invention may further rely upon a method for detecting the presence or absence in a test sample of cleavage activity of an enzyme capable of cleaving a substrate, the method comprising:
(i) bringing an indicator molecule into contact with the test sample, said indicator molecule comprising
   (a) a cleavage region comprising at least one cleavage site, which can be cleaved by said enzyme if said enzyme cleavage activity is present; and
   (b) a capture site;
   wherein cleavage of the at least one cleavage site produces a novel binding site;
(ii) adding to the test sample binding molecules capable of binding to the novel binding site, wherein the binding molecules are incapable of binding to the indicator molecule unless and until cleavage has occurred;
(iii) capturing the part of the indicator molecule containing the novel binding site at a capture zone through binding of capture molecules in the capture zone to the capture site; and
(iv) detecting cleavage of the at least one cleavage site by determining binding of the binding molecules to the novel binding site of the indicator molecule captured in the capture zone.

Similarly, the invention may also incorporate a method for detecting the presence or absence in a test sample of cleavage activity of an enzyme capable of cleaving a substrate, the method comprising:
(i) bringing an indicator molecule into contact with the test sample, said indicator molecule comprising
   (a) a cleavage region comprising at least one cleavage site, which can be cleaved by said enzyme if said enzyme cleavage activity is present; and
   (b) a capture site
   wherein cleavage of the at least one cleavage site produces at least two parts of the cleavage region, at least one part of which remains connected to the capture site;
(ii) adding to the test sample binding molecules capable of binding to the part of the indicator molecule containing the at least one part of the cleavage region connected to the capture site, wherein the binding molecules are incapable of binding to the indicator molecule unless and until cleavage has occurred;
(iii) capturing the part of the indicator molecule containing the at least one part of the cleavage region connected to the capture site at a capture zone through binding of capture molecules in the capture zone to the capture site; and
(iv) detecting cleavage of the at least one cleavage site by determining binding of the binding molecules to the part of the indicator molecule captured in the capture zone.

These specific devices and methods have been shown by the inventors to have exquisite sensitivity. They therefore have specific application in selecting a treatment for an exacerbation of inflammation by measuring the activity of a marker as described herein (which possesses cleavage activity) in blood samples. Typically, the marker is a protease such as MMP (e.g. MMP9, MMP8 or total active MMP) or HNE.

The enzyme detection devices useful in the invention may be supplied in a format ready for immediate use. Alternatively, the essential components may be provided as a kit of parts, optionally together with suitable reagents and/or instructions for assembly of the enzyme detection device. Accordingly, provided herein is an enzyme detection kit for detecting the presence in a blood test sample of cleavage activity of an enzyme capable of cleaving a substrate, the kit comprising:
(i) an indicator molecule for adding to the test sample, said indicator molecule comprising
   (a) a cleavage region comprising at least one cleavage site, which can be cleaved by said enzyme if said enzyme cleavage activity is present; and
   (b) a capture site;
   wherein cleavage of the at least one cleavage site produces a novel binding site;
(ii) capture molecules capable of binding to the capture site of the indicator molecule
(iii) a solid support to which the capture molecules can be attached (i.e. are attachable or attached) to form a capture zone to receive the test sample; and
(iv) binding molecules capable of binding to the novel binding site, wherein the binding molecules are incapable of binding to the indicator molecule unless and until cleavage has occurred.

Also useful in the invention is an enzyme detection kit for detecting the presence in a blood test sample of cleavage activity of an enzyme capable of cleaving a substrate, the kit comprising:
(i) an indicator molecule for adding to the test sample, said indicator molecule comprising
   (a) a cleavage region comprising at least one cleavage site, which can be cleaved by said enzyme if said enzyme cleavage activity is present; and
   (b) a capture site;
   wherein cleavage of the at least one cleavage site produces at least two parts of the cleavage region, at least one part of which remains connected to the capture site;
(ii) capture molecules capable of binding to the capture site of the indicator molecule,
(iii) a solid support to which the capture molecules can be attached (i.e. are attachable or attached) to form a capture zone to receive the test sample; and
(iii) binding molecules capable of binding to the part of the indicator molecule containing the at least one part of the cleavage region connected to the capture site, wherein the binding molecules are incapable of binding to the indicator molecule unless and until cleavage has occurred.

It follows therefore that the invention also provides for use of an enzyme detection device as described and defined herein for selecting a treatment to be administered to a patient suffering from an exacerbation of inflammation in a blood test sample. Similarly, the invention also provides for use of a method as described and defined herein for selecting a treatment to be administered to a patient suffering from an exacerbation of inflammation in a blood test sample. The invention further provides for use of an enzyme detection kit as described and defined herein for selecting a treatment to be administered to a patient suffering from an exacerbation of inflammation in a blood test sample. In each of these uses, the respiratory condition may be chronic obstructive pulmonary disease.

Central to the methods, enzyme detection devices and enzyme detection kits for detecting the presence or absence in a test sample of cleavage activity of an enzyme capable of cleaving a substrate described and defined herein is the indicator molecule. The indicator molecule comprises a cleavage region comprising at least one cleavage site. The cleavage site is cleaved by an effector molecule, typically an enzyme or enzymes, in the blood test sample with the relevant enzyme cleavage activity. The cleavage region provides a suitable context for the cleavage site to ensure cleavage is efficient, if the enzyme is present in the sample. In specific embodiments the cleavage region is a peptide. In addition to the peptide bond representing a protease cleavage site, the additional amino acids in the peptide may ensure specificity and sensitivity of cleavage. The cleavage region may contain multiple cleavage sites in certain embodiments, particularly where the indicator molecule is structurally constrained, for example where it also comprises a scaffold molecule.

The indicator molecule also comprises a capture site (intended to encompass at least one capture site). The capture site is a discrete region of the indicator molecule which permits immobilization of the indicator molecule, whether cleaved or uncleaved, at a capture zone. The capture site is discussed herein below in greater detail.

The indicator molecule also optionally comprises a scaffold molecule, as discussed in greater detail below.

Cleavage of the indicator molecule splits the indicator molecule to reveal or form at least one novel binding site. The two parts of the cleavage region are thus separated from one another at the site of cleavage. Typically, the novel binding site comprises a conformational epitope produced as a consequence of cleavage. Use of binding molecules that bind specifically to the newly revealed binding site or sites but not to the indicator molecule prior to cleavage enables specific and sensitive detection of cleavage activity of an enzyme. Accordingly, in some embodiments, cleavage of the at least one cleavage site produces at least two parts of the indicator molecule (or cleavage region of the indicator molecule), at least one part of which contains (or remains connected to) the capture site and as a consequence of cleavage contains a binding site for binding molecules and wherein the binding molecules are incapable of binding to the binding site unless and until cleavage has occurred. In other words, the binding site is hidden or is not formed until cleavage at the cleavage site occurs.

In some embodiments, cleavage of the at least one cleavage site produces at least two separate parts of the (cleavage region of the) indicator molecule. Thus, cleavage may produce at least two parts or fragments; one part or fragment that contains or is connected to the capture site and a separate part or fragment that does not contain, or is not connected to, the capture site. The binding molecules bind to the new binding site on the part or parts of the indicator molecule that contain or include the capture site. This permits specific detection of cleavage at the site of capture of the indicator molecule through binding to the capture molecules (i.e. binding of the binding molecules is detected in the capture zone).

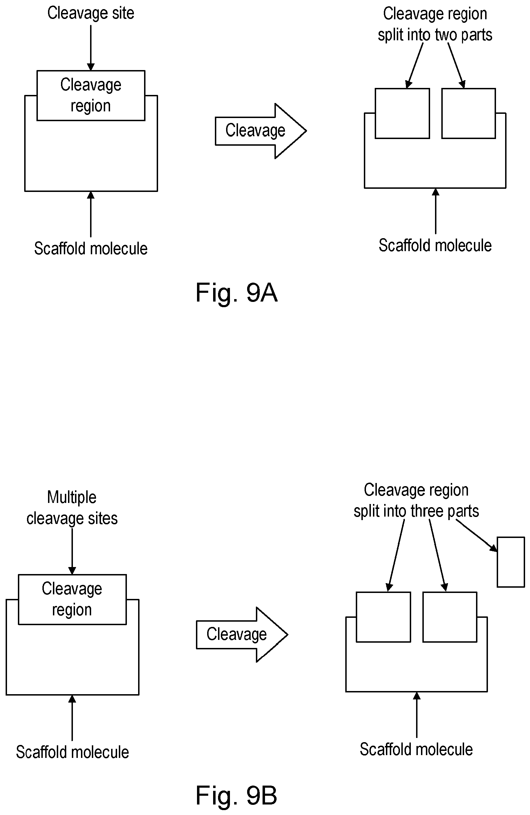

However, it is not essential that cleavage (at the cleavage site) produces at least two completely separate molecules, provided that cleavage produces a novel binding site for the binding molecules and wherein the binding molecules are incapable of binding to the binding site unless and until cleavage has occurred. Thus cleavage produces two parts of the cleavage region which are separated at the cleavage site. Accordingly, in some embodiments, cleavage of the at least one cleavage site produces at least two parts of the cleavage region, at least two parts of which remain connected, either directly or indirectly (for each part), to the capture site. This is shown schematically in Figure 9A. In specific embodiments the indicator molecule contains a further linkage or connection away from the cleavage site or outside of the cleavage region such that cleavage of the at least one cleavage site produces at least two parts of the cleavage region of the indicator molecule which remain connected to one another. This does not exclude the possibility that cleavage produces at least three fragments, at least one of which does not remain connected via the further linkage or connection. This is particularly the case where the cleavage region may comprise more than one cleavage site. This is shown schematically in Figure 9B. The further linkage or connection may comprise a disulphide bond in some embodiments. It has been found that use of scaffold molecules, linked to the indicator molecule, provides a further linkage or connection within the indicator molecules. Such scaffold molecules may act as a structural constraint that is useful for developing binding molecules that bind to the indicator molecule only after cleavage has occurred. Without being bound by theory, the structural constraint is believed to assist in producing a specific and reproducible binding site that is not present unless and until cleavage at the cleavage site has occurred. The scaffold molecule may enhance the differences in spatial conformation between the indicator molecule pre- and post-cleavage, as discussed in greater detail herein. The scaffold may also constrain the cleaved indicator molecule in a particular spatial conformation following cleavage. This may assist in improving specificity of detection in terms of the binding molecules discriminating between cleaved and uncleaved indicator molecules, by providing a clearly defined and different molecule after cleavage against which binding molecules can be designed or raised. Thus, in some embodiments, the binding molecules bind to the region of cleavage. In specific embodiments, the binding site may thus encompass both sides of the cleavage site following cleavage (i.e. at least two parts of the cleavage region). The binding molecules may bind to both parts of the indicator molecule following cleavage.

The invention therefore may also rely upon use of an indicator molecule in detecting the presence in a blood test sample of cleavage activity of an effector molecule, such as an enzyme capable of cleaving a substrate, the indicator molecule comprising:
(a) a cleavage region comprising at least one cleavage site, which can be cleaved by said enzyme if said enzyme cleavage activity is present,
(b) a capture site; and
(c) a scaffold molecule which acts to connect at least two parts of the indicator molecule outside of the cleavage site, such as outside of the cleavage region;
wherein the scaffold further acts to structurally constrain the indicator molecule in a manner such that cleavage of the at least one cleavage site produces a novel binding site to which binding molecules bind, but wherein the binding molecules are incapable of binding to the indicator molecule unless and until cleavage has occurred.

The invention may also incorporate an indicator molecule for use in detecting the presence in a blood test sample of cleavage activity of an effector molecule, in particular an enzyme capable of cleaving a substrate, the indicator molecule comprising:
(a) a cleavage region comprising at least one cleavage site, which can be cleaved by said enzyme if said enzyme cleavage activity is present to produce at least two parts of the cleavage region,
(b) a capture site; and
(c) a scaffold molecule which acts to connect at least two parts of the indicator molecule such that cleavage of the at least one cleavage site produces at least two parts of the cleavage region of the indicator molecule which remain connected to one another wherein the scaffold further acts to structurally constrain the indicator molecule in a manner such that cleavage of the at least one cleavage site produces a (novel) binding site to which binding molecules bind, but wherein the binding molecules are incapable of binding to the indicator molecule unless and until cleavage has occurred.

The scaffold molecule is typically attached to the indicator molecule away from the cleavage site so that cleavage activity of the enzyme is not inhibited by the scaffold. Thus the cleavage region may be separated from the scaffold molecule by one or more linker or spacer regions. Those linker or spacer regions may incorporate the capture site in some embodiments. The scaffold molecule is typically linked to the indicator molecule by two linkages, although it is possible that additional linkages can be employed - for example 3, 4, 5 or 6 etc. - linkages depending upon the scaffold molecule that is used and the nature of the indicator molecule. It is also possible that a single scaffold molecule can be linked to multiple indicator molecules. In embodiments where the scaffold molecules contain more than two halogen substituents, in particular bromomethyl substituents, such as four or six bromomethyl substituents, the scaffold molecule may provide a structural constraint for multiple indicator molecules. Each pair of substituents may be attached to connect at least two parts of a cleavage region. Thus, the scaffold effectively links (and structurally constrains) multiple separate cleavage regions. In specific embodiments, the indicator molecules comprise more than one constrained peptide (cleavage region). The cleavage regions can also be different resulting in a single molecule containing different cleavable sequences. Here it may be possible to detect cleavage of each individual peptide cleavage region using two or more distinct binding molecules (e.g. antibodies raised against its cleaved substrate). Consequently, where an assay signal is required only when two or more proteases are present it is possible that binding molecule (antibody) binding only takes place when all the distinct cleavage sites have been cleaved. In this instance the binding molecule (antibody) would have to be raised to the form of indicator molecule after cleavage by the two or more proteases.

The scaffold molecule assists in constraining the cleaved ends or parts of the indicator molecule (usually a peptide) to produce a novel and specific binding site for a binding molecule (usually an antibody binding to a newly revealed or produced epitope, in particular a conformational epitope). The binding molecule may, therefore, bind specifically to either cleaved end or part of the indicator molecule or to both sides of the cleavage site (i.e. within the cleavage region either side of the cleavage site). In specific embodiments, the scaffold further acts to structurally constrain the indicator molecule in a manner such that cleavage of the at least one cleavage site produces a binding site containing both parts of the cleavage region of the indicator molecule to which binding molecules bind, but wherein the binding molecules are incapable of binding to the indicator molecule unless and until cleavage has occurred. In specific embodiments, the binding site includes the cleavage site. In specific embodiments, the binding site represents a novel structural conformation of the indicator molecule. Cleavage may produce at least one new conformational epitope. The novel binding site for the binding molecule may comprise any part of the indicator molecule, provided that enzyme cleavage activity and capture are not substantially impeded. In certain embodiments, the binding site comprises at least a portion of the cleavage region. In specific embodiments, the binding site comprises at least a portion of the scaffold molecule.

Typically, the cleavage site is specific for cleavage by a protease. However, as discussed herein, the indicator molecules may be cleaved by other enzymes which act as markers of eosinophil and/or neutrophil levels in inflammatory exacerbation events. One or more different proteases may be detected according to the invention. In certain embodiments, the cleavage site is specific for cleavage by a matrix metalloproteinase (MMP). MMPs are zinc-dependent endopeptidases. They are responsible for cleaving various proteins, including extracellular matrix proteins. The MMPs include MMP1, MMP2, MMP3, MMP7, MMP8, MMP9, MMP10, MMP11, MMP12, MMP13, MMP14, MMP15, MMP16, MMP17, MMP19, MMP20, MMP21, MMP23A, MMP23B, MMP24, MMP25, MMP26, MMP27 and MMP28. In particular, the MMP may be MMP9. HNE is another relevant marker that can be detected using this format.

The at least one cleavage site may be biased for cleavage by specific proteases in some embodiments. This permits the invention to be utilised in order to detect specific protease activity in the test sample. Many proteases are known and their sites of preferred cleavage well reported. In certain embodiments, the at least one cleavage site is biased for cleavage by specific matrix metalloproteinases. More specifically, in some embodiments, the at least one cleavage site is biased for cleavage by MMP-9 and/or MMP-8 or for MMP-13 and/or MMP-9. The at least one cleavage site may be biased for cleavage by MMP-13, 9, 2, 12 and 8. The bias may be for the group of MMPs equally or may be in that particular order of preference. As is shown herein, it is possible to design specific indicator molecules and cleavage sites within the indicator molecules that are biased for cleavage by these particular MMPs, in the specified order of preference. Accordingly, in some embodiments, the cleavage site is within the amino acid sequence GPQGIFGQ (SEQ ID NO: 1). This may be considered a specific example of the "cleavage region" of the indicator molecule. In those embodiments, cleavage produces a part of the cleavage region of the indicator molecule containing the amino acid sequence GPQG and a part of the cleavage region of the indicator molecule containing the amino acid sequence IFQG. Either part can be the part connected to the capture site. In specific embodiments, the indicator molecule comprises the amino acid sequence CGPQGIFGQC (SEQ ID NO: 2). Inclusion of the cysteine residues provides thiol groups which represent a convenient linkage point for various scaffold molecules. The cleavage region may be separated from the attachment points for the scaffold molecule by one or more linker or spacer regions in some embodiments. Thus, the indicator molecule may comprise the structure:

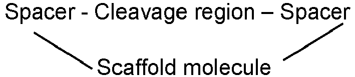

The capture site may be found within one or both of the spacers in some embodiments. Thus, the indicator molecules may comprise suitable amino acids at or near the N and C terminus to facilitate linkage to the scaffold molecule. The amino acids may comprise thiol groups. Suitable residues include cysteine and selenium. The scaffold molecules may be attached to the indicator molecules via thioether linkages.

A range of suitable scaffold molecules and methods for linking the scaffold molecules to a peptide are discussed in WO2004/077062 and WO2008/013454.

The present invention applies these scaffold molecules in a new manner to present cleavage sites and produce new binding sites after cleavage which permit detection of enzyme cleavage activity (especially protease activity) in a test sample in order to select a treatment to be administered to a patient suffering from an exacerbation of inflammation.

In certain embodiments, the scaffold molecule comprises a (hetero)aromatic molecule. In more specific embodiments, the (hetero)aromatic molecule comprises at least two benzylic halogen substitutents. The scaffold molecule is a halomethylarene in some embodiments, such as a halomethylarene selected from the group consisting of bis(bromomethyl)benzene, tris(bromomethyl)benzene and tetra(bromomethyl)benzene, or a derivative thereof. In specific embodiments, the scaffold is selected from the group consisting of ortho-, meta- and para-dihaloxylene and 1,2,4,5-tetrahalodurene, such as meta-1,3-bis(bromomethyl)benzene (m-T2), ortho-1,2-bis(bromomethyl)benzene (o-T2), para-1,4-bis(bromomethyl)benzene (p-T2), meta-1,3-bis(bromomethyl)pyridine (m-P2), 2,4,6-tris(bromomethyl)mesitylene (T3), meta-1,3-bis(bromomethyl)-5-azidobenzene (m-T3-N3) and/or 1,2,4,5-tetrabromodurene (T4).

Suitable derivatives of halomethyl arenes include ortho-, meta- and para-bis(bromomethyl) benzenes. More specifically 1,2-bis(bromomethyl)benzene, 1,3-bis(bromomethyl)benzene and 1,4-bis(bromomethyl)benzene. Further substituted halomethylarenes include 1,3,5-tris(bromomethyl)benzene, 1,2,4,5-tetrakis(bromomethyl)benzene and 1,2,3,4,5,6-hexakis(bromomethyl)benzene. Polycyclic halomethylarenes include 2,7-bis(bromomethyl)-naphthalene, 1,4-bis(bromomethyl)-naphthalene, 1,8-bis(bromomethyl)-naphthalene, 1,3-bis(bromomethyl)-naphthalene, 1,2-bis(bromomethyl)-naphthalene, 2,3-bis(bromomethyl)-naphthalene, 2,6-bis(bromomethyl)-naphthalene, 1,2,3,4-tetrakis(bromomethyl)-naphthalene, 9,10-bis(bromomethyl)-phenanthrene, 5,10-bis(bromomethyl)-anthracene, 9,10-bis(bromomethyl)-anthracene, and 1-(bromomethyl)-3-[3-(bromomethyl)benzyl]benzene. Methyl substituted halomethylarenes include 1,3-bis(bromomethyl)-5-methylbenzene, 2,5-bis(bromomethyl)-1,3-dimethylbenzene, 2,5-bis(bromomethyl)-1,4-dimethylbenzene, 2,4-bis(bromomethyl)-1,3,5-trimethylbenzene and 3,6-bis(bromomethyl)durene. Nitro substituted halomethylarenes include 3, 4-bis(bromomethyl)-nitrobenzene and 2,3-bis(bromomethyl)-nitrobenzene. Hydroxy substituted halomethylarenes include 1,3-bis(bromomethyl)-5-hydroxybenzene and cyano substituted halomethylarenes include 2,6-bis(bromomethyl)-benzonitrile. Methoxy substituted halomethylarenes include 1,3-bis(bromomethyl)-5-methoxybenzene, 1,3-bis(bromomethyl)-2-methoxy-5-methylbenzene, 1,3-bis(bromomethyl)-5-hydroxybenzene, 2,3-bis(bromomethyl)-1,4-dimethoxybenzene, and 2,5-bis(bromomethyl)-1,4-dimethoxybenzene.

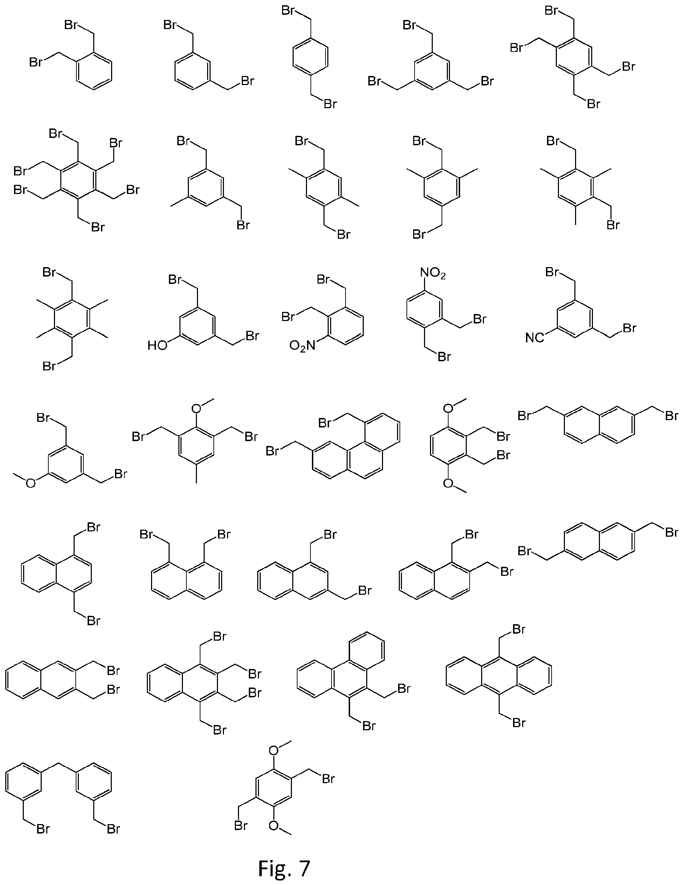

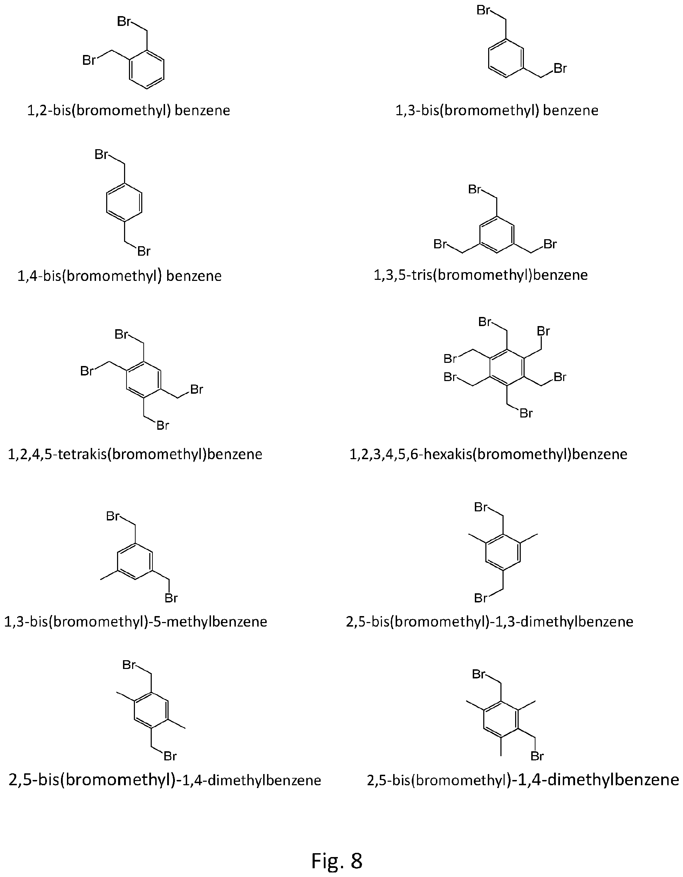

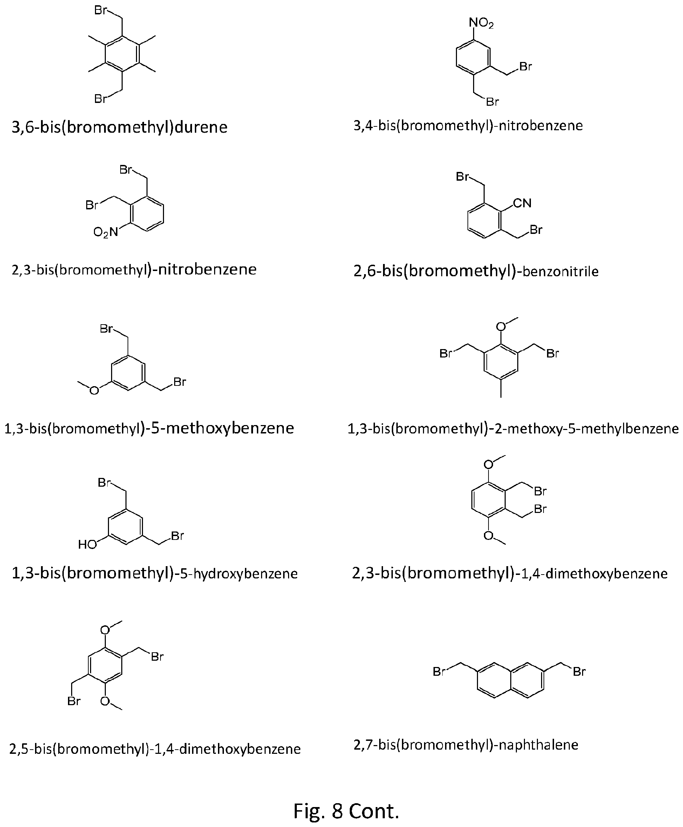

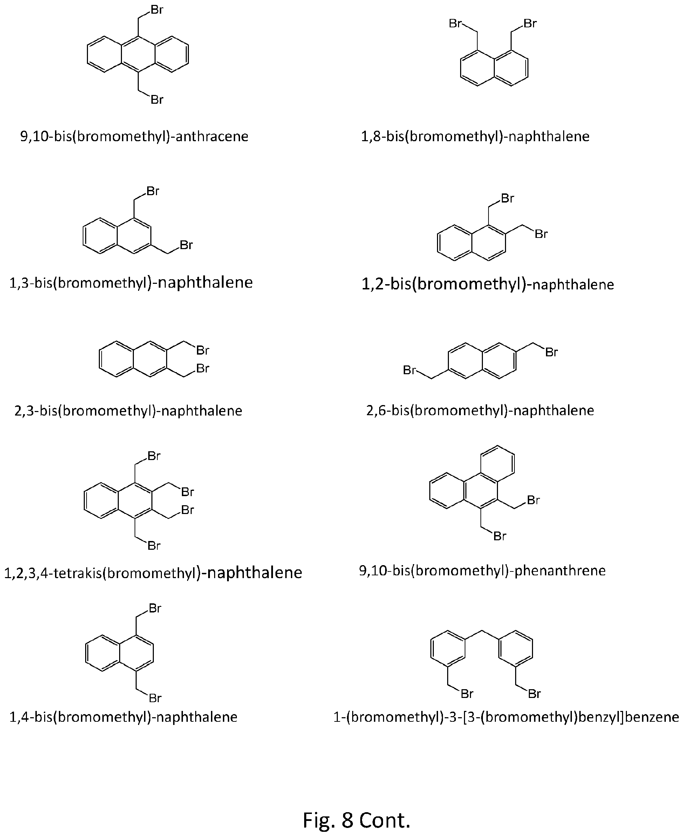

Some suitable scaffold molecules for use in the indicator molecules are shown in Figure 7. A number of specific suitable scaffold molecules are also shown, together with proposed nomenclature, in Figure 8.

Due to their relative rigidity and ease of synthetic use, the halomethyl arene derivatives are preferred candidates to act as scaffold molecules in the present invention. They are particularly convenient for creating constrained peptide substrates. However, one can envisage other appropriate chemistries with which to "cyclise" the indicator molecule, such as a peptide. In the case of peptides containing thiols (eg: in the form of cysteine), a simple disulphide bond formation or a diepoxide derivative can be used to affect covalent closure of the structure. Another appropriate chemistry includes the "click chemistry" method, involving the cycloaddition reaction between azides and alkynes forming stable triazoles. Here for example a peptide bearing two azido lysine amino acids could be intramolecularly cross linked by a dialkyne reagent. Such reactions can be catalysed by copper. However, in some examples such as those where a strained alkyne is used, no catalyst is required. A further chemical route includes that of stable hydrazone formation. Indicator molecules (in particular peptides) containing two phenyl hydrazine moieties may be cross linked intramolecularly via a dialdehyde reagent. A further chemical route is possible through peptide-based indicator molecules containing two tyrosine amino acids. These peptides can be intramolecularly crosslinked using a bis(diazo) scaffold to form the corresponding diazo adduct.

The scaffold molecules may also include further functionalities or reactive groups to facilitate generation of a novel binding site following enzymatic cleavage of the cleavage site. Thus, following cleavage at the cleavage site there are at least two parts of the cleavage region of the indicator molecule which are no longer connected to one another through the cleavage site. One or more of those "free" parts may become further constrained by interaction with the scaffold molecule. This may produce a significant change in structure of the overall molecule. This in turn permits specific binding molecules to be generated which will not cross-react with the indicator molecule prior to cleavage. Thus, by way of example, in the case of peptides constrained by a scaffold molecule one can envisage a specific conformational change after cleavage of the cleavage site. The afforded degrees of freedom in the peptide chain may allow it to self-assemble via non covalent interactions in a new stable conformation, creating a new conformational epitope unique to the molecule and recognised by the binding molecule (such as an antibody raised against the cleaved substrate). These non-covalent interactions may comprise hydrophobic interactions between the amino acid side chains and the aromatic rings in the scaffold molecule. The non-covalent interactions can be further enhanced in scaffolds with extended substitution patterns such that for example a negatively charged nitro substituent can interact with positively charged amino acids such as lysine, arginine or histidine included within the cleavage region. Hydrogen bond interactions are also possible between methoxy and/or hydroxyl aryl substituents and a number of amino acids, including serine, threonine and tyrosine. In addition, the two cleaved peptide parts of the cleavage region may be free to self-assemble with each other inducing a secondary structure such as a helix or beta stranded structure after cleavage. In further embodiments, a combination of both peptide-peptide interactions and peptide-scaffold interactions, as described above, may produce a novel binding site recognised by a binding molecule. Such interactions serve to differentiate the structure in 3 dimensional space between its uncleaved "closed" form and its "open" form following cleavage and hence significantly enhance the specificity of interaction between the cleaved indicator molecule and the binding molecule (e.g. an antibody raised against the cleaved peptide product). The resulting high specificity of interaction is beneficial to the sensitivity of detection of enzyme cleavage activity within the sample because it facilitates use of the indicator molecule in excess without the risk of the binding molecule binding to uncleaved indicator molecule (e.g. the antibody raised against the cleaved peptide from binding to the uncleaved peptide).

The scaffold should not prevent cleavage at the one or more cleavage sites. In some embodiments, the scaffold may orientate the (cleavage region of the) indicator molecule to optimise or improve efficiency of cleavage at the cleavage site. The scaffold may effectively fix or constrain the cleavage region to present the cleavage site in a favourable manner for the enzyme activity to be detected. The effect of the scaffold molecule on cleavage of any given substrate can readily be tested by a simple time course experiment. A test may determine whether cleavage occurs in the presence of the enzyme within a reasonable time (e.g. 5-10 minutes). This testing can be qualified, for example through mass spec analysis, optionally in combination with HPLC as it should evolve a new hydrolysed molecule (with a different molecular mass) which should also retain differently on a reverse phase analytical column. Those indicator molecules incorporating a scaffold molecule can, for example, then be prepared as an immunogen in its purified cleaved form. This can be used to raise antibodies in a suitable animal such as a sheep, either as free peptide or conjugated to a carrier protein. Antisera may then be characterised by ELISA to immobilised antigen and an antigen column may be used to affinity purify and refine the polyclonal response specifically to the cleaved indicator molecule. The complete indicator molecule may then be tested according to the methods of the invention.

A range of suitable binding molecules for use in the invention are disclosed herein, which discussion applies *mutatis mutandis* here. Typically, the binding molecule comprises an antibody (again as defined herein).

For the avoidance of doubt, these indicator molecules may be employed in any of the aspects of the invention (devices, kits, methods, uses etc.).

The one or more cleavage sites may be any site at which an enzymatically-cleavable bond is present. For example, this bond may be present between neighbouring residues of the indicator molecule. Such residues may be selected from nucleotides, monosaccharides, and amino acids. The indicator molecule typically comprises a peptide cleavage region. Thus, in some embodiments, the cleavage region comprises a sequence of amino acids. Preferably,, the cleavage site is a specific peptide bond located between two amino acid residues.

In further embodiments of the invention, the at least one cleavage site is located within a peptide, a protein, a carbohydrate, a lipid or a nucleic acid cleavage region. In certain embodiments, the indicator molecule may be engineered such that it comprises the enzyme's natural substrate or a portion thereof, such that the enzyme is presented with its native cleavage site, optionally in its native state within the cleavage region. In certain other embodiments, the indicator molecule may be engineered such that it comprises an artificial or non-native cleavage site and/or substrate region. For example, the cleavage site in the indicator molecule may be engineered or mutated such that the rate of cleavage activity or specificity of cleavage activity exhibited by the enzyme is increased (or decreased) relative to the rate and/or specificity of cleavage activity of the enzyme measured under comparable conditions against the enzyme's natural substrate.

In certain embodiments of the invention, the cleavage region may comprise multiple cleavage sites, wherein cleavage at any one of the sites produces at least two parts of the cleavage region, at least one part of which remains connected to the capture site. In the context of the present invention, the term 'multiple' means at least two, at least three, at least four, and so forth. In certain embodiments, the cleavage region of the indicator molecule includes between 2, 3, 4, 5 and 10, 11, 12, 13, 14, 15, 16, 17, 18, 19, 20, 25, 50, 100, 500 or 1000 cleavage sites. In some embodiments, the indicator molecule includes between 2 and 5, 6, 7, 8, 9 or 10 cleavage sites.

In one embodiment, the multiple cleavage sites may all be identical. In this configuration, the repeated cleavage site may be relatively non-specific or may be highly specific for one enzyme or enzyme subtype as defined above. Moreover, use of an indicator molecule of this type may help to increase the sensitivity of the enzyme detection device by providing a means to increase the concentration of cleavage sites present within the test sample.

In other embodiments, the cleavage region of the indicator molecule may comprise multiple cleavage sites wherein there are at least two different cleavage sites present within the same indicator molecule. In preferred embodiments of the invention, the indicator molecule may comprise at least three, at least four, at least five, and up to at least 8 different cleavage sites.

In a further preferred embodiment, the different cleavage sites are recognised by different enzymes or different categories, subcategories or subtypes of enzymes as defined above, such that the device can be used to detect the activity of multiple different enzymes. This is particularly the case where multiple effector molecules are measured according to the invention. The activities may be grouped, such that the detection of enzyme activity gives a useful result. For example, a group of MMPs (e.g. MMP 8 and 9) may be involved in an exacerbation event such that detection of the relevant activity of one or more of the enzyme group is useful for selecting a treatment to be administered to a patient suffering from an exacerbation of inflammation.

Use of multiple cleavage sites (whether identical or non-identical) may be particularly useful for situations in which very low levels of enzyme activity are to be detected in a test sample. For example, an indicator molecule having multiple cleavage sites as defined above may be used to detect enzyme activity in a blood sample containing low levels of protease. Use of multiple cleavage sites may also be particularly applicable where the indicator molecule incorporates a scaffold molecule.

In addition to a cleavage region containing at least one cleavage site, the indicator molecule comprises a capture site. The capture site mediates binding of the indicator molecule to a capture molecule present within a capture zone. Thus, the capture site is the portion of the indicator molecule responsible for retaining or localising the indicator molecule within the capture zone. Following cleavage of the indicator molecule, the capture site may remain intact or substantially intact, such that the site is still recognised and bound by a capture molecule present within the capture zone of the device. Under these circumstances, both intact indicator molecules and the part of the indicator molecules comprising the capture site following cleavage will be bound to capture molecules within the capture zone. The capture site may comprise any suitable molecule, for example a biotin molecule. It is also possible for the scaffold molecule to form a part, or the entirety, of the capture site in order to permit immobilization of the indicator molecule at a capture zone. For example, the capture zone may comprise antibodies raised against the scaffold molecule, preferably in the form as attached to the indicator molecule. In these embodiments, the scaffold molecule is not substantially involved in binding to the binding molecules. Key to effectiveness of the indicator molecules is immobilization via the interaction between capture site and capture molecules at the capture zone and simultaneous binding by binding molecules after cleavage has occurred. In those embodiments in which the scaffold molecule defines a part of the binding site for the binding molecules after cleavage, the capture site must be sufficiently distinct to prevent either or both binding events from being impeded.

As noted above, the cleavage site may be within a peptide, a protein, a carbohydrate, a lipid or a nucleic acid cleavage region. In specific embodiments of the invention, the cleavage region and capture site are defined by discrete amino acids or groups of amino acids within a peptide or protein. As used herein the term "peptide" is intended to mean a length of amino acids of no more than (about) 20, 30, 40 or 50 amino acids.

Alternatively, the capture site may be present in a region of the indicator molecule which is separate to the region in which the cleavage site is located. Thus, in certain embodiments of the invention, the capture site may be present within a capture region, and the cleavage site may be present within a separate cleavage region of the indicator molecule. In embodiments wherein the capture site is in a separate region of the indicator molecule to the cleavage site, the capture site may comprise materials or residues entirely distinct from those found in the region of the molecule containing the cleavage site. For example, the cleavage region may comprise amino acid residues whilst the capture site may comprise or consist of a biotin moiety. Moreover, in embodiments wherein the indicator molecule comprises separate regions bearing the cleavage site and capture site, said regions may be associated by any means known to one of skill in the art. In a preferred embodiment, said regions may be associated via a direct covalent linkage. Said regions may be immediately adjacent or may be separated by a linker or spacer, for example, a polyethylene glycol moiety.

The enzymes to be detected must be capable of cleaving the indicator molecule at the cleavage site. This activity is required in order for the indicator molecule to be cleaved at the cleavage site, to produce at least two parts of the cleavage region of the indicator molecule, at least one part of which remains connected to the capture site.

Within the context of the present invention the indicator molecules (via the capture site) may bind to the capture molecules with relatively high affinity. In some embodiments, the dissociation constant (kd) for the indicator molecule will be relatively low and preferably between 1 × 10⁻¹⁷M and 1 × 10⁻⁷M (depending on the sensitivity required of the assay). In certain embodiments of the invention, the dissociation constant for the indicator molecule will be between 1 × 10⁻¹⁵M and 1 × 10⁻⁹M.

In certain embodiments of the invention, such a binding interaction may be achieved as a result of direct binding of the capture site of the indicator molecule to the capture molecule present in the capture zone. In this context, direct binding means binding of the indicator molecule (via the capture site) to the capture molecule without any intermediary.

In some embodiments of the invention, the capture site of the indicator molecule and the capture molecule present in the capture zone are two halves of a binding pair. In this context, a binding pair consists of two molecules or entities capable of binding to each other. In certain embodiments of the invention, the binding interaction is specific such that each member of the binding pair is only able to bind its respective partner, or a limited number of binding partners. Moreover, as detailed above, it is preferable for the binding pair to exhibit relatively high affinity. The binding pair may be a binding pair found in nature or an artificially generated pair of interacting molecules or entities.

In some embodiments of the invention, the capture site of the indicator molecule and the capture molecule are two halves of a binding pair wherein the binding pair is selected from the following:- an antigen and an antibody or antigen binding fragment thereof; biotin and avidin, streptavidin, neutravidin or captavidin; an immunoglobulin (or appropriate domain thereof) and protein A or G; a carbohydrate and a lectin; complementary nucleotide sequences; a ligand and a receptor molecule; a hormone and hormone binding protein; an enzyme cofactor and an enzyme; an enzyme inhibitor and an enzyme; a cellulose binding domain and cellulose fibres; immobilised aminophenyl boronic acid and cis-diol bearing molecules; and xyloglucan and cellulose fibres and analogues, derivatives and fragments thereof.

In particular embodiments of the invention, the binding pair consists of biotin and streptavidin. In a further embodiment of the invention, the capture site of the indicator molecule comprises an epitope and the capture molecule comprises an antibody, which specifically binds to the epitope present at the first capture site. In the context of the present invention, the term antibody covers native immunoglobulins from any species, chimeric antibodies, humanised antibodies, F(ab')2 fragments, Fab fragments, Fv fragments, sFv fragments and highly related molecules such as those based upon antibody domains which retain specific binding affinity (for example, single domain antibodies). The antibodies may be monoclonal or polyclonal. Thus, in specific embodiments, the capture molecule comprises an antibody. In other embodiments, the capture site comprises a biotin molecule and the capture zone comprises a streptavidin molecule.

In certain embodiments of the invention, binding of the capture site of the indicator molecule to the capture molecule of the device may be indirect. In the context of the present invention, "indirect binding" means binding mediated by some intermediate entity capable of bridging the capture site of the indicator molecule and the capture molecule, for example an "adaptor" capable of simultaneously binding the capture site of the indicator molecule and the capture molecule.

Wherein binding of the indicator molecule to the capture molecule is indirect and mediated by an adaptor, it may be possible for a plurality of indicator molecules to bind to each capture molecule. In this context, a plurality means at least two, at least three, at least four, and so forth. This may be achieved by the incorporation of a multivalent adaptor molecule, for example, a streptavidin molecule capable of simultaneous binding to multiple biotin-containing indicator molecules in addition to a capture molecule consisting of or comprising biotin.

Embodiments of the device wherein a plurality of indicator molecules bind to each capture molecule, may be used to achieve improved assay accuracy as described in greater detail herein.

Another key molecule to this implementation of the invention is the binding molecule. The invention relies upon binding molecules capable of binding to the novel binding site produced on cleavage, or the part of the indicator molecule containing the capture site following cleavage, wherein the binding molecules are incapable of binding to the indicator molecule unless and until cleavage has occurred. Thus, in specific embodiments, the binding molecule comprises an antibody. For the avoidance of doubt, the term antibody covers native immunoglobulins from any species, chimeric antibodies, humanised antibodies, F(ab')2 fragments, Fab fragments, Fv fragments, sFv fragments and highly related molecules such as those based upon antibody domains which retain specific binding affinity (for example, single domain antibodies). The antibodies may be monoclonal or polyclonal. The inventors have produced antibodies which recognise the cleavage region only after cleavage and will therefore not bind to the indicator molecule (to any significant degree) unless and until cleavage at the cleavage site has occurred. Antibodies may be produced according to techniques known in the art. This may rely upon immunisation of an animal, such as a sheep, rabbit or goat, with the cleavage products. For example, immunisation may be performed using the part of the cleavage region which remains connected to the capture site after cleavage, optionally including the capture site itself. Polyclonal antibodies may be isolated from serum and affinity purified. Monoclonal antibodies may be produced using well-known and characterised hybridoma technology. The binding molecule may also comprise an aptamer in some embodiments.

Thus, described herein is a binding molecule, typically an antibody, which binds to an indicator molecule as defined herein after cleavage. Described herein is a binding molecule, typically an antibody, which binds to a novel binding site in the indicator molecule produced as a result of cleavage wherein the binding molecule is incapable of binding to the indicator molecule unless and until cleavage has occurred. In some embodiments, the binding molecule binds in the cleavage region. In specific embodiments, cleavage of the at least one cleavage site produces at least two parts of the cleavage region of the indicator molecule, at least one part of which remains connected to the capture site and as a consequence of cleavage contains a binding site for binding molecules and wherein the binding molecules are incapable of binding to the binding site unless and until cleavage has occurred. In some embodiments, cleavage of the at least one cleavage site produces two separate parts of the indicator molecule and thus the binding molecule binds to one or both of the separate parts following cleavage. In agreement with this, described herein is a binding molecule, optionally an antibody, which binds to an indicator molecule comprising the amino acid sequence GPQG but not to an indicator molecule comprising the amino acid sequence GPQGIFGQ (SEQ ID NO: 1) (as the cleavage region). Similarly, described herein is a binding molecule, optionally an antibody, which binds to an indicator molecule comprising the amino acid sequence IFGQ but not to an indicator molecule comprising the amino acid sequence GPQGIFGQ (SEQ ID NO: 1) (as the cleavage region).

In those embodiments of the invention in which the indicator molecule is structurally constrained and in which cleavage of the at least one cleavage site produces at least two parts of the cleavage region of the indicator molecule which remain connected to one another, the binding molecules may bind to the cleavage region following cleavage. In specific embodiments, the binding molecules bind to both parts of the cleavage region of the indicator molecule following cleavage. Thus, the binding molecules may bind a region that effectively spans the cleavage site following cleavage. Structural constraint of the indicator molecule, for example using the scaffold molecules as discussed herein, provides a well-defined and stable binding site for the binding molecules following cleavage. In specific embodiments, the binding site to which the binding molecule binds represents a novel structural conformation of the indicator molecule. Cleavage may produce at least one new conformational epitope. The binding site for the binding molecule may comprise any part of the indicator molecule. This may be with the proviso that enzyme cleavage activity and/or capture of the indicator molecule are not substantially impeded by binding of the binding molecule. In certain embodiments, the binding site comprises at least a portion of the cleavage region and/or at least a portion of the linker or spacer region to which the scaffold molecule is attached and which separates the scaffold molecule from the cleavage region. In other embodiments, the binding molecule may bind to a novel binding site that comprises at least a portion of the scaffold molecule.

The binding molecule may be directly or indirectly labelled with a reporter molecule to permit detection of binding of the binding molecule to the indicator molecule. The reporter molecule may be any substance or moiety suitable for detection by any means available to those skilled in the art. Thus, the reporter molecule is typically capable of signal generation or production. In certain embodiments of the invention, the reporter molecule is selected from the following: - a gold particle; a chromogen; a luminescent compound; a fluorescent molecule; a radioactive compound; a visible compound; a liposome or other vesicle containing signal producing substances; an electroactive species; or a combination of enzyme and its substrate. A suitable enzyme-substrate combination for use as a reporter moiety may be the enzyme alkaline phosphatase and the substrate nitro blue tetrazolium-5-bromo-4-chloro-3-indolyl phosphate. In a particular embodiment of the invention, the reporter moiety is a gold particle.

Indirect labelling of the binding molecules with a reporter molecule is also envisaged within the present invention. Thus, the reporter molecule may be attached to a further binding molecule which in turn binds to the binding molecule to provide the label. This indirect binding may be mediated by an adaptor capable of simultaneously binding the binding molecule and the reporter molecule. As an illustrative embodiment, where the binding molecule is an antibody, indirect labelling could be mediated by a further antibody that binds to the antibody binding molecule in specific fashion. The further antibody may be directly labelled with a reporter molecule such as a gold particle; a chromogen; a luminescent compound; a fluorescent molecule; a radioactive compound; a visible compound; a liposome or other vesicle containing signal producing substances; an electroactive species; or a combination of enzyme and its substrate. A suitable enzyme-substrate combination for use as a reporter moiety may be the enzyme alkaline phosphatase and the substrate nitro blue tetrazolium-5-bromo-4-chloro-3-indolyl phosphate. In a particular embodiment of the invention, the reporter moiety is a gold particle.

In embodiments of the invention wherein the reporter molecule binds to the binding molecule by virtue of an adaptor molecule, the adaptor may be pre-complexed with the binding molecule prior to the addition of the test sample to the indicator molecule, provided that the adaptor does not prevent binding of the binding molecule to the cleaved indicator molecule.

The adaptor may be any material or molecule capable of mediating the indirect interaction of the binding molecule with the reporter molecule. In some embodiments, the adaptor is streptavidin and the binding molecule comprises a biotin molecule. The adaptor may also be an "adaptor binding pair" wherein said binding pair comprises:
(i) a first member capable of binding to the binding molecule; and
(ii) a second member capable of binding to the first member of the pair and to the reporter molecule. In certain embodiments of the invention, the detection region of the indicator molecule comprises biotin, the first member of the adaptor binding pair is avidin or streptavidin, the second member of the adaptor binding pair is biotin, and the reporter molecule comprises a moiety capable of binding biotin.

The inclusion of an adaptor molecule or an adaptor binding pair may facilitate the binding of multiple reporter molecules to each binding molecule. For example, the use of multivalent streptavidin as the adaptor will allow for simultaneous binding of both a biotin-containing binding molecule in addition to multiple biotin-containing reporter molecules.

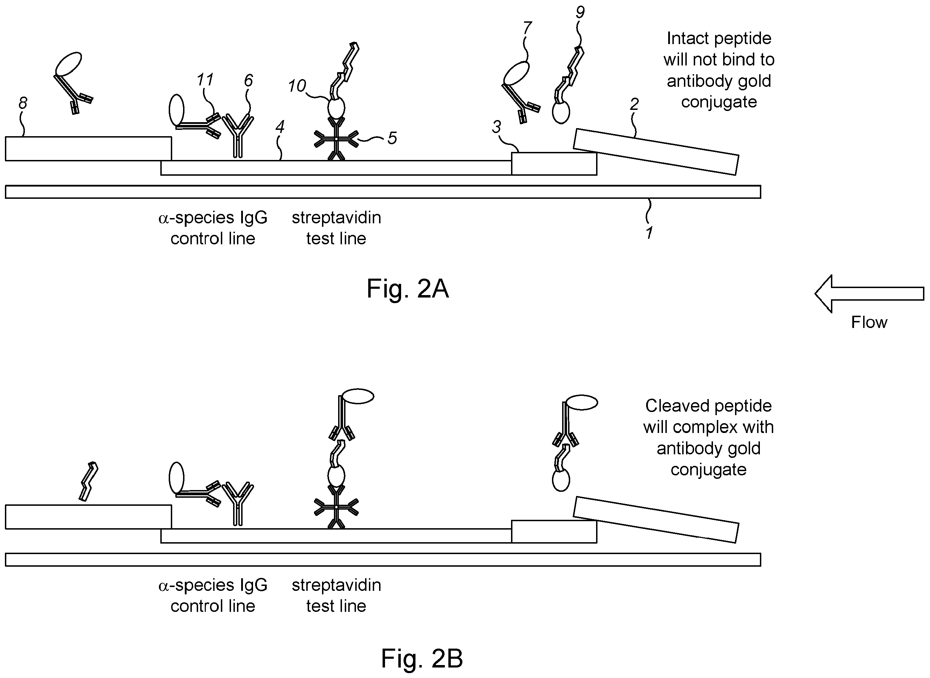

The invention may be performed in lateral flow or vertical flow devices in certain embodiments. Generally, therefore, the invention (or one or more detection devices) may rely upon some form of solid support. The solid support may define a liquid flow path for the sample. In specific embodiments, the solid support comprises a chromatographic medium or a capillary flow device. The invention may be provided in a test strip format in some embodiments. A representative example is shown in Figure 2 and described in further detail herein. The invention may also be provided in a two test strip format in some embodiments. The invention may also be provided in a four test strip format in some embodiments. In particular embodiments, the solid support may comprise one or more microfluidics channels (which may be in a test strip format).

In specific embodiments of the invention, the capture zone is formed on a solid support. Any support to which the capture molecules may be attached to form a capture zone is intended to be encompassed. The solid support may take the form of a bead (e.g. a sepharose or agarose bead) or a well (e.g. in a microplate) for example. Thus, in certain embodiments the device comprises a solid support to which the capture molecules are attached to form the capture zone. In the case of the kits of the invention, the solid support may be provided without the capture molecules attached. In those embodiments, the user of the kit may immobilize the capture molecules on the solid support to form the capture zone prior to use of the device with a test sample. The kit may, therefore, also comprise means for immobilizing the capture molecules on the solid support. The immobilizing means may comprise any suitable reagents to permit the capture zone to be formed. The solid support may be pre-formed with suitable immobilizing means. For example, the solid support may comprise biotin molecules arranged to interact with avidin (e.g. streptavidin) molecules that form (part of) the capture molecules. Of course, other binding pair interactions may be used to immobilize the capture molecules on the solid support to form a capture zone, as discussed herein and as would be readily understood by one skilled in the art.

The capture zone may be defined by the immobilization therein or thereon of capture molecules capable of binding to the capture site of indicator molecules. Immobilization of capture molecules may be achieved by any suitable means. Wherein the device is a flow device comprising a chromatographic medium, the capture molecules may be immobilized by directly binding to the medium or immobilized indirectly via binding to a carrier molecule, such as a protein, associated with, or bound to, the medium.

In further embodiments, the solid support further comprises a sample application zone to which the sample is applied. The sample application zone may be pre-loaded with the indicator molecule, such that when the test sample is applied any enzyme in the sample acts upon the cleavage site of the indicator molecule within the sample application zone. The sample application zone may contain a barrier, which holds the sample in the sample application zone for a pre-determined period of time. This permits the sample to interact with the indicator molecule for a sufficient period to achieve measurable levels of cleavage. This may be 1, 2, 3, 4, 5, 6, 7, 8, 9, 10, 15, 20, 30, 60 minutes or more depending upon the enzyme to be detected, as would be readily understood by one skilled in the art. The barrier may be degraded by the sample, or otherwise removed, after this period of time thus allowing the sample to continue to flow through the device. Alternatively, the test sample and indicator molecule may be pre-mixed or pre-incubated prior to adding the mixture to the device, such as to the sample application zone. However, where the test sample and indicator molecule may be pre-mixed or pre-incubated it is possible to omit the sample application zone. Here, it may be possible to add the mixture directly to the capture zone to permit immobilization of the indicator molecules through interaction with the capture molecules. In some embodiments, the test sample may be applied to the chromatographic medium at a site upstream from the capture zone such that it is drawn, for example by capillary action, through the capture zone. The chromatographic medium may be made from any material through which a fluid is capable of passing, such as a fluidic channel or porous membrane. In certain embodiments of the invention, the chromatographic medium comprises a strip or membrane, for example a nitrocellulose strip or membrane.

The binding molecules must be provided in the device in a manner that permits interaction with the indicator molecule, if cleaved at the cleavage site. The binding molecules may, therefore, be pre-mixed with the indicator molecules prior to application to the device. This may be before or after the indicator molecules have been mixed with the test sample. It is preferably after to avoid any effect the binding molecules may have on enzyme activity (in the test sample) at the cleavage site of the indicator molecule. The binding molecules can also be provided on or in the device at any point upstream of the capture zone, such that the binding molecules encounter the test sample and indicator molecules before the indicator molecules are immobilised (via interaction between the capture site of the indicator molecule and capture molecules defining the capture zone). Alternatively, the binding molecule may be added to the capture zone after the test sample and indicator molecules have been added to the capture zone. This ensures that any indicator molecule will already be immobilized at the capture zone, providing (in the case of cleaved indicator molecule) a binding site for the binding molecules to produce a signal.

Depending upon the particular enzyme cleavage activity that is being detected, it may be necessary to incorporate suitable enzyme inhibitors into the devices or methods. This may be important to prevent the enzyme from acting upon other components of the device or method, such as the binding molecules or capture molecules. Where the test sample is pre-incubated with the indicator molecule, it may be advantageous to add an inhibitor of the enzyme activity at the end of the incubation period. This is preferably before the binding molecules come into contact with the test sample. Alternatively, the enzyme activity inhibitor or inhibitors may be included in the device at any point upstream of the binding molecules, where the binding molecules are provided on or in the device. This is upstream of the capture zone (per the discussion herein above). The inhibitor may be simply dried or passively adsorbed onto the device such that the test sample mobilises the inhibitor as it passes through the device. It should be noted that use of an inhibitor is not essential and may be excluded where the inhibitor would result in an inability to detect a further marker in the blood. For example, some of the enzyme activities detected according to the invention such as specific protease activity may be sufficiently specific that the protease will not act on any other components of the device or method than the substrate. The cleavage sites of particular enzymes are well known in the art and can be used to design the various components of the devices and methods. For example, *in silico* screening may be performed (e.g. using freely available tools such as BLAST according to standard settings) to confirm that the cleavage site of the enzyme to be detected is not contained within any of the relevant molecules; such as the binding molecules and capture molecules. It is also possible to check for cross-reactivity by incubating the relevant molecules (e.g. binding molecules and capture molecules) with the enzyme activity to be tested and detecting whether cleavage occurs. In some embodiments, the relevant molecules will not be acted upon due to the nature of the enzyme cleavage activity to be detected. As an example, if a nuclease activity is being detected, this should not display any cleavage activity in relation to an antibody binding molecule or streptavidin or antibody capture molecule.

The solid support may further comprise a control zone, downstream of the capture zone in relation to sample flow, and the sample application zone if present, containing further binding molecules which bind to the binding molecules to indicate successful completion of an assay using the device. Alternatively, the further binding molecules may bind to a further molecule added to the sample or to the device and which flows with the sample through the device. The further molecule may be labelled, either directly or indirectly, with a reporter molecule as defined herein. Preferably, the reporter molecule is the same reporter molecule as attached to the binding molecules, for ease of detection, although it may be different. The control zone is spatially separated from the capture zone, for example to produce two separate test lines if the reporter is bound or immobilized in each respective zone. This control zone is used to confirm that the test sample, including the binding molecules, has passed through the entire device and confirms that the device is operating correctly. A positive signal is expected at the control zone independent of whether enzyme cleavage activity is present in the sample or not. The further binding molecules are selected based upon the nature of the binding molecules which bind to the cleavage site of the indicator molecules or on the nature of the further molecule added to the sample. The binding molecules and further binding molecules or further molecules and further binding molecules may form a binding pair as defined herein. For example, if the binding molecule is a species specific antibody (e.g. a sheep antibody), the further binding molecule may be an anti-species antibody (e.g. an anti-sheep antibody). Alternatively, if the further molecule is an antibody from a different species, e.g. a chicken or a goat, the further binding molecule may be an appropriate anti-species antibody. This permits immobilization of the binding molecule or further molecule at the control zone by virtue of a specific interaction. The further binding molecules may be immobilized in the control zone by any suitable means, for example by a covalent or non-covalent interaction.

Some examples of suitable assay formats useful for particular markers are outlined in the table below:

| | **Assay** | **Full Marker Name** | **Supplier** | **Catalogue Number** | **Assay type** | **Sample dilution** |
|---|---|---|---|---|---|---|
| 1 | CRP | C reactive protein | R&D systems | DY1707 | ELISA | 1:100K |
| 2 | MPO | Myeloperoxidase | R&D systems | DY3174 | ELISA | 1:750 |
| 3 | MMP9 | Total Matrix Metalloproteinase -9 | R&D systems | DY911 | ELISA | 1:1000 |
| 4 | NGAL | Neutrophil gelatinase-associated lipocalin | R&D systems | DY1757 | ELISA | 1:100 |
| 5 | Periostin | Periostin | R&D systems | DY3548b | ELISA | 1:1000 |
| 6 | Calprotectin | Calprotectin | Biolegend | 439707 | ELISA | 1:200 |
| 7 | RNASE 3 | Eosinophil cationic protein | Cloud-clone | SEB758Hu | ELISA | 1:200 |
| 8 | MBP | Major Basic protein | Cloud-clone | SEB650Hu | ELISA | 1:10 |
| 9 | Active MMP | Active protease (Composite MMP 2,8,9,12,13,7) | ENZO | BML-P276-001 | Substrat e assay | 1:40 |
| 10 | HNE | Human Neutrophil Elastase | Mologic | BHNEV1 | ELISA | 1:100 |
| 11 | Fibrinogen | Fibrinogen | Abcam | 108841 | ELISA | 1:200 |
| 12 | SLPI | Secretory leukocyte protease inhibitor | Mologic | In-house developed | ELISA | 1:100 |
| 13 | IL-6 | Interleukin-6 | R&D systems | DY206 | ELISA | 1:2 |
| 14 | Fibrinogen | Fibrinogen | Mologic | In-house developed | ELISA | 1:2000 |
| 15 | fM LP | N-Formylmethionine-leucyl-phenylalanine | Mologic | BFMLPV1 | Lateral Flow | 1:10 |
| 16 | Desmosine | Desmosine | Mologic | BDESV1 | ELISA | 1:5 |
| 17 | CC16 | Club cell- 16 | R&D systems | DY4218 | ELISA | 1:50 |
| 18 | TIMP1 | Tissue inhibitor of metalloproteinase-1 | R&D systems | DY970 | ELISA | 1:600 |
| 19 | TIMP2 | Tissue inhibitor of metalloproteinase-2 | R&D systems | DY971 | ELISA | 1:600 |
| 20 | CHI3L1 | Chitinase 3 like 1 protein | R&D systems | DY2599 | ELISA | 1:500 |
| 21 | A1AT | Alpha-1 antitrypsin | Mologic | BA1ATV1 | ELISA | 1:200K |
| 22 | Ac-PGP | N-acetyl Proline-Glycine-Proline | Mologic | In-house developed | ELISA | 1:10 |
| 23 | B2M | beta 2 Microglobulin | Abcam | 108885 | ELISA | 1:1000 |
| 24 | B2M | beta 2 Microglobulin | Mologic | In-house developed | ELISA | 1:1000 |
| 25 | Cystatin C | Cystatin C | R&D systems | DY1196 | ELISA | 1:1000 |
| 26 | MMP8 | Total Matrix Metalloproteinase -8 | R&D systems | DY908 | ELISA | 1:1000 |
| 27 | RBP4 | Retinol binding protein-4 | R&D systems | DY3378 | ELISA | 1:100K |
| 28 | HSA | Human serum Albumin | R&D systems | DY1455 | ELISA | 1:100K |
| 29 | A1AT | Alpha-1 antitrypsin | Mologic | BA1ATLF | Lateral Flow | 1:200K |
| 30 | IL-1b | Interleukin-1β | R&D systems | DY201 | ELISA | 1:2 |
| 31 | IL-8 | Interleukin- 8 | R&D systems | DY208 | ELISA | 1:2 |
| 32 | Desmosine Fragment | Desmosine Fragment | Mologic | In-house developed | ELISA | 1:5 |
| 33 | Large Elastin Fragment | Large Elastin Fragment | Mologic | In-house developed | ELISA | 1:5 |
| 34 | Siglec 8 | Siglec 8 | Mologic | In-house developed | ELISA | neat |
| 35 | sRAGE | Soluble receptor for advanced glycation end products | Mologic | In-house developed | ELISA | neat |
| 36 | EDN (RNASE2) | Eosinophil-derived neurotoxin | Alpco | 30-EDNHU-E01 | ELISA | 1:20 |
| 37 | IgE | Immunoglobulin E | Invitrogen | 88-50610-88 | ELISA | 1:20 |
| 38 | PCT | Procalcitonin | Mologic | In-house developed | ELISA | 1:2 |
| 39 | Lactoferrin | Lactoferrin | Mologic | In-house developed | ELISA | 1:50 |
| 40 | SuPAR | Soluble urokinase-type plasminogen activator receptor | Elabscien ce | E-EL-H2584 | ELISA | 1:500 |
| 41 | LTB4 | Leukotriene B4 | R&D systems | SKGE006B | ELISA | 1:5 |

The units for each assay shown in the table above are ng/ml, with the exception of IL-6, IL-1β and IL-8 which are all pg/ml.

Thus, it can be readily seen that ELISA and lateral flow formats are particularly applicable to the present invention. Zymography may be useful for certain markers.

The inventors have devised various assays for determining the levels of the markers described herein.

One marker useful in the present invention is N-acetyl Pro-Gly- Pro (Ac-PGP), a neutrophil chemoattractant, derived from the breakdown of extracellular matrix (ECM) and generated during airway inflammation. Ac-PGP is cleaved from collagen through the proteolytic action of neutrophil leucocytes in inflammatory diseases such as chronic obstructive pulmonary disease (COPD). According to the invention Ac-PGP may be detected by an enzyme immunoassay (EIA). In certain embodiments, the EIA is a competitive assay. Described herein is a competitive enzyme immunoassay for detecting Ac-PGP in a blood sample comprising:
(a) contacting the blood sample with an immunoassay surface on which is immobilised PGP (e.g. in the form of AHX-PGP or Ac-PGP)
(b) adding a reagent (such as an antibody, as defined herein, one specific example being CF1763) that specifically binds to PGP to the sample, which reagent is conjugated to an enzyme (such as alkaline phosphatase)
(c) removing reagent not bound to the immunoassay surface
(d) measuring the levels of enzyme activity at the immunoassay surface as an indication of the levels of Ac-PGP in the sample.

In the absence of Ac-PGP in the sample, the PGP immobilised on the immunocapture surface will be bound by the reagent and thus enzyme activity will be detected. As levels of Ac-PGP in the sample increase, these molecules will compete for binding to the reagent and thus will reduce levels of enzyme activity at the immunocapture surface. A preferred reagent is a sheep anti-Ac-PGP antibody CF1763. An alternative is CF1764. The reagent may be conjugated to alkaline phosphatase in some embodiments.

An alternative assay utilises an immobilised Ac-PGP binding reagent, such as an anti-Ac-PGP antibody (e.g. CF1763 - version 1 or CF1764 - version 2 as capture antibody). Here, the competing reagent may be B-AHX-PGP (biotinylated AHX-PGP) which competes with Ac-PGP in the sample. The third step then utilises streptavidin AP (streptavidin alkaline phosphatase) to label any B-AHX-PGP bound to the antibody capture line in the absence of 'free' Ac-PGP in the sample.

Ac-PGP may be detected in a lateral flow format in other embodiments, including by use of lateral flow as a format for the above referenced assays.

The degradation of elastin fibres during inflammation is caused by enzymes called elastases. Two important inflammatory elastases are neutrophil elastase (released by activated neutrophils) and MMP12 (released by lung macrophages). Desmosine is cleaved from elastin and is a molecular signature of the degradation process, indicating that leukocyte activity is elevated or rising. The amount of desmosine in the blood may correlate with the extent of elastin degradation which in turn is indicative of the level of tissue damage. Excess neutrophil leukocyte activity is a key driver of exacerbation. The inventors have developed desmosine fragment assays as well as Desmosine assays. The Described herein is an assay able to measure Desmosine as well as Desmosine still attached to elastin fibres. This format relies upon use of multiple antibodies raised to different sized elastin fragments resulting from cleavage by human neutrophil elastase. The desmosine fragments may be detected by an enzyme immunoassay (EIA). In certain embodiments, the EIA is a competitive assay. Described herein is a competitive enzyme immunoassay for detecting desmosine fragments in a blood sample comprising:
(a) contacting the blood sample with an immunoassay surface on which is immobilised desmosine fragments (e.g. through conjugation to an albumin molecule such as ovalbumin on the surface)
(b) adding a series of reagents (such as a group of antibodies, as defined herein, one specific example being CF1673, CF1674 and CF1675) that specifically bind to respective desmosine fragments in the sample, each of which reagents is conjugated to an enzyme (such as alkaline phosphatase)
(c) removing reagent not bound to the immunoassay surface
(d) measuring the levels of enzyme activity at the immunoassay surface as an indication of the levels of desmosine fragments in the sample.

In the absence of the desmosine fragments in the sample, the desmosine fragments immobilised on the immunocapture surface will be bound by the reagents and thus enzyme activity will be detected. As levels of desmosine fragments in the sample increase, these molecules will compete for binding to the reagent and thus will reduce levels of enzyme activity at the immunocapture surface. A preferred reagent series are sheep anti-desmosine fragment antibodies CF1673, CF1674 and CF1675. The reagents may each be conjugated to alkaline phosphatase in some embodiments. In some embodiments, the respective reagents in the series are utilised in separate individual assays, referred to herein as versions 1, 2, and 3. Elastin breakdown products can be purified by HPLC and used as immunogens to produce specific antibodies. The elastin fragments may be small elastin fragments. Small elastin fragments typically have a molecular weight of no more than 30,000 Da, such as between 1000 and 30,000 Da. Small elastin fragments not attached to desmosine may also, or separately, be measured in some embodiments.

Similarly, described herein is an assay for measuring large elastin fragments (LEF). By large elastin fragments is meant fragments of elastin with a molecular weight greater than around 30,000 Da. This format relies upon use of multiple antibodies raised to the large elastin fragments resulting from cleavage by human neutrophil elastase (also see Figure 39). Large elastin fragments may be detected by an enzyme immunoassay (EIA). In certain embodiments, the EIA is a competitive assay. Described herein is a competitive enzyme immunoassay for detecting large elastin fragments in a blood sample comprising:
(a) contacting the blood sample with an immunoassay surface on which is immobilised large elastin fragments (e.g. through conjugation to an albumin molecule such as ovalbumin on the surface)
(b) adding a series of reagents (such as a group of antibodies, as defined herein, such as CF1669, CF1670 and CF1673 (all purified against LEF)) that specifically bind to respective large elastin fragments in the sample, each of which reagents is conjugated to an enzyme (such as alkaline phosphatase)
(c) removing reagent not bound to the immunoassay surface
(d) measuring the levels of enzyme activity at the immunoassay surface as an indication of the levels of large elastin fragments in the sample.

In the absence of the large elastin fragments in the sample, the large elastin fragments immobilised on the immunocapture surface will be bound by the reagents and thus enzyme activity will be detected. As levels of large elastin fragments in the sample increase, these molecules will compete for binding to the reagent and thus will reduce levels of enzyme activity at the immunocapture surface. The reagents may each be conjugated to alkaline phosphatase in some embodiments.

In some embodiments, the respective reagents in the series are utilised in separate individual assays, referred to herein as versions 1, 2, and 3.

The inventors have also developed immunoassay formats to detect SLPI, fibrinogen, B2M, Siglec 8 and sRAGE.

Described herein is an enzyme immunoassay for detecting SLPI in a blood sample comprising:
(a) immobilising onto the immunoassay surface a first reagent (such as an antibody, as defined herein, one specific example being CF1099) that can specifically bind to SLPI
(b) removing first reagent not bound to the immunoassay surface
(c) adding the blood sample
(d) adding a second reagent (such as an antibody, as defined herein, one specific example being 431 as provided by Alere (now Abbott Laboratories)) that specifically binds to SLPI immobilised to the immunoassay surface, which reagent is conjugated to an enzyme (such as alkaline phosphatase)
(d) removing reagent not bound to the immunoassay surface
(e) measuring the levels of enzyme activity at the immunoassay surface as an indication of the levels of SLPI in the sample.

In the presence of SLPI in the blood sample, the SLPI will be immobilised on the immunoassay surface by the first reagent which will, in turn, by bound by the second reagent. Thus, enzyme activity (via the enzyme conjugated to the second reagent) will be detected. A preferred first reagent is a sheep anti-SLPI antibody CF1099. A preferred second reagent is a mouse anti-SLPI antibody 431 (as provided by Alere (now Abbott Laboratories)). The second reagent may be conjugated to alkaline phosphatase in some embodiments. Levels of SLPI in the blood sample may be calculated by reference to a standard curve prepared using known concentrations of recombinant SLPI.

Described herein is an enzyme immunoassay for detecting fibrinogen in a blood sample comprising:
(a) immobilising onto the immunoassay surface a first reagent (such as an antibody, as defined herein, one specific example being CF1765) that can specifically bind to fibrinogen
(b) removing first reagent not bound to the immunoassay surface
(c) adding the blood sample
(d) adding a second reagent (such as an antibody, as defined herein, one specific example being CF1766) that specifically binds to fibrinogen immobilised to the immunoassay surface, which reagent is conjugated to an enzyme (such as alkaline phosphatase)
(d) removing reagent not bound to the immunoassay surface
(e) measuring the levels of enzyme activity at the immunoassay surface as an indication of the levels of fibrinogen in the sample.

In the presence of fibrinogen in the blood sample, the fibrinogen will be immobilised on the immunoassay surface by the first reagent which will, in turn, by bound by the second reagent. Thus, enzyme activity (via the enzyme conjugated to the second reagent) will be detected. A preferred first reagent is a sheep anti-fibrinogen antibody CF1765. A preferred second reagent is a sheep anti-fibrinogen antibody CF1766. The second reagent may be conjugated to alkaline phosphatase in some embodiments. Levels of fibrinogen in the blood sample may be calculated by reference to a standard curve prepared using known concentrations of recombinant fibrinogen.

Described herein is an enzyme immunoassay for detecting B2M in a blood sample comprising:
(a) immobilising onto the immunoassay surface a first reagent (such as an antibody, as, defined herein, e.g. NS15 as sold by Ig Innovations) that can specifically bind to B2M
(b) removing first reagent not bound to the immunoassay surface
(c) adding the blood sample
(d) adding a second reagent (such as an antibody, as defined herein, one specific example being NS16 as sold by Ig Innovations) that specifically binds to B2M immobilised to the immunoassay surface, which reagent is conjugated to an enzyme (such as horseradish peroxidase)
(d) removing reagent not bound to the immunoassay surface
(e) measuring the levels of enzyme activity at the immunoassay surface as an indication of the levels of B2M in the sample.

In the presence of B2M in the blood sample, the B2M will be immobilised on the immunoassay surface by the first reagent which will, in turn, by bound by the second reagent. Thus, enzyme activity (via the enzyme conjugated to the second reagent) will be detected. A preferred first reagent is a sheep anti-B2M antibody NS15 as sold by Ig Innovations. A preferred second reagent is a sheep anti-B2M antibody NS16 as sold by Ig Innovations. The second reagent may be conjugated to horseradish peroxidase in some embodiments. Levels of B2M in the blood sample may be calculated by reference to a standard curve prepared using known concentrations of recombinant B2M.

Described herein is an enzyme immunoassay for detecting Siglec 8 in a blood sample comprising:
(a) immobilising onto the immunoassay surface a first reagent (such as an antibody, as defined herein, one specific example being SA122) that can specifically bind to Siglec 8
(b) removing first reagent not bound to the immunoassay surface
(c) adding the blood sample
(d) adding a second reagent (such as an antibody, as defined herein, one specific example being SA122) that specifically binds to Siglec 8 immobilised to the immunoassay surface, which reagent is conjugated to an enzyme (such as alkaline phosphatase)
(d) removing reagent not bound to the immunoassay surface
(e) measuring the levels of enzyme activity at the immunoassay surface as an indication of the levels of Siglec 8 in the sample.

In the presence of Siglec 8 in the blood sample, the Siglec 8 will be immobilised on the immunoassay surface by the first reagent which will, in turn, by bound by the second reagent. Thus, enzyme activity (via the enzyme conjugated to the second reagent) will be detected. A preferred first reagent is a sheep anti-Siglec 8 antibody SA122. A preferred second reagent is a sheep anti-Siglec 8 antibody SA122. The second reagent may be conjugated to alkaline phosphatase in some embodiments. Levels of Siglec 8 in the blood sample may be calculated by reference to a standard curve prepared using known concentrations of recombinant Siglec 8.

Described herein is an enzyme immunoassay for detecting sRAGE in a blood sample comprising:
(a) immobilising onto the immunoassay surface a first reagent (such as an antibody, as defined herein, one specific example being SA065) that can specifically bind to sRAGE
(b) removing first reagent not bound to the immunoassay surface
(c) adding the blood sample
(d) adding a second reagent (such as an antibody, as defined herein, one specific example being RA040) that specifically binds to sRAGE immobilised to the immunoassay surface, which reagent is conjugated to an enzyme (such as alkaline phosphatase) (d) removing reagent not bound to the immunoassay surface
(e) measuring the levels of enzyme activity at the immunoassay surface as an indication of the levels of sRAGE in the sample.

In the presence of sRAGE in the blood sample, the sRAGE will be immobilised on the immunoassay surface by the first reagent which will, in turn, by bound by the second reagent. Thus, enzyme activity (via the enzyme conjugated to the second reagent) will be detected. A preferred first reagent is a sheep anti-sRAGE antibody SA065. A preferred second reagent is a rabbit anti-sRAGE antibody RA040. The second reagent may be conjugated to alkaline phosphatase in some embodiments. Levels of sRAGE in the blood sample may be calculated by reference to a standard curve prepared using known concentrations of recombinant sRAGE.

The methods of the invention rely upon identifying a perturbed level (i.e. a (significant) change in the level) of markers in a blood sample. Typically, the change is determined for each marker by comparison with a "population level" for the marker i.e. a level derived from a subject population. The subject population may not be suffering from an exacerbation of inflammation of a respiratory condition (e.g. a PEx). Alternatively, the subject population may be suffering from an exacerbation of inflammation of a respiratory condition (e.g. a PEx). In particular embodiments, the subject population may comprise individuals not suffering from an exacerbation of inflammation of a respiratory condition (e.g. a PEx) as well as individuals who are suffering from an exacerbation of inflammation of a respiratory condition (e.g. a PEx). The population level may be considered a "threshold" or "cut-off value". Said subject population may be suffering from a respiratory disorder. More specifically, the respiratory disorder may be COPD. Alternatively, the subject population may be suffering from cystic fibrosis (CF) or asthma. By comparing the determined levels of the marker (in the test sample) with the population level for said marker, the determined marker levels will indicate whether the levels of eosinophils and/or neutrophils in the sample (depending on the marker) are high, normal or low. For instance, where the levels of a particular marker positively correlate with eosinophil and/or neutrophil levels, levels of the marker determined in a test sample which are above the population level will indicate high levels of eosinophils and/or neutrophils in the sample (depending on the marker). Conversely, where the levels of a particular marker negatively correlate with eosinophil and/or neutrophil levels, levels of the marker determined in a test sample which are below the population level will indicate high levels of eosinophils and/or neutrophils in the sample. For the purpose of the invention described herein, the cut off for determining whether the level of eosinophils is high or low may be 300 cells/µL, wherein the level of 300 cells/µL and above may be considered high and the level of below 300 cells/µL may be considered low. In addition, the cut off for determining whether the level of neutrophils is high or low may be 1000 cells/µL, wherein the level of 1000 cells/µL or above may be considered high and the level of below 1000 cells/µL may be considered low. In particular embodiments, the levels of the markers determined in a test sample are combined to compute a "risk score". The risk score takes into account the positive or negative or more complex correlations of each marker with eosinophil and/or neutrophil levels. If the risk score is above a population-derived threshold value (based on population-derived marker levels), this will indicate high levels of eosinophils and/or neutrophils in the samples and thus corticosteroids and/or antibiotics should be administered as the treatment for the exacerbation. Conversely, where the risk score is lower than the population-derived threshold value, this will indicate low levels of eosinophils and/or neutrophils in the sample. In some embodiments, a separate risk score may be calculated in respect of each of eosinophil levels and neutrophil levels respectively. Thus, a first risk score (eosinophil risk score) and population-derived (eosinophil) threshold value may be calculated in respect of the marker(s) of eosinophil levels (and one or more supporting markers of eosinophil levels if determined), and a second risk score (neutrophil risk score) and population-derived (neutrophil) threshold value may be calculated in respect of the marker(s) of neutrophil levels (and one or more supporting markers of neutrophil levels if determined). If the eosinophil risk score is above the population-derived eosinophil threshold value, this will indicate high levels of eosinophils in the samples and thus corticosteroids should be administered as the treatment for the exacerbation. If the neutrophil risk score is above the population-derived neutrophil threshold value, this will indicate high levels of neutrophils in the samples and thus antibiotics should be administered as the treatment for the exacerbation. If both the eosinophil risk score and the neutrophil risk score are above the respective population-derived threshold values, this will indicate high levels of eosinophils and neutrophils in the samples and thus antibiotics and corticosteroids should be administered as the treatment for the exacerbation.

Whilst determining whether the levels of a particular marker in a test blood sample are perturbed using a level derived from a subject population as described above is preferred, it is also possible in alternative embodiments that a comparison may be made between the levels of the marker in the test sample and at least one blood sample taken from the same subject at an earlier time point. This provides the ability to personalise the monitoring of inflammation status in order to accurately indicate whether an exacerbation correlates with high eosinophil levels and/or high neutrophil levels and therefore indicate the most appropriate treatment. Thus, according to all aspects of the invention perturbed levels of the at least one marker may be calculated with reference to a threshold level of the marker that is adapted (or personalised) to the subject. The invention may therefore rely upon a personalised baseline level of the relevant marker or markers against which the threshold is calculated. Calculation may be on an on-going basis to coincide with testing. Thus, the threshold may be a rolling threshold derived from the rolling baseline. In this context, it is apparent that levels of the marker or markers do not have to be measured in absolute terms and may be measured in absolute or relative terms. The markers simply have to be measured in a manner which permits a comparison to be made with marker levels in blood samples taken at different time points. Thus "level" should be interpreted accordingly in this context, unless indicated otherwise. For example, levels may be measured relative to a reference analyte present at a stable concentration in blood samples irrespective of exacerbation status.

In some embodiments, the threshold level of the marker is set by determining the levels of the marker in blood samples taken from the subject at earlier time points. In its simplest form, the invention may rely upon a simple comparison between the test sample and the level of the marker in the previously taken blood sample (i.e. a single earlier time point). However, typically, the earlier time points may comprise at least two, and possibly 3, 4, 5, 6, 7, 8, 9, 10 etc, earlier measurements immediately preceding the determination of the level of the marker in the current blood sample. Those earlier measurements may be taken over a period of days or weeks, such as 1, 2, 3, 4, 5 or 6 weeks or longer. The baseline may be set during a period of stable disease to determine the initial thresholds against which future changes are measured. Stable disease may initially be identified by routine methods. Alternatively or additionally, the baseline may be set during a period of exacerbation to determine the initial thresholds against which future changes are measured. An exacerbation may initially be identified by routine methods.

Where marker levels are measured at multiple time points those levels may be averaged to provide the threshold for the test sample. In some embodiments, the threshold may be set with reference to a sliding window within which levels of the markers have been measured to provide a baseline. The threshold level is thus "learned" by the system. It is not a fixed threshold and is adapted to the subject, thereby taking into account insignificant fluctuations in marker levels from the baseline. Accordingly, the threshold may be set around the baseline to specify an allowable range of the marker levels beyond which a statistically significant increase or decrease in level is indicated. In the presence of drift of the baseline level of the marker, it is possible that the parameter limits may be narrowed such that a further change in level of the markers is deemed significant. For example, if the baseline marker level is drifting upwards over time, the difference between a measured increase and baseline may need to be smaller (compared to the situation in which the baseline is relatively stable) to be considered to have exceeded the threshold (i.e. to be significant). For example, a difference from baseline of at least 5, 10, 15, 20% or more may be considered significant generally. This difference may be reduced if there have been multiple previous measurements displaying a trend upwards or downwards but in each case by an amount less than the threshold difference. The difference (in order to be considered significant) may thus be reduced to at least 1, 2, 3, 4, 5% or more as appropriate in the event of a drift upwards or downwards in the baseline.

Typically, the threshold level of the marker is set by determining the levels of the marker in blood samples taken from the subject at earlier time points at which the subject was not suffering from an exacerbation of inflammation. A treatment for the exacerbation is selected based upon observance of a statistically significant deviation from the baseline set with reference to the non-exacerbation levels. Thus, stable state levels may be measured on an individual basis to provide criteria for detecting meaningful changes in future monitoring.

In other embodiments, levels of at least one marker are determined at least twice a week. Marker levels may be determined at least 1, 2, 3, 4, 5, 6 times a week or daily in some embodiments. For the avoidance of doubt marker levels may be detected in a newly collected blood sample on each occasion.

The threshold is intended to permit detection both of a gradual move or "drift" towards elevated eosinophil and/or neutrophil levels at the onset of or during an exacerbation as well as a more sudden increase in eosinophil and/or neutrophil levels at the onset of or during an exacerbation. Thus, the threshold may be a rolling threshold personalised to the subject. It permits any significant (i.e. statistically significant) deviation from baseline in terms of the levels of the one or more markers to be detected. The baseline and calculated threshold may be adapted or trained in relation to previous exacerbation events suffered by the same subject. The baseline and threshold calculated therefrom may be set in relation to 1, 2, 3, 4, 5, 6, 7, 8, 9, 10 etc previous measurements taken by the subject. The threshold may be weighted towards more recent measurements as would be well understood by one skilled in the art.

The threshold may be set in relation to multiple markers as discussed in greater detail herein. Thus, the selection of a treatment for an exacerbation may be based upon a deviation from baseline that is cumulative according to the multiple markers measured. Typically, however, each marker will be measured individually with reference to a marker specific baseline and against a marker specific threshold. It is shown herein that use of multiple individual markers provides an improved ability to select a treatment for an exacerbation. This seems to be because in different individuals an exacerbation may be identified more accurately with different markers. Thus, the invention may rely upon a plurality of rolling baselines/thresholds depending upon the individual markers employed (typically three or more).

The methods and systems described herein may weight the contribution of a plurality of markers. Thus, additional weight in terms of selecting a treatment for an exacerbation may be given to elevation of more than one (be it 2, 3, 4, 5 etc.) markers, for example when measured in the same sample.

The thresholds may also be used to monitor the effectiveness of treatment of the exacerbation event. For example, in some embodiments, the frequency of determining the levels of the at least one marker in blood samples taken from the subject is increased if the levels of the at least one marker continue to be perturbed despite administration of a treatment (selected using the methods described herein). The frequency may be increased from weekly or twice weekly to daily or from daily to twice daily for example. In certain embodiments the frequency of determining the levels of the at least one marker in blood samples taken from the subject is maintained (at the increased level) until the levels of the at least one marker are no longer perturbed (i.e. they are considered stable based on a threshold or baseline or a population level as defined herein). Thus, the monitoring frequency may be maintained until an exacerbation has been successfully treated. In some embodiments, the monitoring frequency may be further increased during the treatment phase (e.g. to testing every 6, 8 or 12 hours for example).

The methodsmay rely upon determining levels of a plurality, such as at least two or three (or 4, 5, 6, 7, 8, 9, 10 or more) markers in blood samples taken from the subject at multiple time points.

Where levels of multiple markers are determined, a suitable algorithm may be employed in order to interpret the data and apply it to select the treatment for an exacerbation. In some embodiments, the marker levels may be inter-dependent and thus the algorithm is based on this predicted relationship (e.g. between protease and protease inhibitor molecules). In certain embodiments, the determined levels of the at least two or three (or more) markers are analysed in a pre-determined sequence. This may give rise to a decision tree, as explained further herein and shown in the figures, to select the most appropriate treatment for the subject based on indicated eosinophil and neutrophil levels. Thus, in some embodiments, for a given sample, the marker levels may be analysed in sequence until a marker is found with a perturbed level (or all markers have been examined). If a marker is detected at a perturbed level the further markers may or may not also be assessed to determine if their level is also perturbed. In some embodiments, the determined levels of the at least two or three (or more) markers are weighted. Weighting is a well-known method of applying a degree of relative significance to the multiple markers. The algorithm may be a threshold based algorithm as discussed herein. The algorithm may be designed to weight the markers based on whether each marker is a marker of eosinophil levels, a marker of neutrophil levels or a supporting marker of eosinophil and neutrophil levels, with greater weight given to markers of eosinophil levels and markers of neutrophil levels as compared with supporting markers of eosinophil and neutrophil levels. The algorithm may be designed in such a way that perturbed levels of one or more supporting markers of eosinophil and neutrophil levels do not indicate any particular treatment for the exacerbation in the absence of perturbed levels of one or more marker of eosinophil levels or one or more markers of neutrophil levels.

As already discussed, in some embodiments, levels of at least one marker are determined by normalising against the levels of a reference marker, also measured in blood. Suitable reference markers useful in the invention may include blood volume, conductivity, albumin levels, serum creatinine and total protein levels. Specific gravity and colour may be other normalising or reference markers.

In illustrative embodiments, relating to use of at least three markers, a perturbation in the levels of each of the at least three markers is used to select a treatment for the exacerbation. These embodiments may be applied *mutatis mutandis* to situations in which 2, 4, 5, 6, 7, 8, 9, 10 etc. markers are measured in blood samples as would readily be appreciated by the skilled person.

Treatments for an exacerbation are known in the art. They include use of inhalers, which may be bronchodilator inhalers (short or long acting). Short-acting bronchodilators include beta-2 agonist inhalers, such as salbutamol and terbutaline and antimuscarinic inhalers, such as ipratropium. Long acting bronchodilators include beta-2 agonist inhalers, such as salmeterol and formoterol and antimuscarinic inhalers, such as tiotropium. Steroid or corticosteroid inhalers may also be used. Further useful therapeutic agents include theophylline, mucolytics such as carbocisteine, antibiotics and steroids. Nebulisers may be employed. They may for example be employed in place of an inhaler where the exacerbation is not managed or does not improve through use of an inhaler. Such monitoring is encompassed by the present invention. Oxygen therapy or non-invasive ventilation may also be employed. Rehabilitation programmes involving physical exercise may also be utilised as appropriate. Again the invention permits monitoring of such programmes to determine whether they are having the desired effect in terms of stabilising the condition (against exacerbations).

According to the invention, in the case of neutrophil-driven exacerbations (i.e. exacerbations concurring with high levels of neutrophils), preferably antibiotics are selected to treat the exacerbation. Suitable antibiotics include macrolides (e.g. azithromycin, clarithromycin), cephalosporins (e.g. cefuroxime, cefpodoxime, cefdinir), ketolides (e.g. telithromycin), fluoroquinolones (e.g. moxifloxacin, gemifloxacin, levofloxacin), doxycycline, trimethoprim/sulfamethoxazole and amoxicillin/clavunate. These may be combined with one or more of the other treatments described above as appropriate.

According to the invention, in the case of eosinophil-driven exacerbations (i.e. exacerbations concurring with high levels of eosinophils), preferably corticosteroids are selected to treat the exacerbation. Suitable corticosteroids include beclomethasone dipropionate (e.g. Beclovent^{®}), beclomethasone dipropionate HFA (e.g. Qvar^{®}), budesonide (e.g. Pulmicort^{®}), flunisolide (e.g. AeroBid^{®}), fluticasone propionate (e.g. Flovent^{®}) and triamcinolone acetonide (e.g. Azmacort^{®}). Typically, these are inhaled using an inhaler or nebulizer. Other suitable corticosteroids include prednisolone (e.g. Prelone^{®}), prednisone (e.g. Deltasone^{®}) and methylprednisolone (e.g. Medrol^{®}). Typically, these are administered in oral form (e.g. in pill, tablet or liquid form). These may be combined with one or more of the other treatments described above as appropriate. In particular, corticosteroids may be combined with bronchodilators in a single dose. Suitable corticosteroid-bronchodilator combinations include salmeterol + fluticasone propionate (e.g. Advair^{®} Diskus), vilanterol + fluticasone furoate (e.g. Breo Ellipta^{®}), mometasone furoate + formoterol fumarate (Dulera^{®}) and budesonide + formoterol fumarate (Symbicort^{®}).

In some embodiments, if no perturbation in the levels of any of the markers is determined, the inflammation status is considered stable (i.e. no exacerbation) or of a COPD phenotype that does not comprise elevated levels of eosinophils and/or neutrophils. Therefore, no treatment is selected. In those circumstances the frequency of testing may be maintained (for example at a basal level).

In certain embodiments, if a perturbation in the level of one of the markers is determined but not in the other two markers the frequency of testing is increased. In specific embodiments, the frequency of testing is increased unless the perturbed level of one of the markers reverts to a non-perturbed level within a set number of repeat tests. That set number can be any suitable number. For example, it may be 1, 2, 3, 4 or 5 (or more). The increased frequency may be daily or twice daily for example.

In further embodiments, if the level of one of the markers reverts to a non-perturbed level within the set number of repeat tests, the frequency of testing reverts to the original frequency. The original frequency may be one to three times a week for example. Alternatively, it may simply be whenever the patient experiences a sudden decline in symptoms indicating onset of an exacerbation. In related embodiments, if the level of one of the markers remains at a perturbed level within the set number of repeat tests, the frequency of testing is increased further. That set number can be any suitable number. For example, it may be 1, 2, 3, 4 or 5 (or more). The further increased frequency of testing may be on a 6, 8 or 12 hourly basis for example.

In related embodiments, if the level of one (or more) of the markers reverts to a non-perturbed level within the further set number of repeat tests at increased frequency, the frequency of testing reverts to the increased (but not further increased) frequency of testing. That set number can be any suitable number. For example, it may be 1, 2, 3, 4 or 5 (or more). Thus, the invention may enable a step-down in frequency of monitoring where there has been a reversion in levels of the one or more markers. More generally, the invention permits stepping up and down of frequency of testing according to the data generated for the individual subject with a view to accurately managing that patient's treatment.

In specific embodiments, if the level of one (or more) of the markers remains at the non-perturbed level within the set number of repeat tests, the frequency of testing reverts to the original frequency. Thus, there may be a second step-down to the original testing protocol.

According to all of these exemplary embodiments, if a perturbation in the level of two of the markers is determined but not in the other marker (or markers if more than three are used) the frequency of testing may be increased. In specific embodiments, the frequency of testing is increased to a frequency greater than if a perturbed level in only one of the markers is detected. Thus, the algorithm may categorise a perturbed level of a plurality of markers as potentially more dangerous than a single marker and adjust the frequency of testing accordingly. This may be a double step-up in frequency of testing.

In some embodiments, if the level of at least one of the markers reverts to a non-perturbed level within the set number of repeat tests, the frequency of testing reverts to a frequency of testing indicative of a determined perturbation in the level of one of the markers. Thus monitoring may be flexible to allow a step-down in frequency to a level suitable for, or commensurate with perturbation of a single marker. However, in some embodiments, if the level of the one of the markers remains at a perturbed level within the set number of repeat tests (which may be 1, 2, 3, 4, 5, or more), the frequency of testing is increased again. This permits a persistent perturbation in a single marker to be monitored and the treatment can be appropriately monitored (e.g. continued, altered or stopped).

The methods, systems and test kits of the invention may be used in conjunction with monitoring other indicators of exacerbation of inflammation. In specific embodiments, the other indicators of exacerbation of inflammation comprise or are selected from one or more of shortness of breath, increased wheeze, increased pulse rate, dyspnoea, increased sputum purulence, increased sputum colour, sore throat, increased cough, cold and fever. Another indicator that may be monitored is Forced Expiratory Volume in one second (FEV₁).

From the foregoing, it is apparent that the nature of the methods of the invention requires significant computational input in order to define relevant cut-off values/thresholds, and to interpret marker levels against those cut-off values/thresholds. Thus, the methods of the invention typically incorporate suitable software to perform the relevant technical steps. Accordingly, the methods of the invention may be performed using systems or test kits as described herein.

The invention also relates to the computer applications used in the systems and test kits. Thus, in certain embodiments, the computer-implemented method, system, and computer program product may be embodied in a computer application, for example, that operates and executes on a processor, such as in the context of a computing machine. When executed, the application performs the relevant analyses to output the selected treatment for the subject suffering from an exacerbation.

As used herein, the processor may be comprised within any computer, server, embedded system, or computing system. The computer may include various internal or attached components such as a system bus, system memory, storage media, input/output interface, and a network interface for communicating with a network, for example.

The computer may be implemented as a conventional computer system, an embedded controller, a laptop, a server, a customized machine, any other hardware platform, such as a laboratory computer or device, for example, or any combination thereof. The computing machine may be a distributed system configured to function using multiple computing machines interconnected via a data network or bus system, for example.

The processor may be configured to execute code or instructions to perform the operations and functionality described herein, manage request flow and address mappings, and to perform calculations and generate commands. The processor may be configured to monitor and control the operation of the components in the computing machine. The processor may be a general purpose processor, a processor core, a multiprocessor, a reconfigurable processor, a microcontroller, a digital signal processor ("DSP"), an application specific integrated circuit ("ASIC"), a graphics processing unit ("GPU"), a field programmable gate array ("FPGA"), a programmable logic device ("PLD"), a controller, a state machine, gated logic, discrete hardware components, any other processing unit, or any combination or multiplicity thereof. The processor may be a single processing unit, multiple processing units, a single processing core, multiple processing cores, special purpose processing cores, co-processors, or any combination thereof. According to certain example embodiments, the processor, along with other components of the computing machine, may be a virtualized computing machine executing within one or more other computing machines.

The storage medium may be selected from a hard disk, a floppy disk, a compact disc read only memory ("CD-ROM"), a digital versatile disc ("DVD"), a Blu-ray disc, a magnetic tape, a flash memory, other non-volatile memory device, a solid-state drive ("SSD"), any magnetic storage device, any optical storage device, any electrical storage device, any semiconductor storage device, any physical-based storage device, any other data storage device, or any combination or multiplicity thereof. The storage media may store one or more operating systems, application programs and program modules such as module, data, or any other information. The storage media may be part of, or connected to, the computing machine. The storage media may also be part of one or more other computing machines that are in communication with the computing machine, such as servers, database servers, cloud storage, network attached storage, and so forth.

The storage media may therefore represent examples of machine or computer readable media on which instructions or code may be stored for execution by the processor. Machine or computer readable media may generally refer to any medium or media used to provide instructions to the processor. Such machine or computer readable media associated with the module may comprise a computer software product.

The input/output ("I/O") interface may be configured to couple to one or more external devices, to receive data from the one or more external devices, and to send data to the one or more external devices. Such external devices along with the various internal devices may also be known as peripheral devices. The I/O interface may include both electrical and physical connections for operably coupling the various peripheral devices to the computing machine or the processor. The I/O interface may be configured to communicate data, addresses, and control signals between the peripheral devices, the computing machine, or the processor. The I/O interface may be configured to implement any standard interface, such as small computer system interface ("SCSI"), serial-attached SCSI ("SAS"), fiber channel, peripheral component interconnect ("PCI"), PCI express (PCIe), serial bus, parallel bus, advanced technology attached ("ATA"), serial ATA ("SATA"), universal serial bus ("USB"), Thunderbolt, FireWire, various video buses, and the like. The I/O interface may be configured to implement only one interface or bus technology.

Alternatively, the I/O interface may be configured to implement multiple interfaces or bus technologies. The I/O interface may be configured as part of, all of, or to operate in conjunction with, the system bus. The I/O interface may include one or more buffers for buffering transmissions between one or more external devices, internal devices, the computing machine, or the processor.

The I/O interface may couple the computing machine to various input devices including mice, touch-screens, scanners, electronic digitizers, sensors, receivers, touchpads, trackballs, cameras, microphones, keyboards, any other pointing devices, or any combinations thereof. The I/O interface may couple the computing machine to various output devices including video displays, speakers, printers, projectors, tactile feedback devices, automation control, robotic components, actuators, motors, fans, solenoids, valves, pumps, transmitters, signal emitters, lights, and so forth.

The computing machine may operate in a networked environment using logical connections through the network interface to one or more other systems or computing machines across the network. The network may include wide area networks (WAN), local area networks (LAN), intranets, the Internet, wireless access networks, wired networks, mobile networks, telephone networks, optical networks, or combinations thereof. The network may be packet switched, circuit switched, of any topology, and may use any communication protocol. Communication links within the network may involve various digital or an analog communication media such as fiber optic cables, free-space optics, waveguides, electrical conductors, wireless links, antennas, radio-frequency communications, and so forth.

The processor may be connected to the other elements of the computing machine or the various peripherals discussed herein through the system bus. It should be appreciated that the system bus may be within the processor, outside the processor, or both. According to some embodiments, any of the processor, the other elements of the computing machine, or the various peripherals discussed herein may be integrated into a single device such as a system on chip ("SOC"), system on package ("SOP"), or ASIC device.

Embodiments may comprise a computer program that embodies the functions described and illustrated herein, wherein the computer program is implemented in a computer system that comprises instructions stored in a machine-readable medium and a processor that executes the instructions. However, it should be apparent that there could be many different ways of implementing embodiments in computer programming, and the embodiments should not be construed as limited to any one set of computer program instructions. Further, a skilled programmer would be able to write such a computer program to implement one or more of the disclosed embodiments described herein. Therefore, disclosure of a particular set of program code instructions is not considered necessary for an adequate understanding of how to make and use embodiments. Further, those skilled in the art will appreciate that one or more aspects of embodiments described herein may be performed by hardware, software, or a combination thereof, as may be embodied in one or more computing systems. Moreover, any reference to an act being performed by a computer should not be construed as being performed by a single computer as more than one computer may perform the act.

The example embodiments described herein can be used with computer hardware and software that perform the methods and processing functions described previously. The systems, methods, and procedures described herein can be embodied in a programmable computer, computer-executable software, or digital circuitry. The software can be stored on computer-readable media. For example, computer-readable media can include a floppy disk, RAM, ROM, hard disk, removable media, flash memory, memory stick, optical media, magneto-optical media, CD-ROM, etc. Digital circuitry can include integrated circuits, gate arrays, building block logic, field programmable gate arrays (FPGA), etc.

The methods, systems and test kits may incorporate means for Automatic Identification and Data Capture (AIDC), such as a Radio-frequency identification tag or card (RIF).

For the avoidance of doubt, the discussion of the invention hereinabove applies to the systems and test kits of the invention and all embodiments can be applied accordingly. However, for clarity and by way of exemplification of how the discussion applies directly to the systems and test kits, further specific embodiments are outlined below.

In some embodiments, the test system or kit takes the form of a portable system. The system may comprise an analyser, into which a test cartridge is inserted. The user may then also insert a sample collection device into the analyser. The analyser may incorporate a full colour screen to read the results. The analyser thus houses the processor and storage medium which permits the assays to be run. The test cartridge thus represents the one or more testing devices for determining levels of the blood markers in these embodiments. The systems or test kits of the invention may incorporate a separate sample collection device or this may be integrated into the one or more testing devices. One example system applicable to the present invention is the LumiraDx platform (LumiraDx).

In specific embodiments, the system or test kit further comprises a display for the output from the processor. This is intended to give a simple visual and/or audible read-out of the assays performed on the blood sample. The display may be operably connected to the processor running the computer application. The output or read-out may be an instruction to the subject in some embodiments. Depending upon the algorithm employed suitable read-outs may be selected from "increase/decrease frequency of testing", which may be to a specified level or frequency for example or equivalent wordings. The output may be colour coded or numerical to reflect the various possible outcomes as discussed herein. It is possible for the display to provide levels of the markers measured in the sample and provide suitable training and/or documentation to assist the user in interpretation of the data. However, this is not preferred for obvious reasons of susceptibility to human error. A combination of both types of information may, however, be presented in some embodiments. Thus, the display may present both quantitative and qualitative read-outs in some embodiments. Probability values related to the predictive and identification outcomes may also represent an output in some embodiments. In some embodiments, the outputs may instead be displayed by a suitable display module on a remote computing device (e.g. tablet, phone or computer), which is in operable connection with the processor/computer application housed in the analyser. This may take the form of a connectivity platform based, for instance, on cloud-based computing services. Thus, the outputs may be displayed on a suitable display module (e.g. tablet, phone or computer) which is in remote connection (e.g. Wireless Internet connection, Bluetooth or any other Near Field Communication connection) with the processor/computer application. One example of such is LumiraDx Connect (LumiraDx). In particular embodiments, the processor and storage medium housed in the analyser may be configured to determine the levels of the markers on the one or more testing devices and transmit the data to a remote computing device (e.g. tablet, phone or computer) which is configured to analyse the determined levels and output the treatment to be administered to the patient suffering from an exacerbation of inflammation as described herein. In other embodiments, the data may be transmitted to a remote computing device (e.g. tablet, phone or computer) via a cloud-based computing service which is configured to analyse the determined levels output the treatment to be administered to the patient suffering from an exacerbation of inflammation as described herein. Thus, the remote computing device is configured to display the output calculated by the cloud-based computing service.

The one or more testing devices can be of any form suitable for home use or use in a primary care setting (e.g. clinic). The various methods of detecting markers are discussed herein and from this discussion the skilled person would be well able to determine the form of a suitable corresponding device.

In specific embodiments, the one or more testing devices comprise disposable single use devices to which the blood sample is applied. Typically, the one or more testing devices may comprise a sample application zone to which the sample is added. Generally, the sample application zone can receive a relatively large volume of sample, for example 10, 20, 30, 40 or 50ml or more. The devices typically also incorporate a solid support which defines a liquid/capillary flow path for the sample once applied to the sample application zone. This may be a microfluidic flowpath. The sample application zone may be an integral part of the solid support. The solid support may comprise a chromatographic medium, such as a membrane material in some embodiments (e.g. nitrocellulose). A blood sample applied to the sample application zone will typically rehydrate the necessary reagents to detect the marker. A chase fluid (diluent) may also be applied depending on the viscosity of the sample. The reagents may include a binding reagent which specifically interacts with the marker or a substrate for effector molecules where activity is measured. A further reagent may be immobilized further along the flow path. This reagent may bind to the complex of marker and binding reagent. The binding reagent is typically labelled to provide a signal at the site of immobilization of the complex of marker and binding reagent (through binding to the further reagent). Suitable labels include fluorescent labels, magnetic labels, latex or gold as would be readily understood by one skilled in the art. One example of a suitable test strip format would be the LumiraDx Test Strip (LumiraDx).

The binding reagent and further reagent are typically antibodies (as defined herein). Thus, in specific embodiments, the one or more testing devices may comprise a lateral flow test strip. In some embodiments, a single lateral flow test strip is employed to permit detection of all markers that are to be determined in the test sample. In other embodiments, a separate lateral flow test strip is provided for each marker that is determined. In yet further embodiments, the one or more testing devices may comprise two lateral flow test strips: one lateral flow test strip for the one or more markers of eosinophil levels and one lateral flow test strip for the one or more markers of neutrophil levels. These two lateral flow test strips may further include one or more supporting markers of eosinophil and neutrophil levels. Alternatively, a third lateral flow test strip may be provided for the one or more supporting markers of eosinophil and neutrophil levels. In yet further embodiments, the one or more testing devices may comprise two lateral flow test strips: one lateral flow test strip for at least 3 markers of eosinophil levels and one lateral flow test strip for at least 3 markers of neutrophil levels.

The devices may also include a control zone to confirm sample has passed through the device satisfactorily. In the absence of confirmation by the control zone, the system or test kit may indicate an invalid result to the user, for example via the display. The devices may act as competitive or sandwich assays, as discussed herein. ELISA (enzyme linked immunosorbent assay) is an example of a suitable assay format that may be incorporated in the testing devices used in the invention. Again, typically all reagents to detect the levels of the one or more markers are pre-loaded onto the testing device such that they can interact with the blood sample once added to the device. This minimizes intervention and thus error caused by the subject. Thus, effectively, the device may only require the user to apply the sample and subsequently observe the output of the assay.

The systems and test kits require a quantitative read-out to permit a treatment to be selected for the subject suffering from an exacerbation. Thus, the systems or test kits may incorporate a suitable reader to provide a quantitative output (in conjunction with the processor and storage medium). As already mentioned this output can be an absolute or a relative output. Suitable readers may incorporate an illuminator to expose the device to a specific wavelength or wavelengths of light and a suitable detector for the reflected or emitted light. The devices may also incorporate a suitable processor and computer application to output the selected treatment based upon the detected signal. Thus, the processor running the computer application will be in operable connection with the reader. By "operable connection" is meant a functional connection that permits the exchange of a signal or information between the elements. An example of such a reader is the opTricon^{®} 'Cube' reader (opTricon Gmbh). Others include the ESEQuant LR3 reader (Qiagen) and the Lumos reader (Lumos). Alternatively, in some embodiments the suitable processor and computer application to output the selected treatment based upon the detected signal may be incorporated on a remote computing device (e.g. tablet, phone or computer), which is in operable connection with the processor/computer application housed in the reader. This may take the form of a connectivity platform based, for instance, on cloud-based computing services. Thus, the output of the selected treatment based upon the detected signal may be displayed on a suitable display module (e.g. tablet, phone or computer) which is in remote connection (e.g. Wireless Internet connection, Bluetooth or any other Near Field Communication connection) with the processor/computer application of the reader. One example of such is LumiraDx Connect (LumiraDx). Thus, the processor/computer application housed in the reader may be configured to determine the levels of the markers on the one or more testing devices and transmit the data to a remote computing device (e.g. tablet, phone or computer) which is configured to analyse the determined levels and output the treatment to be administered to the patient suffering from an exacerbation of inflammation as described herein. In other embodiments, the data may be transmitted to a remote computing device (e.g. tablet, phone or computer) via a cloud-based computing service which is configured to analyse the determined levels output the treatment to be administered to the patient suffering from an exacerbation of inflammation as described herein. Thus, the remote computing device is configured to display the output calculated by the cloud-based computing service.

The testing device may comprise one or more specific binding reagents to bind to the marker whose level is detected in the blood sample. As discussed above, where protein levels are measured the reagent may comprise an antibody (to include derivatives, fragments and aptamers). Where RNA levels are measured suitable reagents may comprise nucleic acid amplification reagents such as primers, probes, dNTPs, polymerases etc. to permit amplification reactions to be run and results reported from the testing device.

The one or more testing devices may comprise an enzyme detection device as discussed in greater detail hereinabove. These devices may be particularly useful for investigating enzymatic activity (e.g. MMPs and HNE). The one or more testing devices may comprise a testing device for measuring cleavage of a peptide substrate as an indicator of protease activity.

In specific embodiments, the testing device comprises:
a. an indicator molecule for adding to the blood sample, said indicator molecule comprising
   i. a cleavage region comprising at least one cleavage site, which can be cleaved by said protease activity if present; and
   ii. a capture site;
   wherein cleavage of the at least one cleavage site produces a novel binding site;
b. a capture zone to receive the blood sample, wherein the capture zone comprises capture molecules capable of binding to the capture site of the indicator molecule in order to immobilise the indicator molecule including the novel binding site; and
c. binding molecules capable of binding to the novel binding site, wherein the binding molecules are incapable of binding to the indicator molecule unless and until cleavage has occurred.

Where a plurality of markers is determined in the sample, the system or test kit may incorporate the appropriate number of testing devices to permit each marker to be determined. This is particularly the case where the markers are detecting using different platforms. Thus, in some embodiments, the one or more testing devices may comprise one or more lateral flow activity assays, ELISAs, fluorogenic substrate assays, competition assays or microfluidics assays (e.g. LumiraDx platform technology) etc.

As discussed above, the invention relies upon marker cut-off values/thresholds, which may be calculated based on a level derived from a subject population. The subject population may not be suffering from an exacerbation of inflammation (e.g. a PEx). Alternatively, the subject population may be suffering from an exacerbation of inflammation (e.g. a PEx). In particular embodiments, the subject population may comprise individuals not suffering from an exacerbation of inflammation (e.g. a PEx) as well as individuals who are suffering from an exacerbation of inflammation (e.g. a PEx). Accordingly, in some embodiments, the computer application causes the processor to calculate levels of the at least one marker with reference to a cut-off value/threshold level of the marker as discussed above. Suitable approaches which can be adopted for such analyses are known in the art and include fluctuation analyses, such as detrended fluctuation analysis (DFA) or other forms of line fitting.

In certain embodiments, the computer application causes the processor to indicate to the subject the requirement to determine the levels of at least one marker. In other embodiments, the computer application is further configured to output from the processor a requirement to increase the frequency of determining the levels of the at least one marker in blood samples taken from the subject where a perturbation in the levels of the at least one marker is calculated. The computer application may be further configured to output via the processor a requirement to maintain the increased frequency of determining the levels of the at least one marker until the levels of the at least one marker are calculated as returned to a non-perturbed level.

In specific embodiments, the computer application is configured to calculate perturbed levels of at least one marker of eosinophil levels and output via the processor that a calculated perturbation in levels of the at least one marker of eosinophil levels indicates that corticosteroids should be administered as the treatment for the exacerbation of inflammation. In other embodiments, the computer application is configured to calculate perturbed levels of at least one marker of neutrophil levels and output via the processor that a calculated perturbation in levels of the at least one marker of neutrophil levels indicates that antibiotics should be administered as the treatment for the exacerbation of inflammation. For systems and test kits designed to measure the levels of at least one marker of eosinophil levels and at least one marker of neutrophil levels, the computer application may be configured to calculate perturbed levels of the at least one marker of eosinophil levels and at least one marker of neutrophil levels and output via the processor that:
(i) a calculated perturbation in the levels of the at least one marker of eosinophil levels and no perturbation in the levels of the at least one marker of neutrophil levels indicates that corticosteroids should be administered as the treatment for the exacerbation of inflammation; or
(ii) a calculated perturbation in the levels of the at least one marker of neutrophil levels and no perturbation in the levels of the at least one marker of eosinophil levels indicates that antibiotics should be administered as the treatment for the exacerbation of inflammation; or
(iii) a calculated perturbation in the levels of the at least one marker of eosinophil levels and the at least one marker of neutrophil levels indicate that corticosteroids and antibiotics should be co-administered as the treatment for the exacerbation of inflammation.

Where the levels of at least one supporting marker of eosinophil and neutrophil levels are additionally determined, the computer application may be configured to calculate perturbed levels of the at least one supporting marker of eosinophil and neutrophil levels in combination with at least one marker of eosinophil levels and/or at least one marker of neutrophil levels and output via the processor that a calculated perturbation in the levels of the at least one supporting marker of eosinophil and neutrophil levels:
(a) in combination with a calculated perturbation in the levels of the at least one marker of eosinophil levels and no perturbation in the levels of the at least one marker of neutrophil levels indicates that corticosteroids should be administered as the treatment for the exacerbation of inflammation; or
(b) in combination with a calculated perturbation in the levels of the at least one marker of neutrophil levels and no perturbation in the levels of the at least one marker of eosinophil levels indicates that antibiotics should be administered as the treatment for the exacerbation of inflammation; or
(c) in combination with a calculated perturbation in the levels of the at least one marker of eosinophil levels and the at least one marker of neutrophil levels indicate that corticosteroids and antibiotics should be co-administered as the treatment for the exacerbation of inflammation.

The computer application may be further configured to calculate perturbed levels of one or more of the markers relative to levels in blood samples taken from the subject at multiple time points, to indicate increased/reduced frequency of testing, to analyse the calculated levels of two or more markers in a determined sequence, as described herein. As discussed above, the markers may be weighted. Thus, the computer application may be configured to apply and/or calculate as appropriate a weighting to the determined levels of the markers.

In specific embodiments, the computer application is configured to calculate levels of at least one marker by normalising against the levels of a reference marker, typically also measured in the same blood sample. Suitable reference markers are discussed herein.

In certain embodiments, the computer application is further configured to incorporate inputs from other indicators of exacerbation of inflammation into the calculation. Those other indicators of exacerbation of inflammation may comprise shortness of breath, increased wheeze, increased pulse rate, dyspnoea, increased sputum purulence, increased sputum colour, sore throat, increased cough, cold and fever. Another indicator that may be monitored is Forced Expiratory Volume in one second (FEV₁).

The computer application thus runs the relevant algorithms to select the treatment for the exacerbation. For the avoidance of doubt all of the outputs described may be displayed by a suitable display module, which is in operable connection with the processor/computer application. This may take the form of a connectivity platform based, for instance, on cloud-based computing services. Thus, the outputs may be displayed on a suitable display module (e.g. tablet, phone or computer) which is in remote connection with the processor/computer application. One example of such is LumiraDx Connect (LumiraDx).

Without wishing to be bound by any particular theory, it is also possible that the markers described herein may, in fact, reflect the levels of eosinophil and/or neutrophil activity (e.g. eosinophil and/or neutrophil activation). Thus, in alternative embodiments, reference to a "marker of eosinophil levels" as used throughout this specification may be replaced with a "marker of eosinophil activity". The levels of said markers correlate (positively or negatively or exhibit a more complex pattern depending on the marker) with levels of eosinophil activity in the blood (which may include, but not necessarily, levels of eosinophil cells). That is to say, the marker of eosinophil activity is distinct from eosinophils themselves. Typically, the marker is a protein or peptide. Advantageously, the markers of eosinophil activity can be conveniently detected using, for instance, labelled antibodies as further described herein. Thus, the levels of the at least one marker of eosinophil activity reflect the level of eosinophil activity in the blood. The same applies *mutatis mutandis* to a "supporting marker of eosinophil levels". Thus, in alternative embodiments, reference to a "supporting marker of eosinophil levels" as used throughout this specification may be replaced with a "supporting marker of eosinophil activity".

Similarly, in alternative embodiments, reference to a "marker of neutrophil levels" as used throughout this specification may be replaced with a "marker of neutrophil activity". The levels of said markers correlate (positively or negatively or exhibit a more complex pattern depending on the marker) with levels of neutrophil activity in the blood (which may include, but not necessarily, levels of neutrophil cells). That is to say, the marker of neutrophil activity is distinct from neutrophils themselves. Typically, the marker is a protein or peptide. Advantageously, the markers of neutrophil activity can be conveniently detected using, for instance, labelled antibodies as further described herein. Thus, the levels of the at least one marker of neutrophil activity reflect the level of neutrophil activity in the blood. The same applies *mutatis mutandis* to a "supporting marker of neutrophil levels". Thus, in alternative embodiments, reference to a "supporting marker of neutrophil levels" as used throughout this specification may be replaced with a "supporting marker of eosinophil activity".

The same applies *mutatis mutandis* to a "supporting marker of eosinophil and neutrophil levels". Thus, in alternative embodiments, reference to a "supporting marker of eosinophil and neutrophil levels" as used throughout this specification may be replaced with a "supporting marker of eosinophil and neutrophil activity". The levels of said markers correlate (positively or negatively or exhibit a more complex pattern depending on the marker) with levels of both eosinophil and neutrophil activity in the blood (which may include, but not necessarily, levels of eosinophil and neutrophil cells). That is to say, the marker of eosinophil and neutrophil activity is distinct from eosinophils and neutrophils themselves. Their combination with at least one marker of eosinophil activity and/or at least one marker of neutrophil activity increases the predictive power in relation to correctly identifying an increase in eosinophil activity and/or neutrophil activity in a blood sample (as appropriate depending on the specific marker combination employed).

### DESCRIPTION OF THE FIGURES

The invention will now be described by way of example with respect to the accompanying drawings in which:
Figure 1 is a schematic view of four different formats of the assay useful in the invention. Each format relies upon the same basic components of solid support (1), capture molecule (2), an indicator molecule containing a capture site (3) and a cleavage site (4) and a binding molecule (5) that binds to the indicator molecule only after cleavage (6) has occurred.
Figure 2 is a schematic view of an enzyme detection device useful in the present invention and shows operation of the device in the absence (Fig. 2A) or presence (Fig. 2B) of enzyme cleavage activity.
Figure 3 shows the visual read-out of the assay (shown in Figure 2) as levels of MMP activity in the test sample are increased.
Figure 4 is a schematic view of an enzyme detection device useful in the present invention. The figure specifies the exact longitudinal dimensions and position of each of the card components.
Figure 5 shows an example of synthesis of a structurally constrained indicator molecule.
In Fig. 5A initially, a linear peptide (1) is synthesised, for example using solid phase Fmoc chemistry. The peptide may be purified for example by High Performance Liquid Chromatography (HPLC). The peptide is then constrained, or cyclised, by reaction between thiol groups on the peptide (2) and the scaffold molecule (3). This reaction produces a structurally constrained "clipped" peptide (4).
In Fig. 5B, the indicator molecule is synthesised to include the capture site (1), for example by synthesis of the linear peptide on a pre-loaded Biotin-PEG resin.
Figure 6 shows schematically the ability of the binding molecules used in the invention to bind exclusively to the cleaved indicator molecule. In the absence of enzyme cleavage activity, the structurally constrained indicator molecule (1) is not bound by the antibody binding molecule (2). This antibody is generated using the cleaved indicator molecule (3) as antigen and thus only binds to this "open" form of the molecule.
Figure 7 shows a number of scaffold molecules useful in the indicator molecules described herein.
Figure 8 shows a number of scaffold molecules useful in the indicator molecules described herein, together with proposed nomenclature.
Figure 9 shows some attachment options for scaffold molecules to the indicator molecules. Fig. 9A shows products of cleavage at a single cleavage site and Fig. 9B shows products of cleavage at two separate cleavage sites.
Figure 10 - General algorithm for using the calculated marker levels to select treatment for an exacerbation of inflammation. BM = marker (any marker as described herein).
Figure 11 - Correlation of MBP with blood eosinophil levels (a) overall and (b) split between exacerbation and stable state.
Figure 12 - Correlation of Calprotectin with blood neutrophil levels (a) overall and (b) split between exacerbation and stable state.
Figure 13 - Correlation of MMP9 with blood neutrophil levels (a) overall and (b) split between exacerbation and stable state.
Figure 14 - Model 1: Correlations with eosinophil levels, the combination of 5 biomarkers produced an R² of 0.752. The 5 biomarkers with significant levels in this model were: EDN (<0.001), MMP9 (<0.001), HNE ((<0.001), NGAL (0.001) and MBP (0.020) (in level of importance).
Figure 15 - Model 2: Correlations with neutrophil levels, the combination of 5 biomarkers produced an R² of 0.489. The 5 biomarkers with significance levels in this model were: Calprotectin (0.00), MBP (0.003), MMP9 (0.012), CRP (0.043) and NGAL (0.057) (in level of importance).
Figure 16A - Model 3: Correlation with neutrophil levels. The combination of 3 biomarkers produced an R² of 0.514. The 3 biomarkers in this model were: MBP, Calprotectin and A1AT (as measured by Lateral flow).
Figure 16B - Sensitivity and Specificity for Model 3 (MBP, Calprotectin and A1AT (LF). Optimal sensitivity of 90.91% and specificity of 92.11% with a cut-off of 8.66. a) scatter plot with median and interquartile ranges with Mann Whitney test p value b) Tukey's Box and whiskers plot c) ROC curve with AUC.
Figure 17A - Model 4: Correlation with neutrophil levels. The combination of 5 biomarkers produced an R² of 0.518. The 5 biomarkers in this model were: MBP, Calprotectin, A1AT (as measured by Lateral flow), MMP9 and CRP.
Figure 17B - Sensitivity and Specificity for Model 4 (MBP, Calprotectin, A1AT (LF), MMP9 and CRP). Optimal sensitivity of 90.91% and specificity of 94.74% with a cut-off of 8.89. a) scatter plot with median and interquartile ranges with Mann Whitney test p value b) Tukey's Box and whiskers plot c) ROC curve with AUC.
Figure 18A - Model 5: Correlation with neutrophil levels. The combination of 6 biomarkers produced an R² of 0.514. The 6 biomarkers in this model were: MBP, Calprotectin, A1AT (as measured by Lateral flow), MMP9, CRP and NGAL.
Figure 18B - Sensitivity and Specificity for Model 5 (MBP, Calprotectin, A1AT (LF), MMP9, CRP and NGAL). Optimal sensitivity of 90.91% and specificity of 92.11% with a cut-off of 8.695. a) scatter plot with median and interquartile ranges with Mann Whitney test p value b) Tukey's Box and whiskers plot c) ROC curve with AUC.
Figure 19 - Model 6: Correlations with neutrophil levels. The combination of 5 biomarkers produced an R² of 0.308. The 5 biomarkers in this model were: MMP9, Calprotectin, HNE, CRP and A1AT (as measured by ELISA).
Figure 20 - Model 6 characterised on further defined group (removal of subgroup 2 and removal of grey zone samples) a) scatter plot with median and interquartile ranges with Mann Whitney test p value b) Tukey's Box and whiskers plot c) ROC curve with AUC.
Figure 21 - Model 7: Correlations with neutrophil levels. The combination of 4 biomarkers produced an R² of 0.33. The 4 biomarkers in this model were: MMP9, Calprotectin, HNE and CRP.
Figure 22 - Model 8: Correlations with neutrophil levels. The combination of 6 biomarkers produced an R² of 0.334. The 6 biomarkers in this model were: MMP9, Calprotectin, HNE, CRP, A1AT (as measured by LF) and NGAL.
Figure 23 - Model 9: Correlations with neutrophil levels. The combination of 5 biomarkers produced an R2 of 0.332. The 5 biomarkers in this model were: MMP9, Calprotectin, HNE, CRP and A1AT (as measured by LF).
Figure 24A - Model 2: Correlations with neutrophil levels. The combination of 5 biomarkers produced the AUC for the training and validation sets of 0.9, and for the test set of 0.7. The 5 biomarkers in this model were: MMP9, LTB4, EDN, A1AT and SuPAR.
Figure 24B - Model 4: Correlations with neutrophil levels. The combination of 5 biomarkers produced the AUC for the training and validation sets of 0.84, and for the test set of 0.87. The 5 biomarkers in this model were: MMP9, LTB4, EDN, A1AT and CRP.
Figure 25 - Model 1: Correlations with eosinophil levels. The combination of 5 biomarkers produced the AUC for the training and validation sets of 0.94 and 0.92, respectively, and for the test set of 0.81. The 5 biomarkers in this model were: EDN, MPO, RNASE3, HNE and SuPAR.

### DETAILED DESCRIPTION

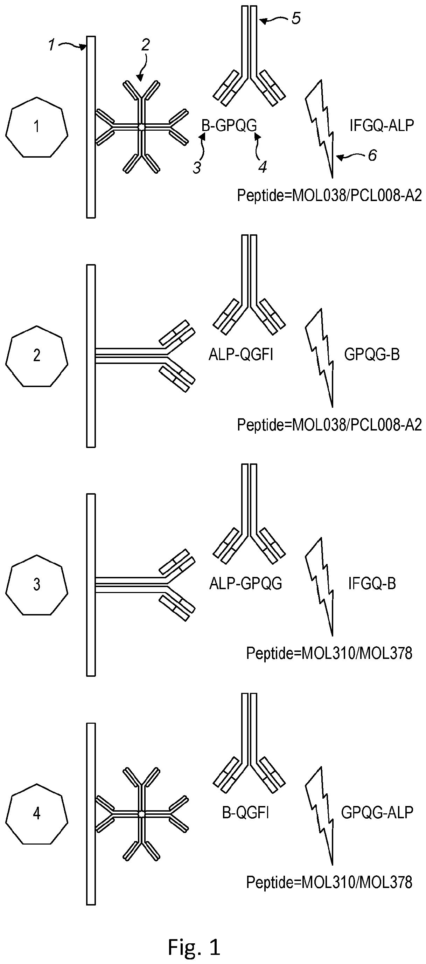

Figure 1 is a schematic view of four different formats of an assay useful for performance of the invention, in particular for detecting molecules such as proteases (e.g. MMPs) in blood samples. Each format relies upon the same basic components of solid support (1), capture molecule (2), an indicator molecule containing a capture site (3) and a cleavage site (4) and a binding molecule (5) that binds to the indicator molecule only after cleavage (6) has occurred.

In formats 1 and 4, the capture molecule (2) is streptavidin. Here, the capture molecule (2) binds to a biotin capture site (3) within the indicator molecule. In formats 2 and 3, the capture molecule (2) is an antibody. Here, the capture molecule (2) binds to an epitope capture site (3) within the indicator molecule. The epitope is found in the alternative long peptide (ALP) which is derived from human chorionic gonadotropin (hCG).

Once the indicator molecule is added to a test sample, any enzyme specifically recognising the cleavage site (4) present, may cleave the indicator molecule (6). This cleavage event (6) produces a binding site for the specific antibody binding molecule (5). The binding molecule (5) is unable to bind to the indicator molecule until cleavage (6) has occurred. Thus, in formats 1 and 3 the antibody binding molecule (5) binds to the amino acid sequence GPQG produced as a result of cleavage of the GPQGIFGQ sequence. In formats 2 and 4, on the other hand, the antibody binding molecule (5) binds to the amino acid sequence QGFI, also produced as a result of cleavage of the GPQGIFGQ sequence. In each format, the antibody binding molecule (5) does not bind to the GPQGIFGQ sequence prior to cleavage (not shown).

Figure 2 is a schematic view of an enzyme detection device used in the present invention and shows operation of the device in the absence (Fig. 2A) or presence (Fig. 2B) of enzyme cleavage activity in the blood sample. The test strip includes an adhesive liner (1) upon which the other components of the device are assembled. From right to left, the sample application zone (2) is in the form of an absorbent pad. This is laid partially overlapping the conjugate pad (3), which is impregnated with the labelled binding molecules (7). In alternative embodiments, the labelled binding molecules may be impregnated in the sample application zone and this removes the need for a separate conjugate pad. The conjugate pad (3) is in fluid connection with a nitrocellulose membrane (4). The nitrocellulose membrane (4) contains immobilized streptavidin molecules (5) which define a capture zone. The membrane (4) further contains immobilized further binding molecules (6) downstream of the capture zone which bind to further labelled molecules (11) which pass through the device with the sample and form a separate control zone. Alternatively, the immobilised further binding molecules may bind to labelled binding molecules (7). The device optionally further comprises an absorbent pad (8) to absorb any test sample and reagents reaching the end of the device.

In use, the indicator molecule (9) is added to the test sample prior to bringing the test sample into contact with the sample application zone (8) of the device. As shown in Figure 2A, in the absence of enzyme cleavage activity in the test sample, the indicator molecule (9) remains uncleaved at the cleavage site. Upon sample flow into the conjugate pad (3), the binding molecules (7) are unable to bind to the indicator molecule (9) because cleavage of the cleavage site has not occurred. The indicator molecules become bound at the capture zone via the interaction between streptavidin (5) and the biotin capture site (10) of the indicator molecule (9). The labelled binding molecules (7) are not immobilized at the capture zone because they cannot bind to the indicator molecules (9). Accordingly, the labelled binding molecules flow through to the control zone and beyond. Further labelled molecules (11) also pass through the device to the control zone where they are immobilized by binding to the immobilized further binding molecules (6). Thus, absence of enzyme cleavage activity is displayed as a signal only at the control zone, but not at the capture zone. Excess sample, potentially containing labelled binding molecules (7), flows into the absorbent pad (8).

As shown in Fig. 2B, in the presence of enzyme cleavage activity in the test sample, the indicator molecule (9) is cleaved at the cleavage site. Upon sample flow into the conjugate pad (3), the binding molecules (7) are able to bind to the indicator molecule (9) because cleavage of the cleavage site has occurred. The indicator molecules become bound at the capture zone via the interaction between streptavidin (5) and the biotin capture site (10) of the indicator molecule (9). The labelled binding molecules (7) are immobilized at the capture zone due to binding to the indicator molecules (9) at the cleavage site. Due to the relative excess of labelled binding molecule (7) to binding sites at the capture zone some labelled binding molecules (7) still flow through to the control zone and beyond. Further labelled molecules (11) also pass through the device to the control zone where they are immobilized by binding to the immobilized further binding molecules (6). Thus, level of enzyme cleavage activity may be measured via a signal at the capture zone (and a signal will also be present at the control zone). Excess sample, potentially containing cleavage products of the indicator molecule that do not contain the biotin capture site (10), flows into the absorbent pad (8).

It should be noted that the control zone is optional. The level of enzyme cleavage activity in the blood sample can be monitored based upon a measurement of the corresponding signal at the capture zone.

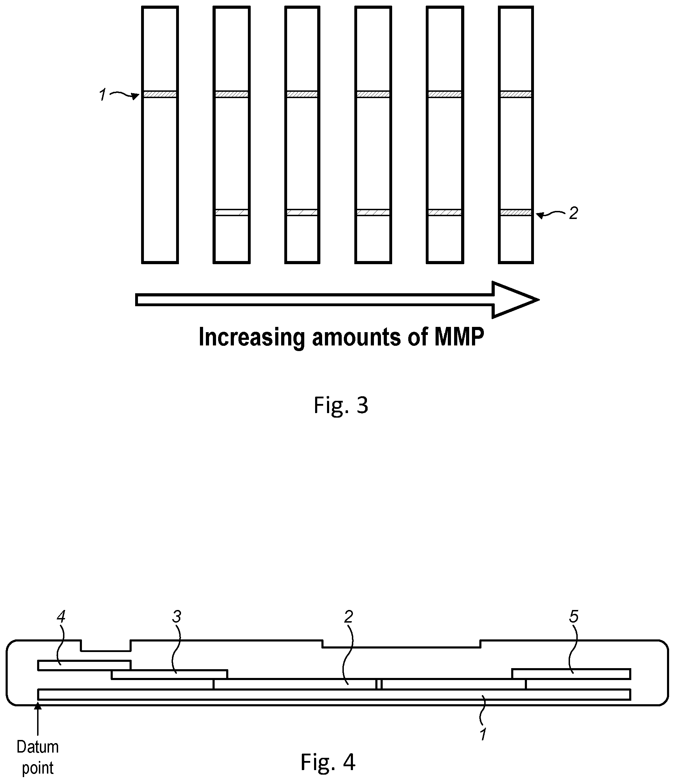

Figure 3 shows the visual read-out of the assay (shown in Figure 2) as levels of MMP activity in the test sample are increased. As can readily be seen, the signal at the control zone (1) is constant as MMP amounts increase. In contrast, as MMP amounts increase, the signal at the capture zone (2) also increases. This is due to cleavage of the indicator molecule at the cleavage site by MMP activity. This reveals a binding site, enabling binding of the binding molecules which is detected at the capture zone (2) via interaction between capture molecules defining the capture zone and the capture site of the indicator molecules. The intensity of the signal at the capture zone can be measured to provide the level of effector molecule in the blood sample. This may employ a suitable reader.

Figure 4 is a schematic view of one specific enzyme detection device useful with the present invention. The table below provides a legend for the figure and specifies the longitudinal dimensions and position of each of the card components in this particular embodiment. Of course, the dimensions and positions may be varied as would be readily understood by one skilled in the art.

| **Component** | **Size** | **Position from Datum point** |
|---|---|---|
| Backing card (1) | 60mm | 0mm |
| Nitrocellulose Membrane (2) | 25mm | 20mm |
| Conjugate Pad (3) | 17mm | 5mm |
| Sample Pad (4) | 10mm | 0mm |
| Absorbent Pad (5) | 22mm | 38mm |

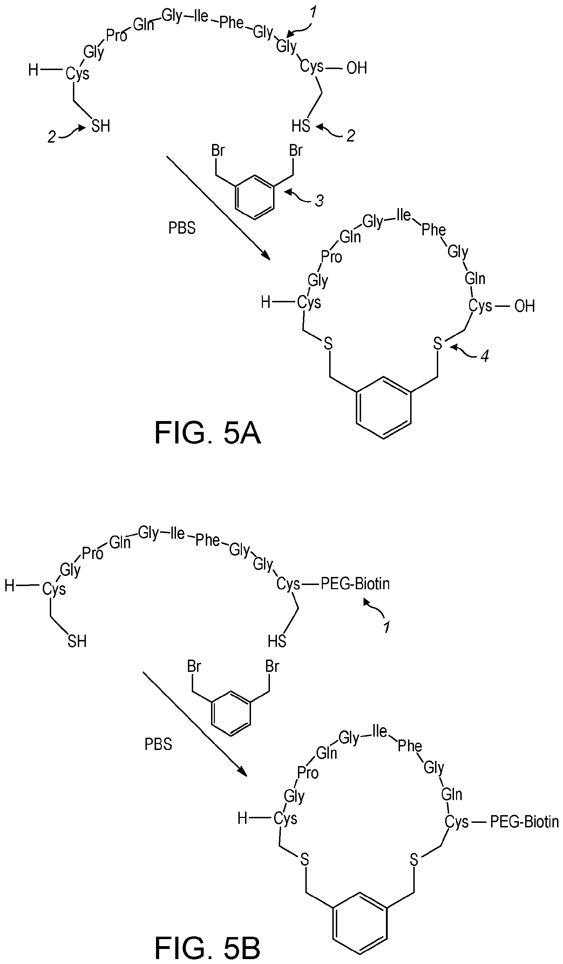

Figure 5 shows an example of synthesis of a structurally constrained indicator molecule. It should be noted that additional spacer or linker regions may be included between the cleavage region and the site of attachment of the scaffold molecule.

In Fig. 5A initially, a linear peptide (1) is synthesised, for example using solid phase Fmoc chemistry. The peptide may be purified for example by High Performance Liquid Chromatography (HPLC). The peptide is then constrained, or cyclised, by reaction between thiol groups on the peptide (2) and the scaffold molecule (3). This reaction produces a structurally constrained "clipped" peptide (4).

In Fig. 5B, the indicator molecule is synthesised to include the capture site (1), for example by synthesis of the linear peptide on a pre-loaded Biotin-PEG resin.

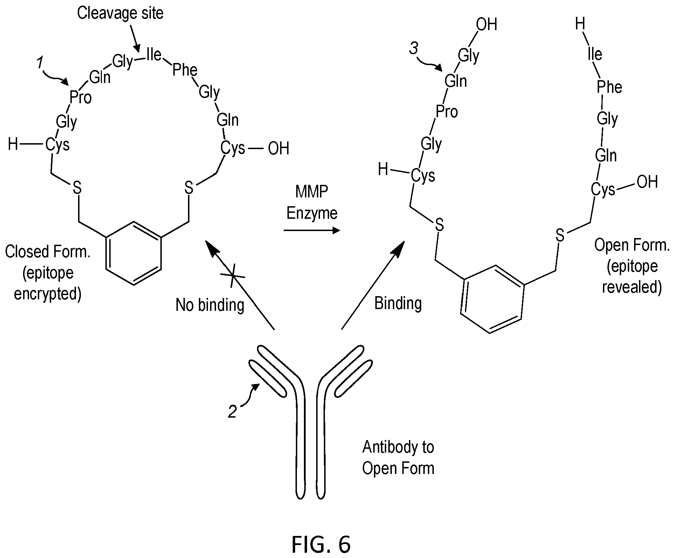

Figure 6 shows schematically the ability of the binding molecules used in some embodiments of the invention to bind exclusively to the cleaved indicator molecule. In the absence of enzyme cleavage activity, the structurally constrained indicator molecule (1) is not bound by the antibody binding molecule (2). This antibody is generated using the cleaved indicator molecule (3) as antigen and thus only binds to this "open" form of the molecule.

Figures 7 and 8 show a range of suitable scaffold molecules for use in the invention.

Figure 9 shows, in schematic form, some attachment options for scaffold molecules to the indicator molecules. Fig. 9A shows products of cleavage at a single cleavage site and Fig. 9B shows products of cleavage at two separate cleavage sites.

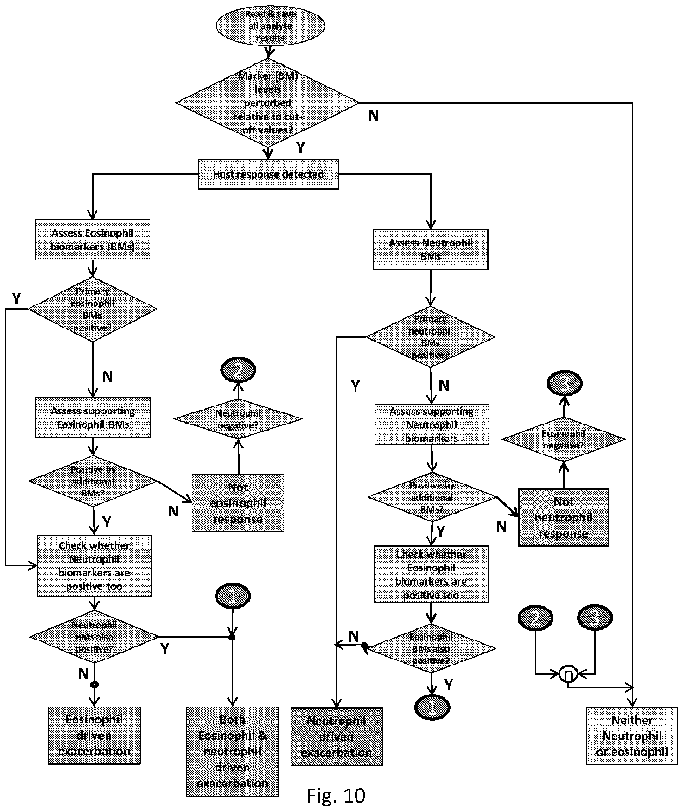

Figure 10 presents an algorithm useful in the invention. This particular algorithm was designed based on the observed perturbations in marker levels in patients suffering from an exacerbation and the derived correlations between said perturbations and changes in eosinophil and/or neutrophil levels in the blood samples. The principles embodied in this algorithm are applicable to all of the markers and combinations described herein.

The invention will be further understood with reference to the following experimental examples.

### EXAMPLES

### Example 1 - Preliminary experimentation concerning treatment stratification for COPD patients in response to an exacerbation

### Introduction

COPD is a common disease that predominantly affects the elderly population. Its prevalence is increasing and it contributes to substantial morbidity and mortality. There are an estimated 80 million people that have moderate to severe COPD worldwide. COPD has an estimated annual death rate of over 4 million people globally. By 2020 it is predicted to be the 3rd leading cause of mortality worldwide. According to the World Health Organization (WHO) World Health Report 2007, the top five respiratory diseases, including COPD, account for 17% of all deaths and 13% of all Disability-Adjusted Life Years. The Global Strategy for the prevention and control of non-communicable diseases endorsed by the 53rd World Health Assembly cites chronic respiratory disease as one of the four priority disease groups to be addressed. In Europe, COPD annual expenditure is over €40 billion.

Patients with COPD have daily symptoms, a poorer health status, reduced exercise capacity, and impairment in lung function. The symptoms can deteriorate rapidly in response to infection or pollution. The acute and sustained worsening of the symptoms is termed a COPD exacerbation. COPD exacerbations account for 15% of all medical admissions, 1 million bed days and an annual UK NHS expenditure of £500 million. COPD and in particular COPD exacerbations are of a high public health and financial relevance associated with a significant negative impact on the quality of life.

The main therapeutic options are: antibiotics to tackle the infection (amoxicillin or doxycycline as first line, or co-amoxiclav); bronchodilators (beta-2-agonists, anticholinergics and theophylline) to make breathing easier; and corticosteroids (Prednisolone) to accelerate recovery by reducing inflammation. Bacterial and viral respiratory infections have an acknowledged association with exacerbations. Recent improvements in bacterial and moreover viral identification have increased the proportion of exacerbations that are associated with a pathogen. The proportion associated with bacteria alone is about 30%, virus alone about 25% and combined 25%. COPD exacerbations are also associated with an increase in airway inflammation. Both sputum neutrophil and eosinophil total cell counts and activation markers increase in COPD exacerbations. Interestingly the neutrophil count increases irrespective of the associated pathogen, but the eosinophil count increases in viral infection either alone or in combination with bacteria.

Current guidelines advocate the use of oral corticosteroids for patients with a COPD exacerbation who have increased dyspnoea and antibiotics in those with a history of more purulent sputum. A Cochrane review of systemic corticosteroids that included 10 studies reported that corticosteroids increase the rate of recovery following a severe exacerbation, reduce the length of hospital admission by 1-2 days and reduce the proportion of patients that have treatment failure. This beneficial effect is similar for both oral and parenteral corticosteroids. In moderate exacerbations oral corticosteroids increase the time to next exacerbation. However, it is likely these small corticosteroid-related benefits are confined to a subgroup of patients. Likewise, antibiotic therapy in COPD exacerbations is beneficial. A recent Cochrane review that included 11 trials with a total of 917 patients found that antibiotics, regardless of choice, reduced the risk of short-term mortality by 77%, decreased the risk of treatment failure by 53% and the risk of sputum purulence by 44%. However, the range of response was large and it is estimated that antibiotics are of clinical benefit in only 25-50% of COPD exacerbations. Our inability to identify accurately which patients with a COPD exacerbation should receive antibiotics and or corticosteroids inevitably leads to inappropriate and excessive use of treatment.

The widespread use of antibiotics in the community has been implicated in the increase of antibiotic resistance, particularly MRSA, which now accounts for over 40% of Staphylococcus aureus blood isolates and the substantial increase of 17.2% in the number of cases of Clostridium difficile infection in patients aged 65 years and above in England during 2015. Systemic corticosteroids are also associated with well-established side-effects and in particular their use is complicated in patients with co-morbid diabetes mellitus and ischaemic heart disease. Indeed, the number needed to harm i.e. the number that need to receive systemic corticosteroids to observe an additional adverse reaction is only 6 patients. Therefore, there is a pressing need for either a single or more likely a composite of biomarkers to direct therapy in COPD exacerbations.

Targeted therapy for COPD exacerbations therefore requires reliable tests that can be performed in minutes without the need for laboratory support. Measuring biomarkers in blood samples provides a real opportunity to develop a near patient test for both secondary and primary care.

### Solution

The Mologic multiplexed, blood biomarker diagnostic (Rightstart), with its integrated biomarker level interpretation algorithm, determines the levels of markers in a blood sample which have been found to correlate with levels of eosinophils and/or neutrophils. Thus, the levels of the markers can be used to determine whether an exacerbation is eosinophilic (i.e. high levels of eosinophils) or neutrophilic (i.e. high levels of neutrophils) in order to guide steroid or antibiotic treatment. Through the use of blood as the sample, the proposed point-of-care test has been designed to be minimally invasive, easy to use, rapid and simple to understand results, so that it can easily be integrated into a primary care setting (e.g. clinic).

### Methods

### Measurement of blood (serum) biomarkers

Samples (banked, frozen) were provided from a Leicester study (MRC funded BEAT-COPD study !SRCTN2422949) Study details: From a two-staged single centre study, blood, sputum and urine samples from COPD subjects were longitudinally collected at four visit types: namely stable state (defined as being eight weeks free from an exacerbation visit), exacerbation (defined according to Anthonisen criteria and healthcare utilisation), two weeks post therapy and at recovery (six weeks post exacerbation visit). Exacerbations were treated with oral corticosteroids and antibiotics according to guidelines or trial study design. Clinical data including demographics, symptoms, lung function, inflammatory profiling in blood and sputum, bacteriology including standard culture, qPCR for common pathogens and microbiomics, viruses by PCR and fungal culture were undertaken.

### Blood biomarkers for differentiating Eosinophil and neutrophil driven exacerbations (to guide treatment)

37 different biomarker assays were tested with serum.

Limited samples were available for testing (few exacerbations), therefore analysis focused only on which biomarkers correlated with blood eosinophils or neutrophils levels.

The most promising from a selection of 41 samples (where Leicester serum samples were also available)
- 24 stable samples
- 17 exacerbation samples

The biomarkers were selected on a rational basis and in the light of our increasing experience with urine samples from other clinical studies. Inflammatory leukocytes active in the lung cause a wide range of biomarkers to be released into lung fluid and blood, some originating from the leukocytes, some from the damage they cause to the surrounding tissue and some as a consequence of the signalling pathways that call them into the lung or control their activity.

The distribution of the continuous variables was studied using histograms, values of skewness and kurtosis, and normality was tested by the Kolmogorov-Smirnov test. Paired t test and Wilcoxon matched-pairs signed rank test were used to compare quantitative data in the two groups. Receiver operating characteristic (ROC) curve analysis was used to study the accuracy of the various diagnostic tests and logistic regression to find the best combination of biomarkers. P values<0.05 were considered to be statistically significant. Statistical analyses were carried out through the use of computer IBM software SPSS 21 (Chicago, IL, USA),Graphpad Prism 5 and in R.

### Results

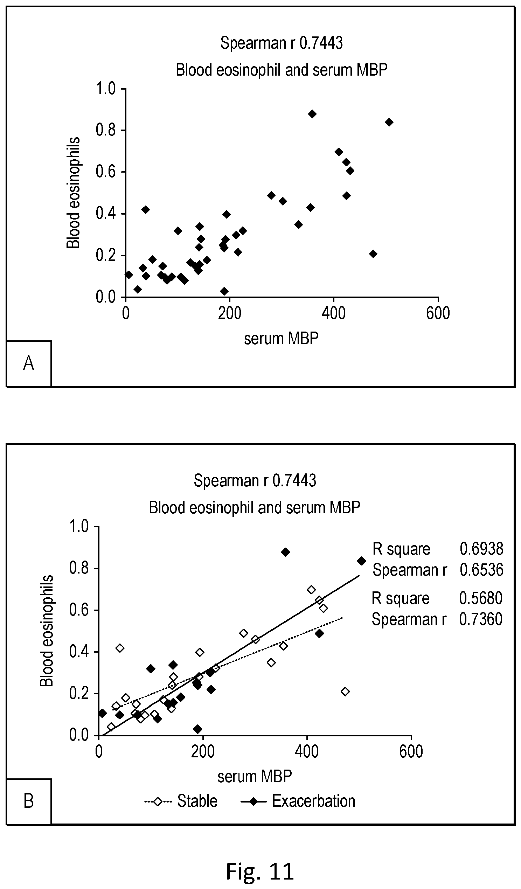

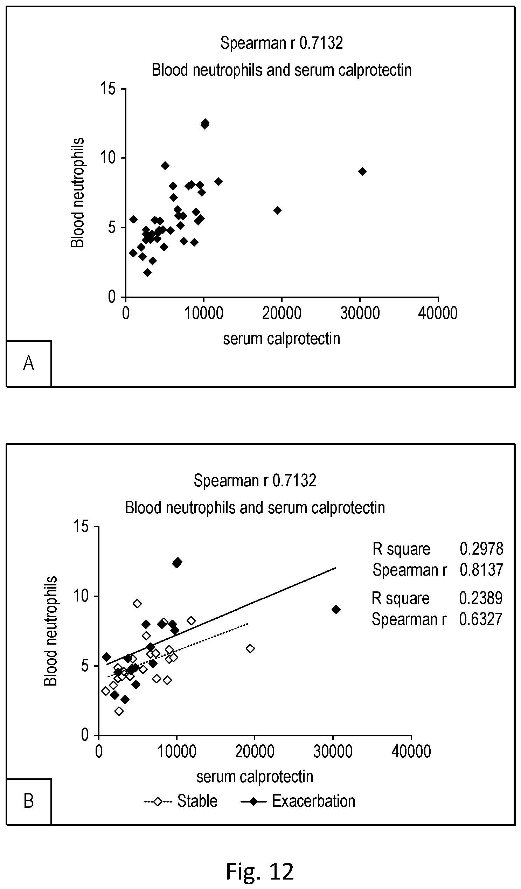

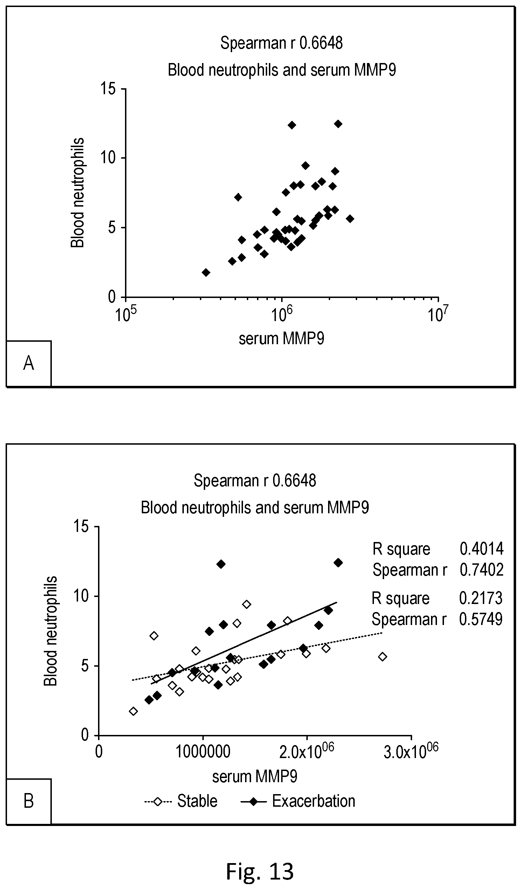

Major Basic Protein correlated well with blood eosinophils. Calprotectin correlated well with blood neutrophils. MMP9 was tested by both Mologic and Leicester and correlated well with blood neutrophils in both cases. In general, the correlation was better with the exacerbation samples than stable (Figures 11-13).

### Conclusion

As proof of principle, a panel of potential biomarkers had been selected based on blood neutrophil and eosinophil levels. Blood biomarkers may be used without the requirement of establishing a baseline value. As the test needs only to be used in the event of an exacerbation, a finger prick sample would be acceptable for less frequent testing.

### Example 2 - Biomarkers for treatment stratification for COPD patients in response to an exacerbation

### Samples

Samples (banked, frozen) were provided from University of Leicester study (MRC funded BEAT-COPD (Biomarkers to Target Antibiotic and Systemic Corticosteroid Therapy in COPD Exacerbations) study ISRCTN2422949).

Study details: From a two-staged single centre study, blood, sputum and urine samples from COPD subjects were longitudinally collected at four visit types: namely stable state (defined as being eight weeks free from an exacerbation visit), exacerbation (defined according to Anthonisen criteria [Anthonisen 2006] and healthcare utilisation), two weeks post therapy and at recovery (six weeks post exacerbation visit). Exacerbations were treated with oral corticosteroids and antibiotics according to guidelines or trial study design. Clinical data including demographics, symptoms, lung function, inflammatory profiling in blood and sputum, bacteriology including standard culture, qPCR for common pathogens and microbiomics, viruses by PCR and fungal culture were undertaken.

Laboratory methods: Blood samples were analysed for white cell count and C-reactive protein measurement as per usual care, and serum and plasma were isolated by centrifuge (10 minutes, 3000rpm) before storage at -80°C. Sputum samples were sent for standard laboratory microscopy, culture and sensitivity analysis where patients were able to produce a sample.

### Assays

Assays used for measuring the biomarkers in the samples were majority ELISAs (n=33) with some lateral flow assays (n=2) and a substrate assay for measurement of active Matrix metalloproteinase. Sample dilutions were optimised for each assay, as indicated by the table below.

| | **Assay** | **Full Marker Name** | **Supplier** | **Catalogue Number** | **Assay type** | **Sample dilution** |
|---|---|---|---|---|---|---|
| 1 | CRP | C reactive protein | R&D systems | DY1707 | ELISA | 1:100K |
| 2 | MPO | Myeloperoxidase | R&D systems | DY3174 | ELISA | 1:750 |
| 3 | MMP9 | Total Matrix Metalloproteinase -9 | R&D systems | DY911 | ELISA | 1:1000 |
| 4 | NGAL | Neutrophil gelatinase-associated lipocalin | R&D systems | DY1757 | ELISA | 1:100 |
| 5 | Periostin | Periostin | R&D systems | DY3548b | ELISA | 1:1000 |
| 6 | Calprotectin | Calprotectin | Biolegend | 439707 | ELISA | 1:200 |
| 7 | RNASE 3 | Eosinophil cationic protein | Cloud-clone | SEB758Hu | ELISA | 1:200 |
| 8 | MBP | Major Basic protein | Cloud-clone | SEB650Hu | ELISA | 1:10 |
| 9 | Active MMP | Active protease (Composite MMP 2,8,9,12,13,7) | ENZO | BML-P276-001 | Substrat e assay | 1:40 |
| 10 | HNE | Human Neutrophil Elastase | Mologic | BHNEV1 | ELISA | 1:100 |
| 11 | Fibrinogen | Fibrinogen | Abcam | 108841 | ELISA | 1:200 |
| 12 | SLPI | Secretory leukocyte protease inhibitor | Mologic | In-house developed (see below) | ELISA | 1:100 |
| 13 | IL-6 | Interleukin-6 | R&D systems | DY206 | ELISA | 1:2 |
| 14 | Fibrinogen | Fibrinogen | Mologic | In-house developed (see below) | ELISA | 1:2000 |
| 15 | fM LP | N-Formylmethionine-leucyl-phenylalanine | Mologic | BFMLPV1 | Lateral Flow | 1:10 |
| 16 | Desmosine | Desmosine | Mologic | BDESV1 | ELISA | 1:5 |
| 17 | CC16 | Club cell- 16 | R&D systems | DY4218 | ELISA | 1:50 |
| 18 | TIMP1 | Tissue inhibitor of metalloproteinase-1 | R&D systems | DY970 | ELISA | 1:600 |
| 19 | TIMP2 | Tissue inhibitor of metalloproteinase-2 | R&D systems | DY971 | ELISA | 1:600 |
| 20 | CHI3L1 | Chitinase 3 like 1 protein | R&D systems | DY2599 | ELISA | 1:500 |
| 21 | A1AT | Alpha-1 antitrypsin | Mologic | BA1ATV1 | ELISA | 1:200K |
| 22 | Ac-PGP | N-acetyl Proline-Glycine-Proline | Mologic | In-house developed (see below) | ELISA | 1:10 |
| 23 | B2M | beta 2 Microglobulin | Abcam | 108885 | ELISA | 1:1000 |
| 24 | B2M | beta 2 Microglobulin | Mologic | In-house developed (see below) | ELISA | 1:1000 |
| 25 | Cystatin C | Cystatin C | R&D systems | DY1196 | ELISA | 1:1000 |
| 26 | MMP8 | Total Matrix Metalloproteinase -8 | R&D systems | DY908 | ELISA | 1:1000 |
| 27 | RBP4 | Retinol binding protein-4 | R&D systems | DY3378 | ELISA | 1:100K |
| 28 | HSA | Human serum Albumin | R&D systems | DY1455 | ELISA | 1:100K |
| 29 | A1AT | Alpha-1 antitrypsin | Mologic | BA1ATLF | Lateral FLow | 1:200K |
| 30 | IL-1b | Interleukin-1β | R&D systems | DY201 | ELISA | 1:2 |
| 31 | IL-8 | Interleukin- 8 | R&D systems | DY208 | ELISA | 1:2 |
| 32 | Desmosine Fragment | Desmosine Fragment | Mologic | In-house developed (see below) | ELISA | 1:5 |
| 33 | Large Elastin Fragment | Large Elastin Fragment | Mologic | In-house developed (see below) | ELISA | 1:5 |
| 34 | Siglec 8 | Siglec 8 | Mologic | In-house developed (see below) | ELISA | neat |
| 35 | sRAGE | Soluble receptor for advanced glycation end products | Mologic | In-house developed (see below) | ELISA | neat |
| 36 | EDN (RNASE2) | Eosinophil-derived neurotoxin | Alpco | 30-EDNHU-E01 | ELISA | 1:20 |

The units for each assay shown in the table above were ng/ml, with the exception of IL-6, IL-1β and IL-8 which were all pg/ml.

As shown above, the inventors have developed a number of enzyme immunoassays to detect marker levels. These are described in further detail below.

### Secretory leukocyte protease inhibitor (SLPI) measurement

Disposable 96-well polystyrene plates were obtained from Fisher Scientific. The plate was sensitised with sheep anti SLPI (Mologic, CF1099 IgG cut) at 20µg/ml in PBS overnight at ambient, 100µl/well. After a wash step, the sensitised-well surfaces were blocked with buffer 1 (10mM phosphate buffered saline pH7.5, supplemented with 1% (w/v) BSA) with 120µl/well for 1hour at room temperature.

*Assay running procedure:* recombinant SLPI (R&D systems cat. 1274-P1) was diluted in buffer 2 (10mM phosphate buffered saline pH7.5, supplemented with 0.1% (v/v) Tween20 and 1% (w/v) BSA) to give concentrations between 0.781 and 50ng/ml (1 in 2 serial dilution) to generate the standard curve. The standard and sample (diluted 1 in 100 in buffer 2) was added to the plate 100µl/well after a wash step and incubated for 1.5 hours at room temperature with gentle agitation. After a further wash step, mouse anti-SLPI (Alere, 431) alkaline phosphatase conjugate at 1 in 2500 diluted in sample diluent were added 100µl/well and incubated for 1 hour at room temperature with gentle agitation. After the final plate wash, the colour reaction was initiated with the addition of 100µl of pNPP solution to each well. The absorbance was measured at 405 using an Omega plate reader and the standard curve was approximated in a sigmoid 4 parameter logistic model.

### Fibrinogen measurement

Disposable 96-well polystyrene plates were obtained from Fisher Scientific. The plate was sensitised with sheep anti Fibrinogen (Mologic, CF1765 affinity purified) at 2µg/ml in PBS overnight at ambient, 100µl/well. After a wash step, the sensitised-well surfaces were blocked with buffer 1 (10mM phosphate buffered saline pH7.5, supplemented with 1% (w/v) BSA) with 120µl/well for 1hour at room temperature.

*Assay running procedure:* Fibrinogen (Scipac) was diluted in buffer 2 (10mM phosphate buffered saline pH7.5, supplemented with 0.1% (v/v) Tween20 and 1% (w/v) BSA) to give concentrations between 0.625 and 40ng/ml (1 in 2 serial dilution) to generate the standard curve. The standard and sample (diluted 1 in 2000 in buffer 2) was added to the plate 100µl/well after a wash step and incubated for 1 hour at room temperature with gentle agitation. After a further wash step, sheep anti Fibrinogen (Mologic, CF1766) alkaline phosphatase conjugate at 1 in 4000 diluted in sample diluent were added 100µl/well and incubated for 1 hour at room temperature with gentle agitation. After the final plate wash, the colour reaction was initiated with the addition of 100µl of pNPP solution to each well. the absorbance was measured at 405 using an Omega plate reader and the standard curve was approximated in a sigmoid 4 parameter logistic model.

### B2M measurement

Disposable 96-well polystyrene plates were obtained from Fisher Scientific. The plate was sensitised with sheep anti-bet-2-Microglobulin (B2M) (Ig Innovations, NS15 affinity purified) at 1µg/ml in PBS overnight at ambient, 100µl/well. After a wash step, the sensitised-well surfaces were blocked with buffer 1 (10mM phosphate buffered saline pH7.5, supplemented with 1% (w/v) BSA) with 120µl/well for 1hour at room temperature.

*Assay running procedure:* B2M (Scipac) was diluted in buffer 2 (10mM phosphate buffered saline pH7.5, supplemented with 0.1% (v/v) Tween20 and 1% (w/v) BSA) to give concentrations between 0.01 and 50ng/ml (1 in 4 serial dilution) to generate the standard curve. The standard and sample (diluted 1 in 1000 in buffer 2) was added to the plate 100µl/well after a wash step and incubated for 1 hour at room temperature with gentle agitation. After a further wash step, sheep anti B2M (Ig Innovations, NS16) HRP conjugate at 1 in 20,000 diluted in sample diluent were added 100µl/well and incubated for 1 hour at room temperature with gentle agitation. After the final plate wash, the colour reaction was initiated with the addition of 100µl of OPD substrate to each well. the absorbance was measured at 450 using an Omega plate reader and the standard curve was approximated in a sigmoid 4 parameter logistic model.

### Siglec 8 measurement

Disposable 96-well polystyrene plates were obtained from Fisher Scientific. The plate was sensitised with Sheep anti Siglec 8 (Mologic, SA122 purified against peptide MOL624) at 2µg/ml in PBS overnight at ambient, 120µl/well. After a wash step, the sensitised-well surfaces were blocked (buffer 3) with 120µl/well for 1 hour at room temperature.

*Assay running procedure:* Recombinant SIGLEC8 binding domain (Mologic, York) was diluted in buffer 3 to give concentrations between 7.81 and 500ng/ml to generate the standard curve. The standard and serum sample (neat) were added to the plate 100µl/well after a wash step and incubated for 1 hour at room temperature with gentle agitation. After a further wash step, sheep anti-siglec 8 (Mologic, SA122 purified against Siglec 8) alkaline phosphatase conjugate at 1 in 2000 were added 100µl/well and incubated for 1 hour at room temperature with gentle agitation. After the final plate wash, the colour reaction was initiated with the addition of 100µL of pNPP solution to each well. The absorbance was measured at 405 using an Omega plate reader and the standard curve was approximated in a sigmoid 4 parameter logistic model.

### Soluble receptor for advanced glycation end products (sRAGE) measurement

Disposable 96-well polystyrene plates were obtained from Fisher Scientific. The plate was sensitised with sheep anti sRAGE (Mologic, SA056 affinity purified) at 1µg/ml in PBS overnight at ambient, 100µl/well. After a wash step, the sensitised-well surfaces were blocked (buffer 2) with 120µl/well for 1hour at room temperature.

*Assay running procedure:* recombinant sRAGE (Novoprotein cat. C423) was diluted in buffer 2 to give concentrations between 0.02 and 5ng/ml (1 in 2 serial dilution) to generate the standard curve. After a wash step, the standard and sample (neat) was added to the plate 50µl/well with 50µl/well of sample diluent and incubated for 1.5 hours at room temperature with gentle agitation. After a further wash step, rabbit anti-sRAGE (Mologic, RA040) alkaline phosphatase conjugate at 1 in 5000 diluted in sample diluent were added 100µl/well and incubated for 1 hour at room temperature with gentle agitation. After the final plate wash, the colour reaction was initiated with the addition of 100µl of pNPP solution to each well. The absorbance was measured at 405 using an Omega plate reader and the standard curve was approximated in a sigmoid 4 parameter logistic model.

### Ac-PGP, Desmosine and LEF

Ac-PGP, Desmosine and LEF levels were measured using an in-house developed ELISA lateral flow assay based on a competition principle, where free marker in the sample competed with bound marker on a solid phase for a sheep polyclonal antibody conjugated to alkaline phosphatase. After washing, an alkaline phosphate enzyme substrate was added and subsequent colour was measured at 405nm.

### Results

### Correlations with eosinophil and neutrophil levels - high eosinophilllow neutrophil group

This subgroup of COPD samples consisted of samples collected from patients with high eosinophil and low neutrophil levels (1 sample with a neutrophil level of 10.85 which was just above the cut-off of 10 (× 10⁹ cells/L)). The eosinophil cut-off level was 0.3 (× 10⁹ cells/L) with levels of eosinophil ranging from 0.34 to 2.09. This is shown in the table below:

| **BEAT** - **COPD (SERUM)** | **n** | **status** | **Range** |
|---|---|---|---|
| Neutrophil | 38 | Low | 2.5-8.96 |
| | 1 | High | 10.85 |
| Eosinophil | 0 | Low | - |
| | 39 | High | 0.34-2.09 |
| Total | 39 | | |

Multiple linear regression was performed using SPSS (version 21) for all analysis. The Automated Linear Modelling function in SPSS provides data transformation by trimming outliers. This was employed to generate models to predict neutrophil and eosinophil levels by combination of markers using a stepwise method with entry and removal of markers based on the information criterion (AICC).

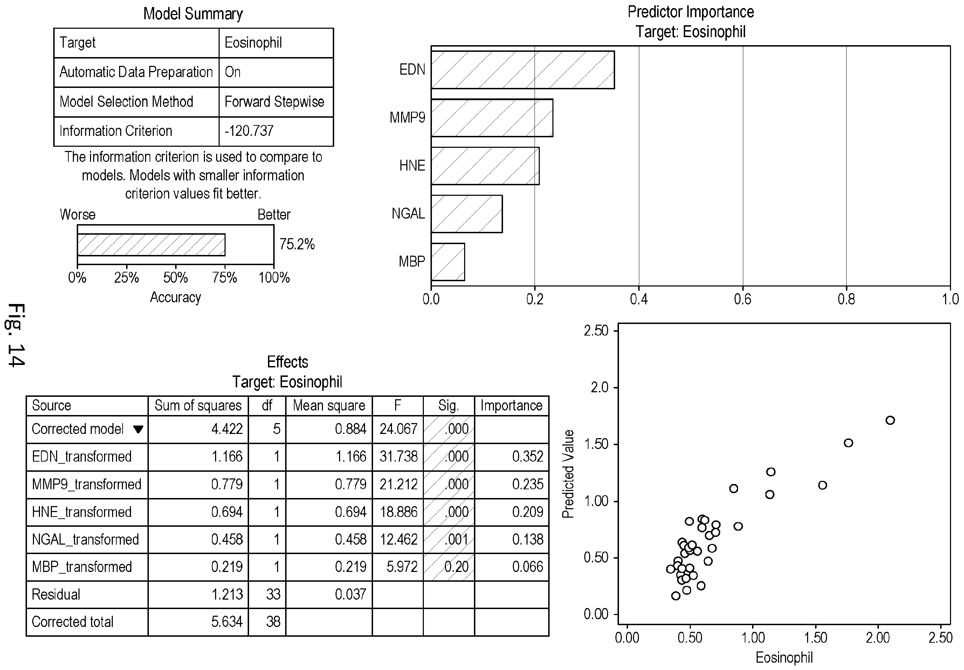

Model 1: For the eosinophil correlation, a combination of 5 biomarkers produced an R² of 0.752. The 5 biomarkers with significant levels in this model were: EDN (<0.001), MMP9 (<0.001), HNE ((<0.001), NGAL (0.001) and MBP (0.020) (in level of importance). Of note is that HNE and NGAL are negatively correlated with eosinophil levels. The results are also shown in Figure 14.

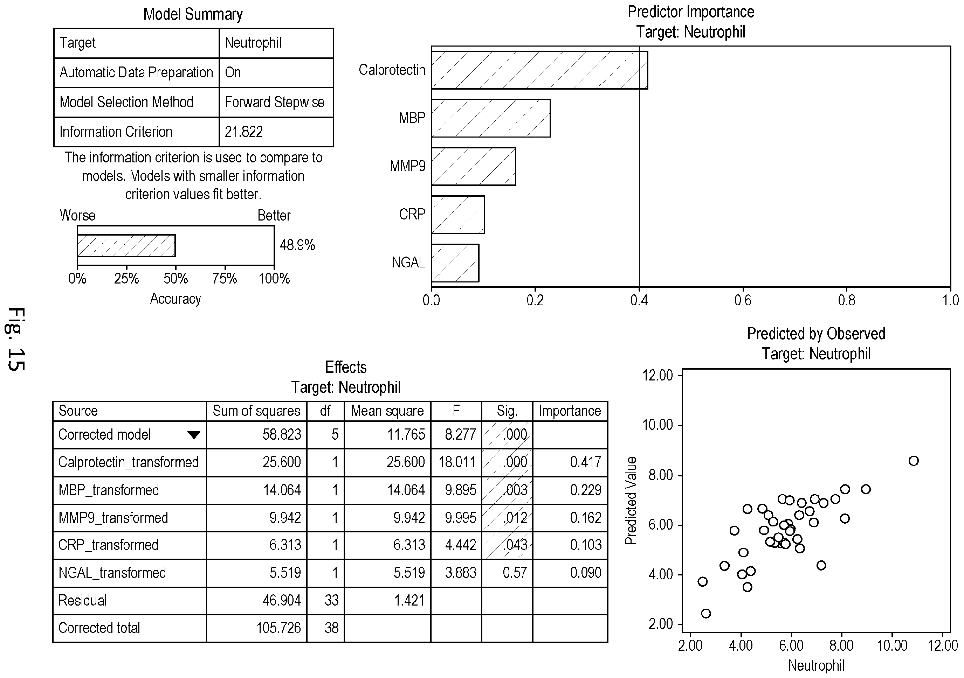

Model 2: For the neutrophil correlations a combination of 5 biomarkers produced an R² of 0.489. The 5 biomarkers with significance levels in this model were: Calprotectin (0.00), MBP (0.003), MMP9 (0.012), CRP (0.043) and NGAL (0.057) (in level of importance). Of note is that MBP, CRP and NGAL were negatively correlated with neutrophil levels. The results are also shown in Figure 15.

Conclusion: The models generated significantly correlate with eosinophil and neutrophil levels. Thus, the models can be used to predict eosinophil and neutrophil levels.

### Correlations with neutrophil levels - Mixed levels of low and high eosinophil and neutrophil group

This subgroup of COPD samples consisted of samples collected from patients with mixed levels of high and low eosinophil and neutrophil levels. The neutrophil cut-off level was 10 (× 10⁹ cells/L) and the neutrophil levels ranged from 2.5-18.96. The eosinophil biomarkers selected from example 1 were not available for all samples; therefore only neutrophil correlations were undertaken in this analysis. This is shown in the table below:

| **BEAT** - **COPD (SERUM)** | **n** | **status** | **Range** |
|---|---|---|---|
| Neutrophil | 38 | Low | 2.5-8.96 |
| | 22 | High | 10-18.94 |
| Eosinophil | 17 | Low | 0.03-0.28 |
| | 43 | High | 0.3-2.09 |
| Total | 60 | | |

Multiple linear regression was performed using a forward stepwise function using SPSS for all analysis. For each model developed the optimal sensitivity and Specificity was calculated based on the receiver operating characteristic (ROC) curve and area under the curve (AUC).

There were 3 models generated with a range of 3-6 biomarkers with similar R² values:
- Model 3: MBP, Calprotectin and A1AT (as measured by Lateral flow) - producing an R² of 0.514. Of note is that MBP, and A1AT were negatively correlated with neutrophil levels.
- Model 4: MBP, Calprotectin, A1AT (as measured by Lateral flow), MMP9 and CRP - producing an R² of 0.518. Of note is that MBP, A1AT and CRP were negatively correlated with neutrophil levels.
- Model 5: MBP, Calprotectin. A1AT (as measured by Lateral flow), MMP9, CRP and NGAL - producing an R² of 0.514. Of note is that MBP, A1AT and CRP were negatively correlated with neutrophil levels.

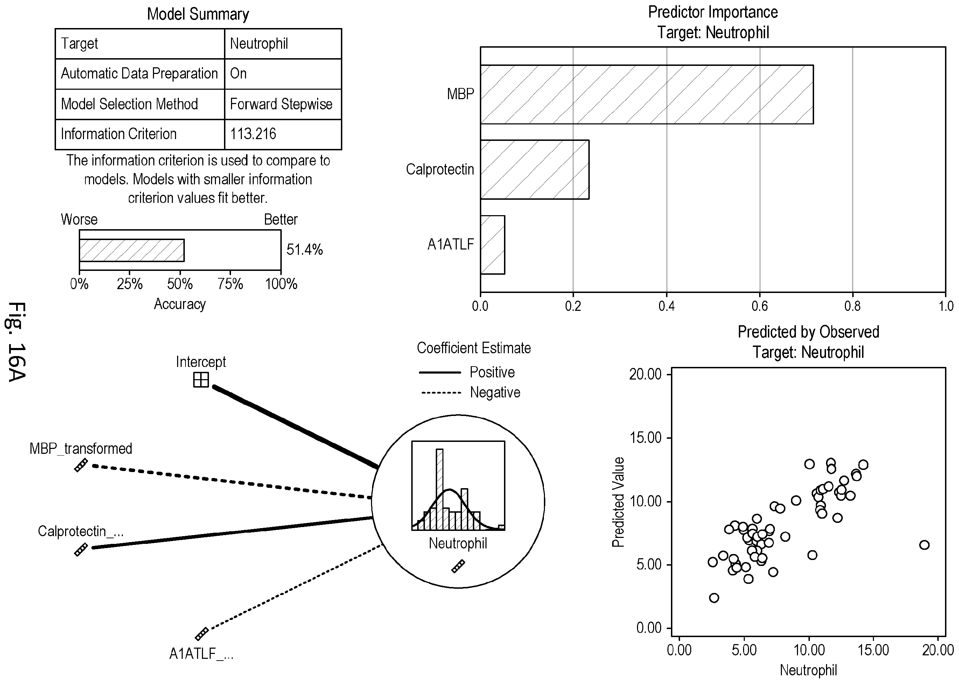

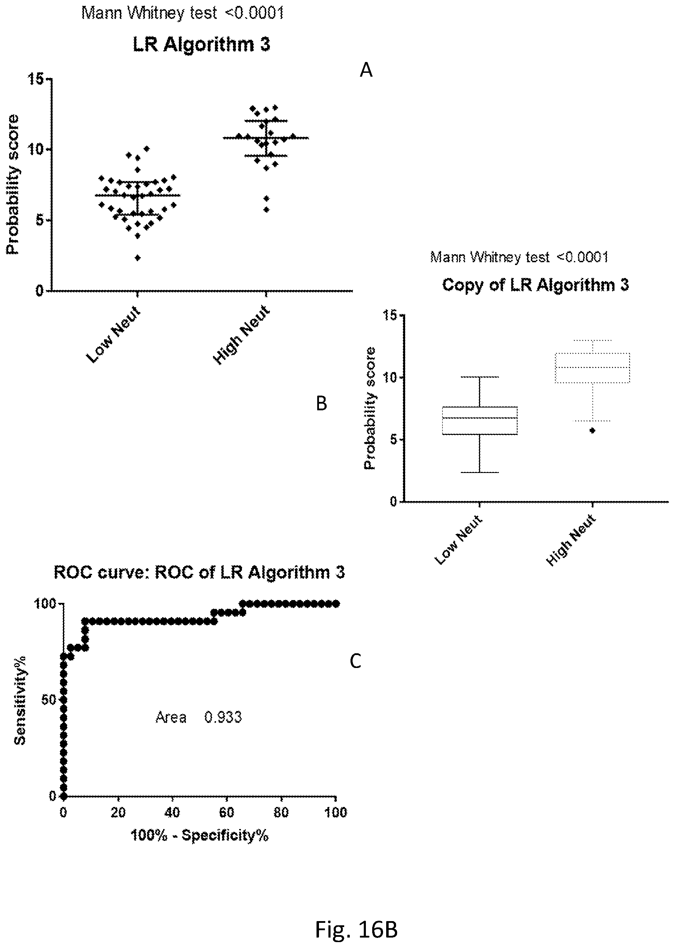

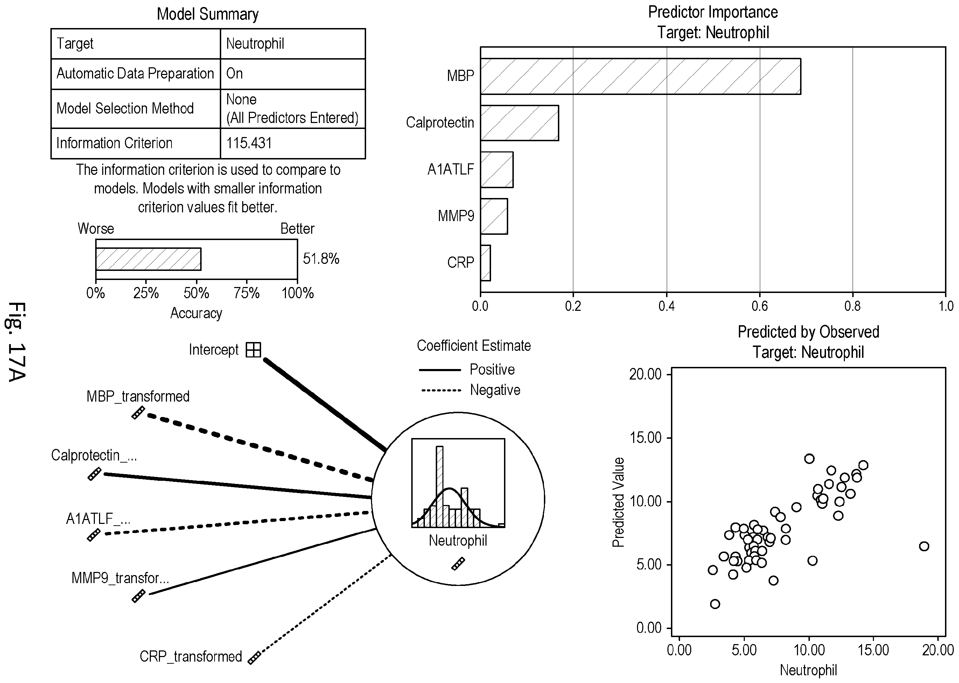

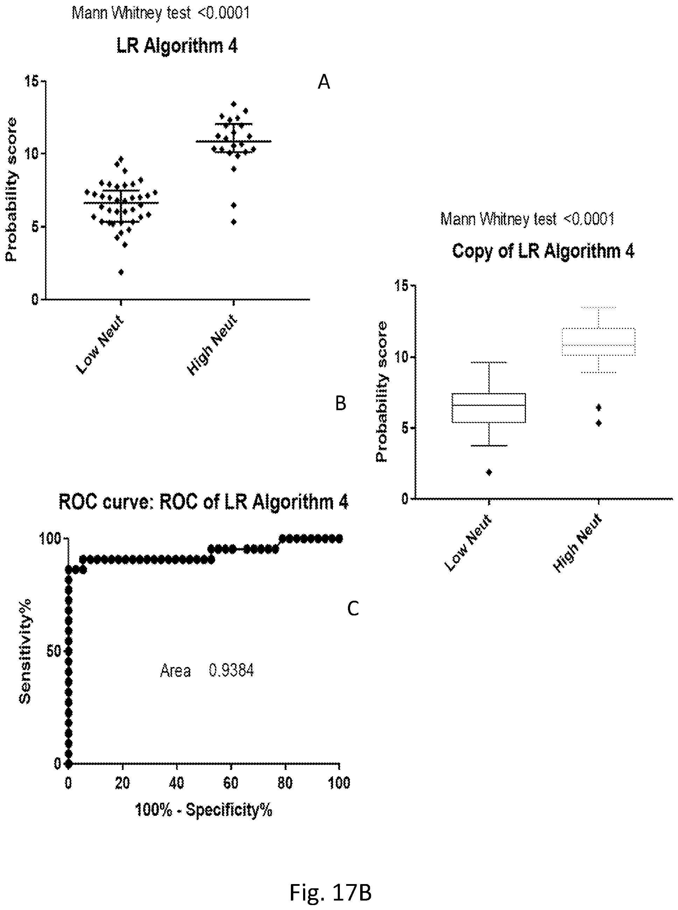

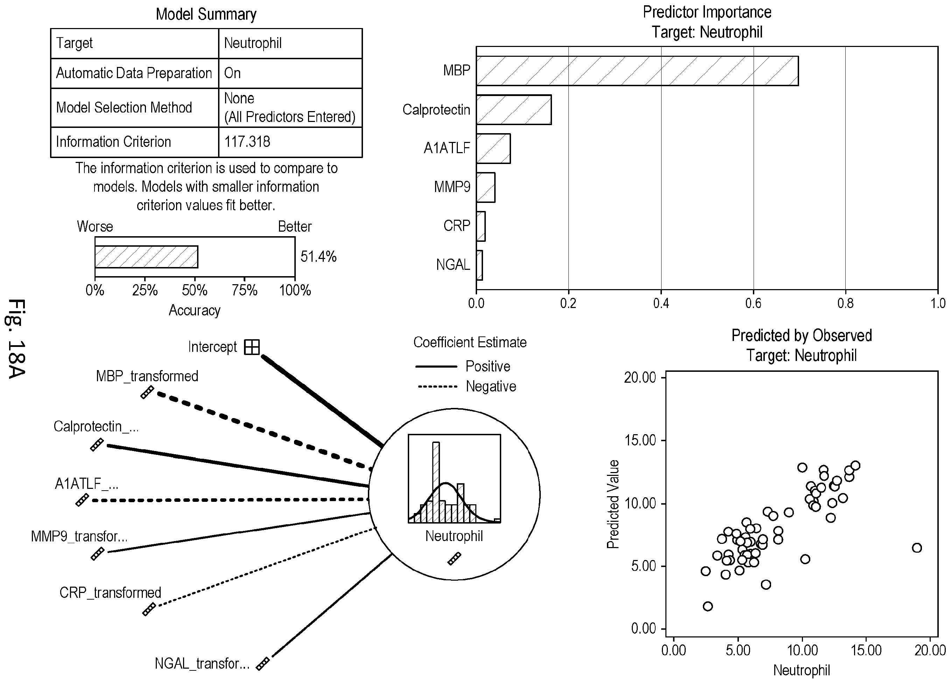

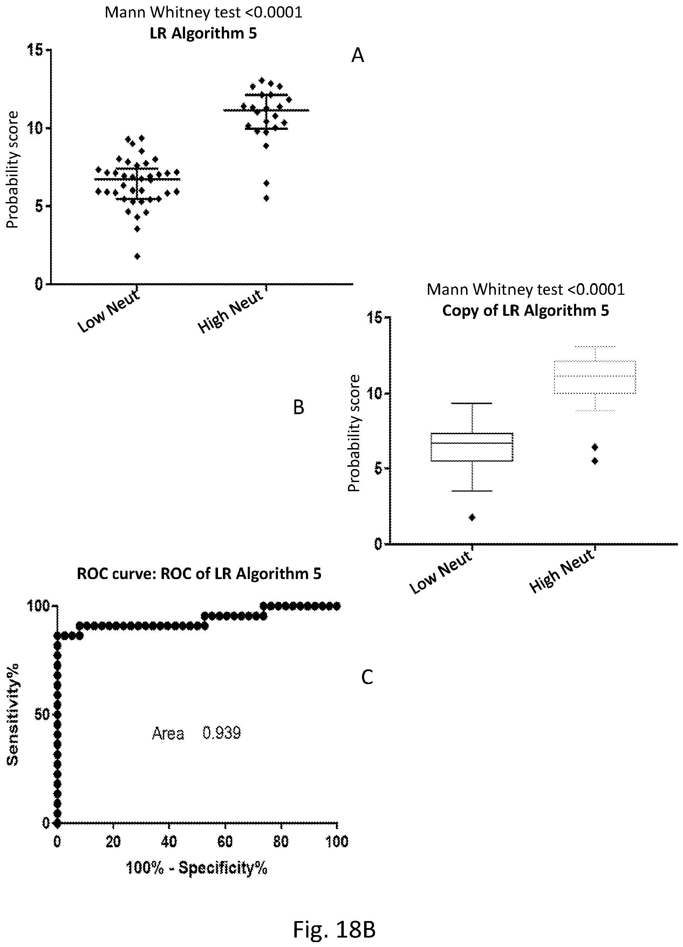

For all three models an AUC of approximately 0.93 was obtained. The sensitivity for all models using different cut off levels were 90.9% whereas the specificity varied slightly with the best result of 94.7% obtained from model 2 (slightly improved from the other two models). The results for models 3-5 are shown in Figures 16-18 respectively.

Conclusion: The models generated significantly correlate with neutrophil levels, with similar R² values, AUC and sensitivity and specificity performance. The results indicate that the core biomarkers were MBP, Calprotectin and A1AT, with minor performance improvement with the addition of MMP9 and CRP. Thus, the models can be used to predict neutrophil levels.

### Correlations with neutrophil levels - Mixed levels of low and high eosinophil and neutrophil group - larger group analysis and sub analysis

BEAT-COPD (n=601) samples were subdivided as shown in the table below according to eosinophil and neutrophil levels. The groups were defined using a cut-off of 0.3 × 10⁹ cells/L for eosinophils and 10 × 10⁹ cells/L for neutrophils.

| **BEAT** - **COPD (SERUM)** | **n** | | **status** | **Range** |
|---|---|---|---|---|
| Subgroup 1 (L/L) | 402 | Neutrophil | Low | 1.01-9.98 |
| | | Eosinophil | low | 0-0.29 |
| Subgroup 2 | 172 | Neutrophil | Low | 1.82-9.92 |
| (L/H) | | Eosinophil | High | 0.3-2.09 |
| Subgroup 3 (H/L) | 17 | Neutrophil | High | 10.66-18.94 |
| | | Eosinophil | Low | 0.03-0.28 |
| Subgroup 4 (H/H) | 5 | Neutrophil | High | 10-10.99 |
| | | Eosinophil | High | 0.3-0.68 |
| Total | 601 | | | |

Multiple linear regression was performed using a forward stepwise function using SPSS for all analysis. For each model developed the optimal sensitivity and specificity was calculated based on the ROC curve and AUC.

Multiple linear regression models were generated from data from all groups (n=601) and from a further stratified group - exclusion of sub-group 2 which contained all the samples with 'high eosinophil' levels (n=402). The developed models were then characterised on a further subgroup which removed all the grey zone samples - those samples with neutrophil levels between 5-10. This new group consisted of total number of 247 samples.

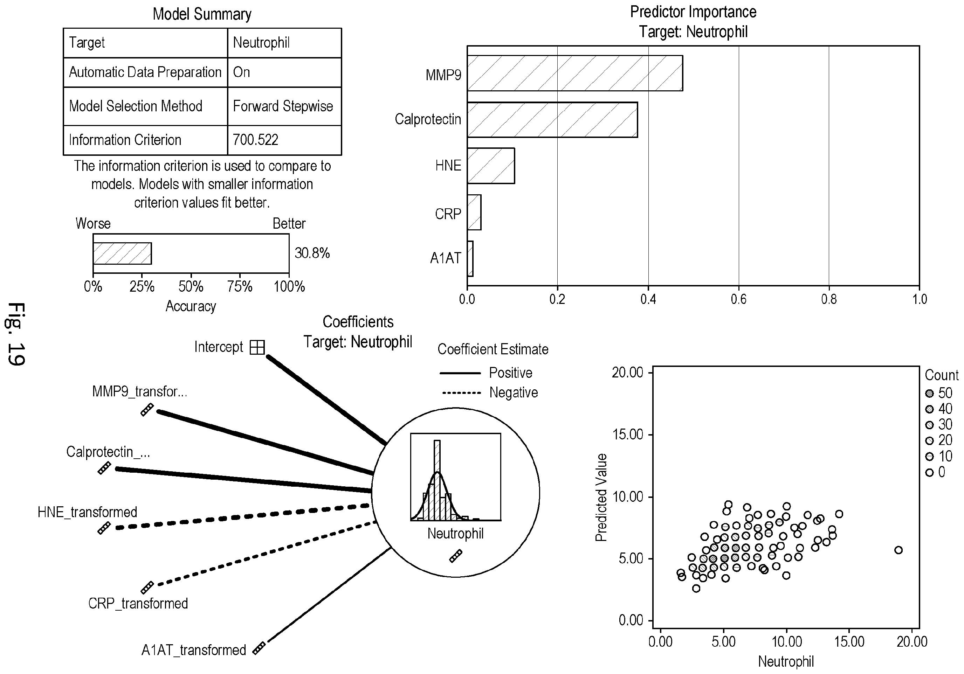

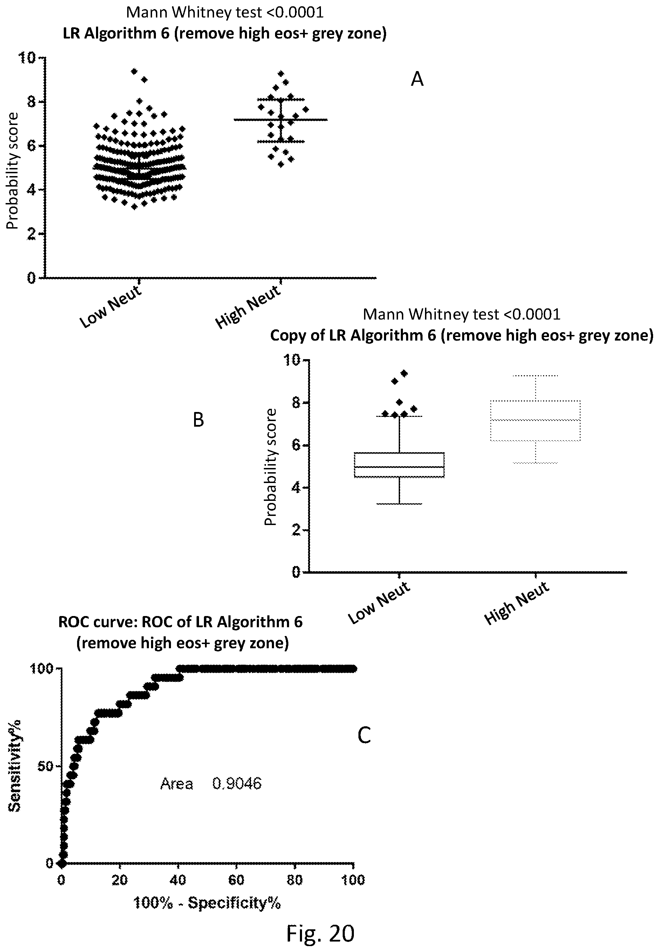

Model 6 was generated from all 601 samples and characterised on the other 2 subgroups described in the methods.
- Model 6: MMP9, Calprotectin, HNE, CRP and A1AT - producing an R² of 0.308. Of note is that HNE and CRP were negatively correlated with neutrophil levels (see Figure 19).
   o N = 601, ROC AUC of 0.8071, sensitivity 73%, specificity 74%
   o N = 402, ROC AUC of 0.8143, sensitivity 77%, specificity 75%
   o N = 247, ROC AUC of 0.9046, sensitivity 82%, specificity 80% (Figure 20)

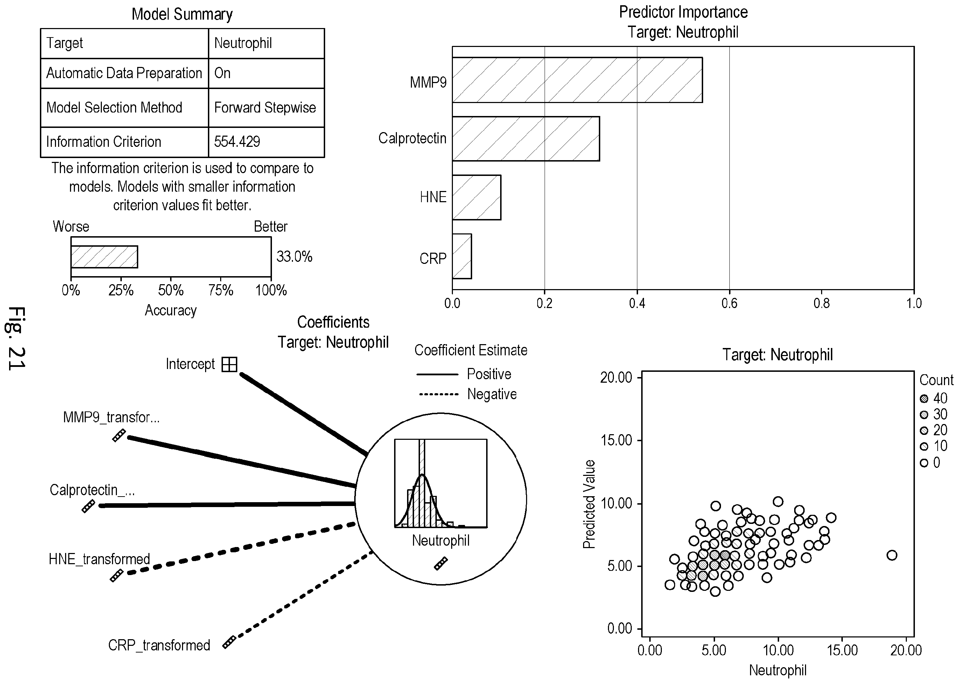

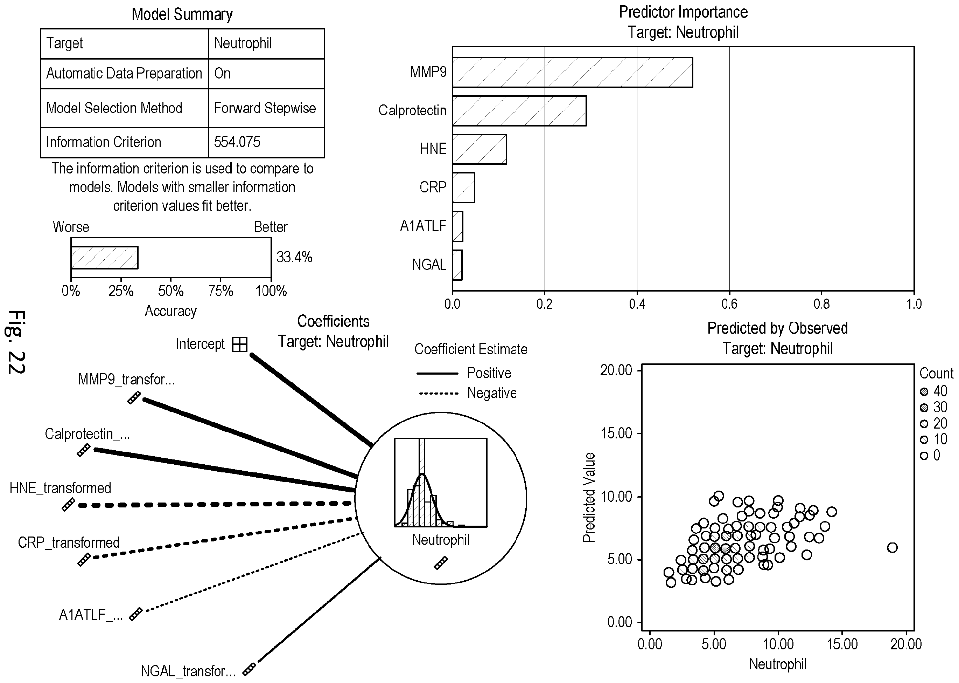

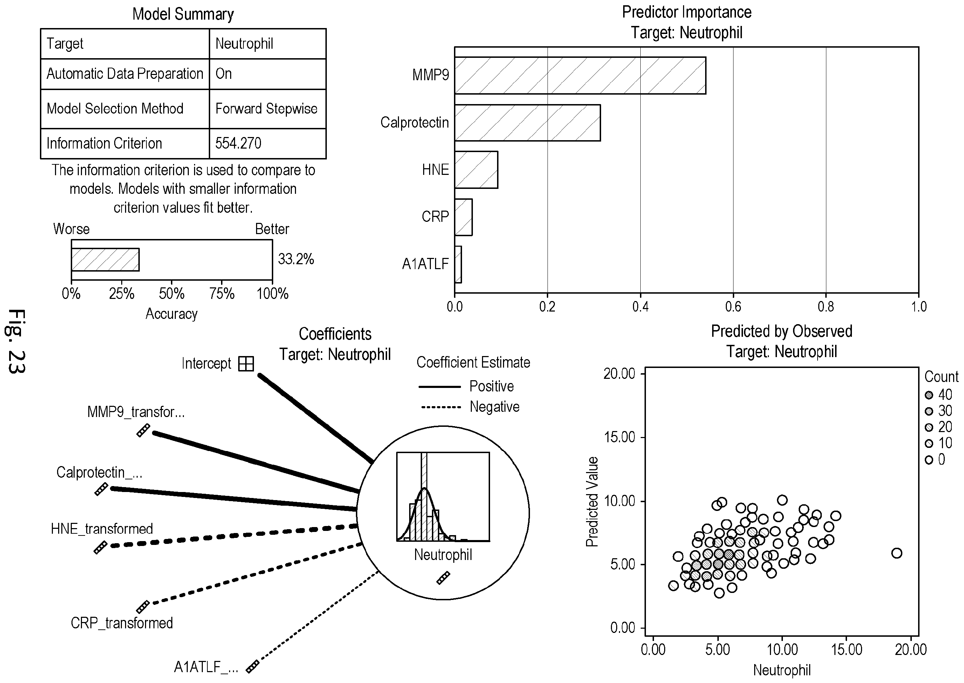

Models 7-9 were created on the newly defined group data (removed sub-group 2) and characterised on the further stratified group (removed grey-zone samples).
- Model 7: MMP9, Calprotectin, HNE and CRP- producing an R² of 0.33. Of note is that HNE and CRP were negatively correlated with neutrophil levels (see Figure 21).
   ∘ N = 402, ROC AUC of 0.8263, sensitivity 77%, specificity 76%
   ∘ N = 247, ROC AUC of 0.8948, sensitivity 82%, specificity 78%
- Model 8: MMP9, Calprotectin, HNE, CRP, A1AT (LF) and NGAL - producing an R² of 0.334. Of note is that HNE, CRP and A1AT were negatively correlated with neutrophil levels (see Figure 22).
   ∘ N = 402, ROC AUC of 0.8341, sensitivity 77%, specificity 78%
   ∘ N = 247, ROC AUC of 0.8945, sensitivity 82%, specificity 79%
- Model 9: MMP9, Calprotectin, HNE, CRP and A1AT (LF) - producing an R² of 0.332. Of note is that HNE, CRP and A1AT were negatively correlated with neutrophil levels (see Figure 23).
   ∘ N = 402, ROC AUC of 0.8301, sensitivity 77%, specificity 78%
   ∘ N = 247, ROC AUC of 0.8936, sensitivity 82%, specificity 79%

The results are summarised in the two following tables. The first table below describes the results of all 4 models tested on different stratified groups. The cohorts were split into 2 groups based on neutrophil levels with a cut-off of 10. Model 6 which was derived from all 601 samples was applied to all data (all 601) samples and a further stratified sample which had removed all high eosinophil samples in the low group.

| Model | Biomarkers | Total Number | Number Low Neutrophil | Number High Neutrophil | Mann Whitney test p value | AUC | Sens | Spec | Cut off |
|---|---|---|---|---|---|---|---|---|---|
| Model 6 | MMP9 | 601 | 579 | 22 | <0.0001 | 0.8071 | 72.73% | 73.92% | >6.33 |
| | Calprotectin | | | | | | | | |
| | HNE | | | | | | | | |
| | CRP | | | | | | | | |
| | A1AT | | | | | | | | |
| Model 6 tested or stratified cohort | MMP9 | 424 | 402 | 22 | <0.0001 | 0.8143 | 77.27% | 74.63 | >6.305 |
| | Calprotectin | | | | | | | | |
| | HNE | | | | | | | | |
| | CRP | | | | | | | | |
| | A1AT | | | | | | | | |
| Model 7 | MMP9 | 424 | 402 | 22 | <0.0001 | 0.8263 | 77.27% | 75.87 | >6.555 |
| | Calprotectin | | | | | | | | |
| | HNE | | | | | | | | |
| | CRP | | | | | | | | |
| Model 8 | MMP9 | 424 | 402 | 22 | <0.0001 | 0.8341 | 77.27% | 77.61 | >6.685 |
| | Calprotectin | | | | | | | | |
| | HNE | | | | | | | | |
| | CRP | | | | | | | | |
| | A1AT (LF) | | | | | | | | |
| | NGAL | | | | | | | | |
| Model 9 | MMP9 | 424 | 402 | 22 | <0.0001 | 0.8301 | 77.27% | 78.11% | >6.705 |
| | Calprotectin | | | | | | | | |
| | HNE | | | | | | | | |
| | CRP | | | | | | | | |
| | A1AT(LF) | | | | | | | | |

The next table (below) describes the results of all 4 models as applied to a second stratified group which removed all grey zone samples (neutrophil levels 5-10) from the low group.

| Model | Biomarkers | Total Number | Number Low Neutrophil | Number High Neutrophil | Mann Whitney test p value | AUC | Sens | Spec | Cut off |
|---|---|---|---|---|---|---|---|---|---|
| Model 6 | MMP9 | 247 | 225 | 22 | <0.0001 | 0.9046 | 81.82% | 80% | >5.85 |
| | Calprotectin | | | | | | | | |
| | HNE | | | | | | | | |
| | CRP | | | | | | | | |
| | A1AT | | | | | | | | |
| Model 7 | MMP9 | 247 | 225 | 22 | <0.0001 | 0.8948 | 81.82% | 77.78% | >5.86 |
| | Calprotectin | | | | | | | | |
| | HNE | | | | | | | | |
| | CRP | | | | | | | | |
| Model 8 | MMP9 | 247 | 225 | 22 | <0.0001 | 0.8945 | 81.82% | 78.67% | >5.865 |
| | Calprotectin | | | | | | | | |
| | HNE | | | | | | | | |
| | CRP | | | | | | | | |
| | A1AT (LF) | | | | | | | | |
| | NGAL | | | | | | | | |
| Model 9 | MMP9 | 247 | 225 | 22 | <0.0001 | 0.8936 | 81.82% | 78.67% | >5.865 |
| | Calprotectin | | | | | | | | |
| | HNE | | | | | | | | |
| | CRP | | | | | | | | |
| | A1AT (LF) | | | | | | | | |

Conclusion: There are clear linear regression models that can be used for neutrophil level prediction. From 33 biomarkers this is possible with a combination of key 4 biomarkers MMP9, Calprotectin, HNE and CRP, with some added value by the addition of A1AT and NGAL. When removing the 'grey-zone' samples i.e. those with neutrophil values between 5 and 10, this improves the performance as would be expected with standard variation around a single cut-off value.

### Example 3 - Eosinophil and neutrophil levels in stable, exacerbation and mixed samples and marker correlations

A total of 362 samples were analysed with a median and interquartile range of 5.18 (4.0-6.3) for blood neutrophil levels and 0.22 (0.1-0.4) for eosinophil levels, as shown in the table below.

| | **Neutrophil** | **Eosinophil** |
|---|---|---|
| Minimum | 1.36 | 0 |
| 25% Percentile | 4.048 | 0.13 |
| Median | 5.175 | 0.22 |
| 75% Percentile | 6.268 | 0.3625 |
| Maximum | 12.46 | 1.13 |

Further sub group analysis was performed, stable group (n=322) exacerbation group (n=40) presented different neutrophil and eosinophil characteristics (see table below). Evaluation of neutrophil and eosinophil levels with discrimination between stable and exacerbation states produced a Mann Whitney test p value of 0.0002 and 0.5591 respectively. Thus, neutrophil levels significantly increased in exacerbation samples as compared with stable samples whereas eosinophil levels showed no significant change.

| | **Neutrophil** | | **Eosinophil** | |
|---|---|---|---|---|
| | Stable | Exacerbation | Stable | Exacerbation |
| Minimum | 1.36 | 2.63 | 0 | 0.03 |
| 25% Percentile | 3.998 | 5.008 | 0.13 | 0.135 |
| Median | 4.99 | 6.08 | 0.225 | 0.22 |
| 75% Percentile | 6.173 | 7.998 | 0.38 | 0.29 |
| Maximum | 10.85 | 12.46 | 1.13 | 0.88 |

### Biomarker data

### Biomarkers that correlate with Neutrophil levels

A Spearman's r value >0.4 was deemed to be significant. To note, CRP levels measured by the hospital labs at time of collection correlated with the exacerbation samples only with an r-value of 0.55 with exacerbation samples and 0.20 with stable samples. Calprotectin was the only marker that correlated with neutrophil levels in both stable and exacerbation states.

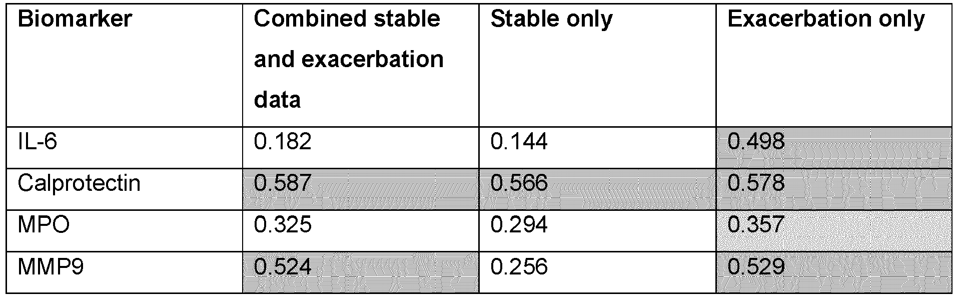

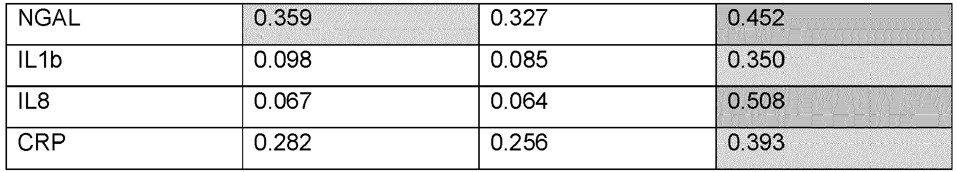

### Biomarkers that correlate with eosinophil levels

A Spearman's r value >0.4 was deemed to be significant. To note, other promising biomarkers i.e. EDN were not measured. MBP correlated with eosinophil levels in both stable and exacerbation states.

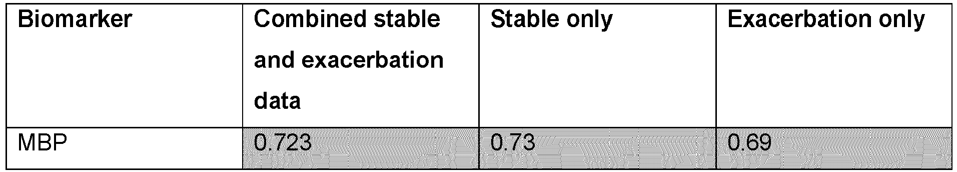

### Example 5 - Biomarkers for treatment stratification for COPD patients in response to an exacerbation - large study

### Samples

A total of 292 patients were recruited into the study (44.7% Males). All samples were collected from blood of patients diagnosed with COPD and during an COPD exacerbation.

The data exists in a matrix with 16 biomarkers measurements. The frequency of measurements varied between the 2 classes for the Neutrophil model and the Eosinophil model. The Eosinophil model contained 70 high cases and 192 low cases, and the Neutrophil model contained 75 high and 187 low cases.

The cut-off used to discriminate between low and high eosinophil levels is 300 cells/µL and low and high neutrophil levels is 1000 cells/µL.

### Results and analysis

### A. Preliminary biomarker selection

### Neutrophil subset

The levels of 16 biomarkers in blood samples from patients with low and high neutrophil levels were measured. The biomarker with the greatest significant difference between the low and high subgroups is MMP9 with a p value <0.0001 and an AUC of 0.75 (95% Cl 0.68-0.82 p value <0.0001). Additional biomarkers with a positive correlation are MMP8, MPO, PCT, CRP, HNE, NGAL and Calprotectin and with a negative correlation is LTB4 as determined by AUC values above or below 0.5.

| | | **Neutrophil <1000** | | **Neutrophil >1000** | | **t test** | **ROC** | | |
|---|---|---|---|---|---|---|---|---|---|
| **Biomarker** | Unit | Median | IQR | Median | IQR | p value | AUC | 95 Cl | sig |
| **HNE** | ng/ml | 10.8 | 5.8-17.0 | 13.8 | 8.1-28.9 | **0.000729544** | **0.59** | **0.51-0.67** | **0.01800398** |
| **RNASE3** | ng/ml | 5.1 | 2.3-15.7 | 5.1 | 2.0-9.6 | 0.775587778 | 0.48 | 0.40-0.56 | 0.568089722 |
| **Lactoferrin** | ng/ml | 21.4 | 7.4-34.8 | 20.5 | 5.4-39.4 | 0.60217932 | 0.50 | 0.42-0.58 | 0.929574107 |
| **Calprotectin** | ng/ml | 16239 | 7764-40000 | 26731 | 9553-44454 | **0.043347224** | **0.59** | **0.51-0.66** | **0.078618207** |
| **EDN** | ng/ml | 15.5 | 9.5-25.1 | 13.6 | 10.9-21.7 | 0.292616197 | 0.47 | 0.40-0.55 | 0.520214613 |
| **LTB4** | pg/ml | 70.8 | 30.5-117.3 | 31.6 | 0-91.2 | **0.017534999** | **0.39** | **0.31-0.46** | **0.003685023** |
| **MBP** | ng/ml | 1864 | 1150-2681 | 1648 | 927.7-3272 | 0.580089356 | 0.50 | 0.42-0.58 | 0.947509512 |
| **SuPAR** | ng/ml | 2099 | 1672-2470 | 2275 | 1725-2616 | 0.282353394 | **0.56** | 0.48-0.64 | 0.162426795 |
| **CRP** | ng/ml | 9820 | 3543-22614 | 16659 | 6461-41237 | **0.002670766** | **0.60** | **0.53-0.68** | **0.008101424** |
| **MMP8** | pg/ml | 16004 | 9420-30605 | 32699 | 14820-59998 | **2.40476E-05** | **0.67** | **0.59-0.74** | **2.13399E-05** |
| **MMP9** | ng/ml | 488 | 307.6-822.3 | 1229 | 572-1774 | **1.98043E-11** | **0.75** | **0.68-0.82** | **2.06812E-10** |
| **MPO** | pg/ml | 74848 | 54287-109333 | 105585 | 78555-165884 | **0.00013088** | **0.66** | **0.58-0.73** | **7.75398E-05** |
| **NGAL** | ng/ml | 286.8 | 193.2-391.2 | 355.1 | 272.3-451.7 | **0.005262488** | **0.63** | **0.56-0.71** | **0.000796647** |
| **PCT** | ng/ml | 19.8 | 0-52.2 | 41.5 | 12.5-108 | **0.007506713** | **0.67** | **0.54-0.69** | **0.003059904** |
| **A1AT** | mg/ml | 20.9 | 11.8-1254283 | 16.7 | 10.7-1161259 | 0.09441043 | 0.45 | 0.37-0.52 | 0.166257366 |
| **IgE** | ng/ml | 299.2 | 0-833.6 | 490.2 | 189.7-962.3 | 0.906877455 | **0.57** | 0.50-0.64 | 0.078340166 |

The findings may be summarised as shown below.

| **Biomarker** | **p value** | **AUC** | **ROC significance** |
|---|---|---|---|
| HNE | 0.0007 | 0.59 | 0.018 |
| RNASE3 | 0.7756 | 0.48 | 0.5681 |
| Lactoferrin | 0.6022 | 0.50 | 0.9296 |
| Calprotectin | 0.0434 | 0.59 | 0.0286 |
| EDN | 0.2926 | 0.47 | 0.5202 |
| LTB4 | 0.0175 | 0.39 | 0.0037 |
| MBP | 0.5801 | 0.50 | 0.9475 |
| SuPAR | 0.2824 | 0.56 | 0.1624 |
| CRP | 0.0027 | 0.60 | 0.0081 |
| MMP8 | 2.405E-05 | 0.67 | 2.134E-05 |
| MMP9 | 1.980E-11 | 0.75 | 2.068E-10 |
| MPO | 0.0001 | 0.66 | 7.754E-05 |
| NGAL | 0.0053 | 0.63 | 0.0008 |
| PCT | 0.0075 | 0.62 | 0.0031 |
| A1AT | 0.0944 | 0.45 | 0.1663 |
| IgE | 0.9069 | 0.57 | 0.0783 |

### Eosinophil subset

The levels of 16 biomarkers in blood samples from patients with low and high eosinophil levels were measured. The biomarker with the greatest significant difference between the low and high subgroups is EDN with a p value of <0.0001 and an AUC of 0.76 (95% Cl 0.70-0.83, p value <0.0001). Additional biomarkers with a positive correlation are RNASE3, Lactoferrin, IgE and MBP and with negative correlations are CRP, MPO and PCT as determined by the AUC values above or below 0.5.

| | | **Eosinophil <300** | | **Eosinophil >300** | | **t test** | | **ROC** | |
|---|---|---|---|---|---|---|---|---|---|
| **Biomarker** | Unit | Median | IQR | Median | IQR | p value | AUC | 95 Cl | sig |
| **HNE** | ng/ml | 12.7 | 6.5-19.4 | 9.4 | 5.3-18.8 | 0.423346882 | 0.43 | 0.35-0.51 | 0.093421706 |
| **RNASE3** | ng/ml | 4.9 | 2.0-10.1 | 6.6 | 3.3-30.0 | **0.00384342** | **0.60** | **0.52-0.68** | **0.013102969** |
| **Lactoferrin** | ng/ml | 18.2 | 5.6-34.7 | 24.8 | 13.5-39.1 | **0.029402338** | **0.58** | 0.50-0.68 | 0.053373548 |
| **Calprotectin** | ng/ml | 18717 | 7993-40000 | 20657 | 8250-40000 | 0.96860856 | 0.50 | 0.4-0.58 | 0.93538545 |
| **EDN** | ng/ml | 12.9 | 9.0-19.1 | 25.2 | 13.9-35.4 | **1.48929E-10** | **0.76** | **0.70-0.83** | **9.97967E-11** |
| **LTB4** | pg/ml | 53.5 | 10.7-112.0 | 76.6 | 28.8-126.7 | 0.621528688 | **0.67** | 0.49-0.64 | 0.099324444 |
| **MBP** | ng/ml | 1689 | 1024-2653 | 1910 | 1247-2876 | **0.040618482** | 0.54 | 0.47-0.62 | 0.290654269 |
| **SuPAR** | ng/ml | 2146 | 1717-2541 | 2105 | 1635-2446 | 0.757370831 | 0.47 | 0.39-0.55 | 0.489599202 |
| **CRP** | ng/ml | 13527 | 5091-35845 | 7432 | 1794-13823 | **0.00102223** | **0.34** | **0.27-0.41** | **4.60655E-05** |
| **MMP8** | pg/ml | 18822 | 9918-41198 | 15658 | 9997-28049 | 0.176637158 | 0.44 | 0.36-0.51 | 0.113273563 |
| **MMP9** | ng/ml | 631 | 347.5-1218 | 499.5 | 327.3-803.5 | 0.112433516 | 0.43 | 0.35-0.50 | 0.072128963 |
| **MPO** | pg/ml | 89457 | 59500-127266 | 69462 | 46198-102904 | **0.005925688** | **0.38** | **0.30-0.46** | **0.002862553** |
| **NGAL** | ng/ml | 310.2 | 226.7-417.9 | 326 | 220.6-463.9 | 0.80965567 | 0.53 | 0.45-0.61 | 0.465047524 |
| **PCT** | ng/ml | 25.5 | 4.9-73.3 | 20.1 | 3.0-60.8 | **0.044973392** | 0.45 | 0.37-0.53 | 0.227843769 |
| **A1AT** | mg/ml | 18.8 | 11.6-1237821 | 20.8 | 10.6-1195161 | 0.487902417 | 0.50 | 0.42-0.58 | 0.986769514 |
| **IgE** | ng/ml | 321.3 | 0-738.8 | 491.5 | 0-1242 | **0.001351863** | **0.57** | 0.49-0.66 | 0.063536059 |

The findings may be summarised as shown below.

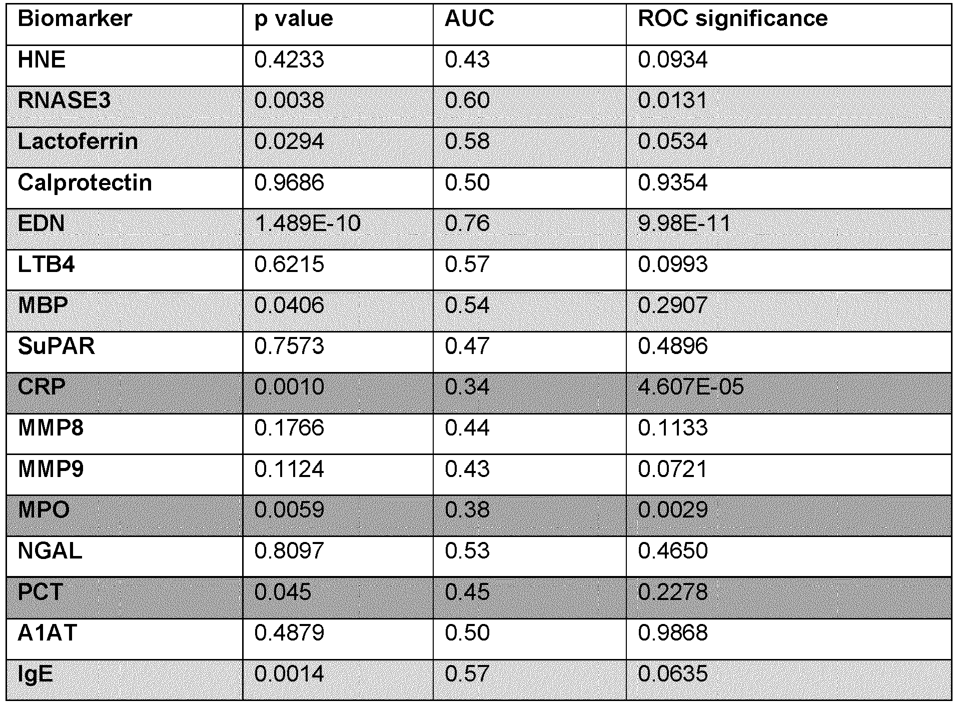

### Neural network analysis

A stepwise multilayer perceptron back propagation algorithm was applied to the data in order to determine the optimal panel of markers for inclusion into the final classification algorithm (Lancashire et al, 2010 A validated gene expression profile for detecting clinical outcome in breast cancer using artificial neural networks. Breast cancer research and treatment, 120 (1), pp. 83-93; and US patent US8,788,444.

To prevent overtraining of the model, the Monte-Carlo cross validation method was used as follows: the sample set was randomly divided into three groups: the training set, validation set and test set in a 60:20:20 ratio, respectively. The training set was used to train the model, the validation sets were used to stop training and prevent overfitting, while the test set was used to assess the accuracy of the trained model. After the model was trained once, the dataset was repeatedly divided again into the training, test and validation set, and this re-division process occurred 50 times, with new splits being randomly generated each time.

### Stability analysis

For both neutrophil and eosinophil biomarkers: 80 independent Artificial Neural Network cycles (loops) were performed each producing a diagnostic model to identify the most stable combination of features. The greater number of times the biomarkers appear in the top five the more stable the biomarkers are.

| **Neutrophil biomarker** | **Number of loops where biomarker in the top 5 hits** | **Stability %** | **Eosinophil biomarkers** | **Number of loops where biomarker in the top 5 hits** | **Stability %** |
|---|---|---|---|---|---|
| MMP9 | 80 | 100 | EDN | 80 | 100 |
| LTB4 | 48 | 60 | MPO | 66 | 83 |
| MBP | 39 | 49 | SuPAR | 41 | 51 |
| EDN | 37 | 46 | RNASE3 | 30 | 38 |
| A1AT | 28 | 35 | HNE | 26 | 33 |
| SuPAR | 26 | 33 | Calprotectin | 23 | 29 |
| HNE | 23 | 29 | A1AT | 20 | 25 |
| MMP8 | 18 | 23 | PCT | 18 | 23 |
| CRP | 15 | 19 | MMP9 | 17 | 21 |
| RNASE3 | 15 | 19 | CRP | 15 | 19 |
| Lactoferrin | 14 | 18 | MBP | 14 | 18 |
| Calprotectin | 14 | 18 | LTB4 | 10 | 13 |
| NGAL | 14 | 18 | Lactoferrin | 8 | 10 |
| MPO | 10 | 13 | MMP8 | 5 | 6 |
| PCT | 10 | 13 | IgE | 4 | 5 |
| IgE | 7 | 9 | NGAL | 2 | 3 |

Based on the stability analysis, the top five biomarkers of the neutrophil and eosinophil subset were carried forward for further optimisation (denoted as neutrophil model 1 and eosinophil model 1). In addition, alternative models exploring markers further down the list of stability were taken forward (denoted as neutrophil models 2-4 and eosinophil models 2 and 3).

### Neutrophil panel

| **Model 1** | **Model 2** | **Model 3** | **Model 4** |
|---|---|---|---|
| MMP9 | MMP9 | MMP9 | MMP9 |
| LTB4 | LTB4 | LTB4 | LTB4 |
| EDN | EDN | EDN | EDN |
| A1AT | A1AT | A1AT | A1AT |
| MBP | SuPAR | HNE | CRP |

### Eosinophil panel

| **Model 1** | **Model 2** | **Model 3** |
|---|---|---|
| EDN | EDN | EDN |
| MPO | MPO | MPO |
| RNASE3 | RNASE3 | RNASE3 |
| HNE | HNE | SuPAR |
| SuPAR | Calprotectin | Calprotectin |

### B. Preliminary model selection

Not all combinations of biomarkers were successful after further analysis and recreation of the models. For the neutrophil panel, models 2 and 4 were taken forward for optimisation and for the eosinophil panel, model 1 was taken forward for further optimisation. Criteria included an AUC>0.8, R²>0.3, sensitivity and specificity >70% (cut-off 0.5).

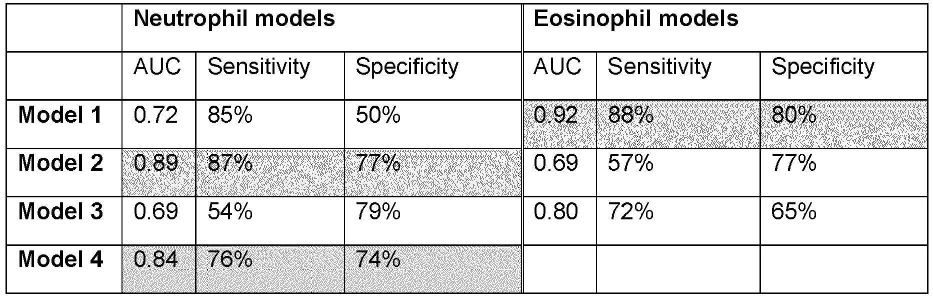

### C. Optimisation of models

Multiple architectures of increasing complexity were assessed for performance. Once the most stable combination of features was identified further analysis was performed to recreate that model. The number of hidden nodes were optimised to maximise the predictive performance. Proprietary software developed in OpenCL, R python and Neurosolutions software was used to develop the algorithm.

### Performance

The performance was assessed using the ROC curve and AUCs. In general, an AUC of 0.5 suggests no discrimination (i.e., ability to diagnose patients with and without the disease or condition based on the test), 0.7 to 0.8 is considered acceptable, 0.8 to 0.9 is considered excellent, and more than 0.9 is considered outstanding.

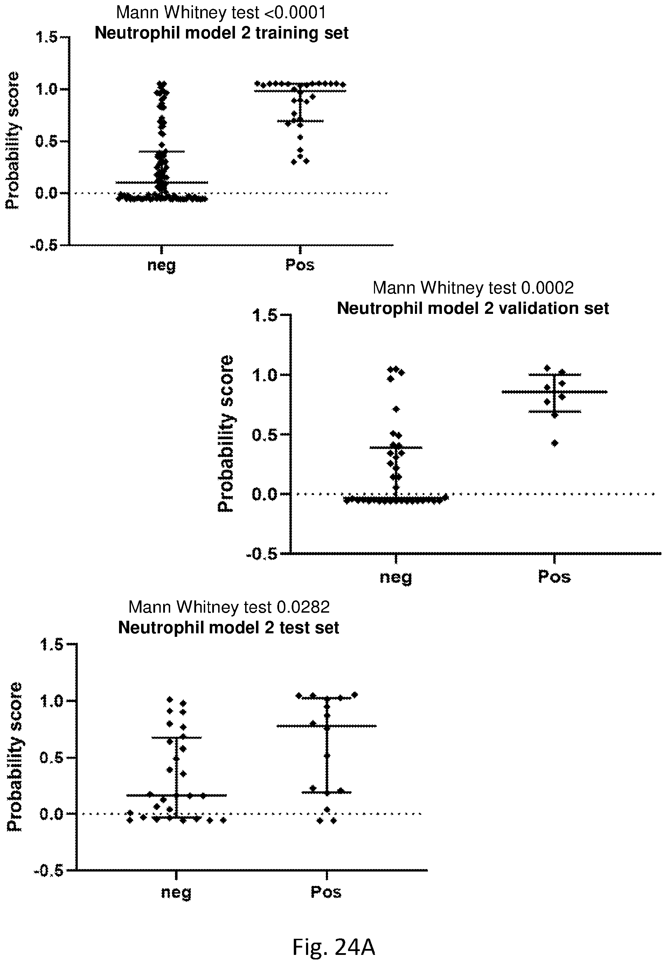

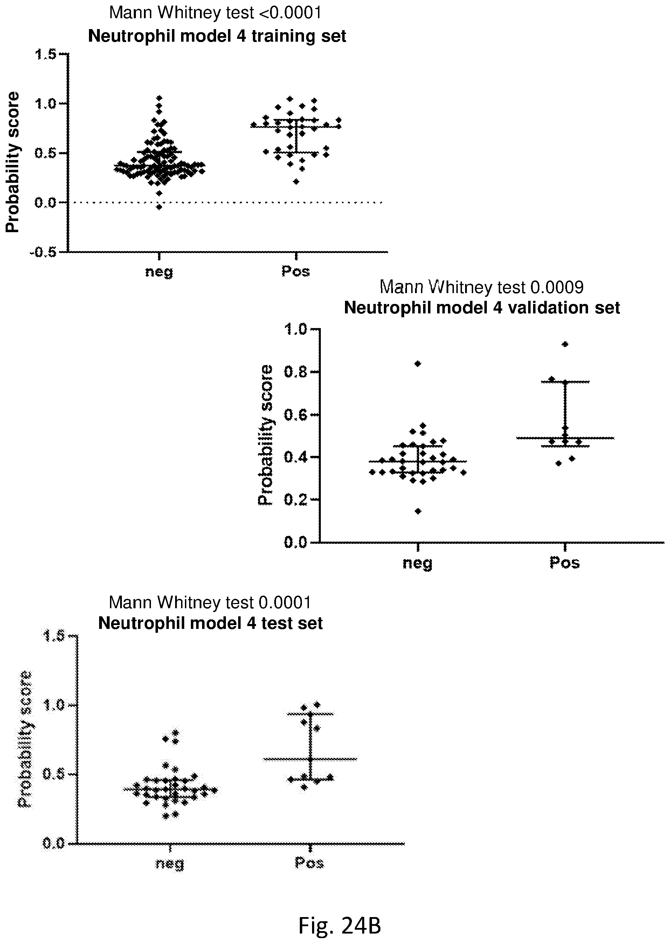

Two neutrophil models (model 2 and model 4) were developed, the results from the training, validation and test data are shown in Figure 24.

Model 2 consists of 5 biomarkers: Matrix metalloproteinases 9 (MMP9), Eosinophil-derived neurotoxin (EDN), Soluble urokinase-type plasminogen activator receptor (SuPAR), Leukotriene B4 (LTB4) and Alpha-1 antitrypsin (A1AT). A significant p value <0.05 was obtained for all three data sets. The AUC for the training and validation sets was 0.9 and for the test set was 0.7. The combined AUC value was 0.84 (95% Cl 0.78-0.90) p <0.0001. At a cut off of 0.5 the sensitivity and specificity were 80% and 77%, respectively.

Model 4 consists of 5 biomarkers: Matrix metalloproteinases 9 (MMP9), C-reactive protein (CRP), EDN, A1AT and LTB4. A significant p value <0.05 was obtained for all three data sets. The AUC for the training and validation sets was 0.84 and for the test set was 0.87. The combined AUC value was 0.84 (95% Cl 0.78-0.90) p <0.0001. At a cut off of 0.4718 the sensitivity and specificity were 80% and 76%, respectively.

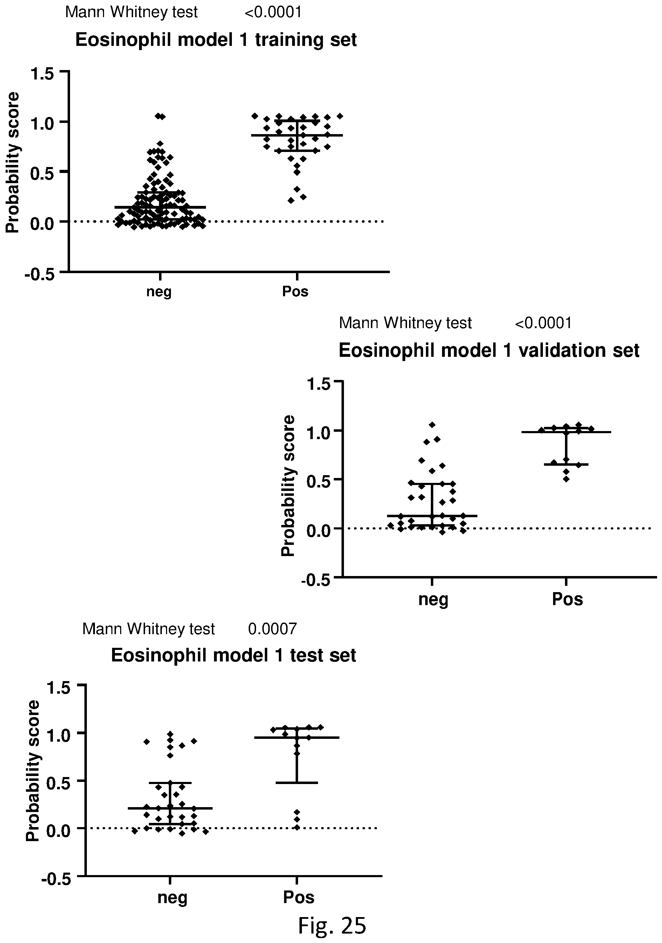

One eosinophil model (model 1) was developed, the results from the training, validation and test data are shown in Figure 25.

Model 1 consists of 5 biomarkers: EDN, Eosinophil cationic protein (RNASE3), SuPAR, Human neutrophil elastase (HNE) and Myeloperoxidase (MPO). A significant p value <0.05 was obtained for all three data sets. The AUC for the training and validation sets was 0.94 and 0.92, respectively, and for the test set was 0.81. The combined AUC value was 0.9 (95% Cl 0.86-0.95) p <0.0001. At a cut off of 0.4843 the sensitivity and specificity were 90% and 84%, respectively.

### Conclusion

The two neutrophil models and the eosinophil models how excellent performance.

### D. Comparison of the performance of individual biomarkers and the optimised neutrophil and eosinophil models

The performance of individual biomarkers from neutrophil models 2 and 4 and eosinophil model 1 was compared to the overall performance of neutrophil models 2 and 4 and eosinophil model 1 using the AUCs values.

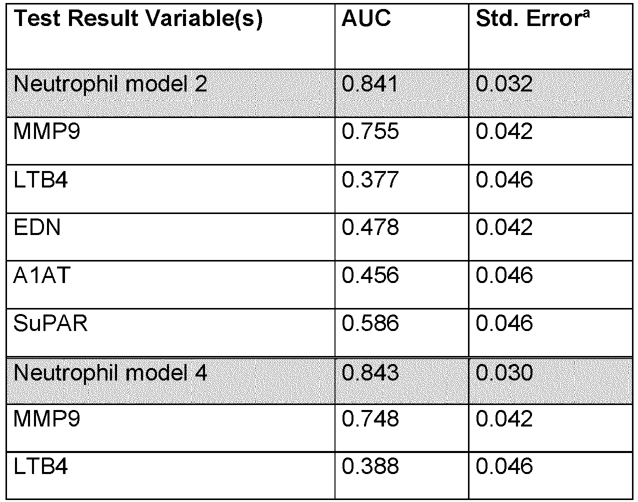

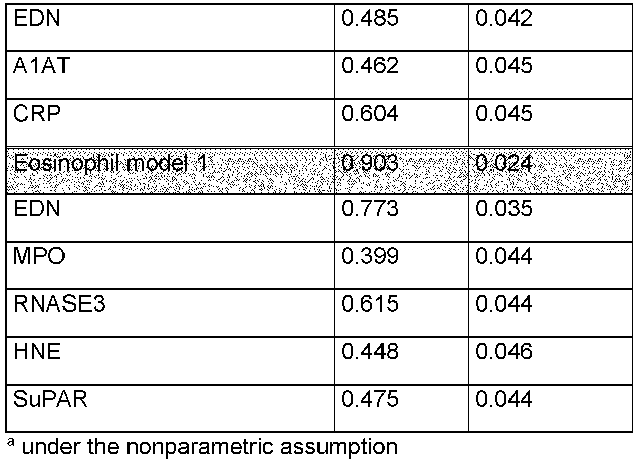

The overall performance of a panel of biomarkers is considerably better than the performance of individual biomarkers. The results demonstrate that the performance is enhanced by the combination of the biomarkers.

The combination of stability assays and further optimisation experiments shows that an advantageous core set of biomarkers for the neutrophil panel includes MMP9 and EDN, and at least one of: LTB4, CRP, A1AT and SuPAR, whereas an advantageous core set of biomarkers for the eosinophil panel includes EDN, RNASE3 and MPO.

## Claims

1. A method for selecting a treatment to be administered to a patient suffering from an exacerbation of inflammation of a respiratory condition, the method comprising determining the levels of at least 3 markers of eosinophil levels and at least 3 markers of neutrophil levels in a blood sample which has been taken from the patient suffering from an exacerbation of inflammation of a respiratory condition wherein:
(i) perturbed levels of the at least 3 markers of eosinophil levels and no perturbation in the levels of the at least 3 markers of neutrophil levels result in selection of corticosteroids to be administered as the treatment for the exacerbation of inflammation;
(ii) perturbed levels of the at least 3 markers of neutrophil levels and no perturbation in the levels of the at least 3 markers of eosinophil levels result in selection of antibiotics to be administered as the treatment for the exacerbation of inflammation; or
(iii) perturbed levels of the at least 3 markers of eosinophil levels and the at least 3 markers of neutrophil levels result in selection of corticosteroids and antibiotics to be co-administered as the treatment for the exacerbation of inflammation;
wherein determining the levels of the at least 3 markers of eosinophil levels comprises determining the levels of at least Eosinophil-derived neurotoxin (EDN), Myeloperoxidase (MPO) and Eosinophil cationic protein (RNASE3);
and wherein determining the levels of the at least 3 markers of neutrophil levels comprises determining the levels of at least (i) Matrix metallopeptidase 9 (MMP9) and Eosinophil-derived neurotoxin (EDN); and (ii) at least one of leukotriene B4 (LTB4), C-reactive protein (CRP), Soluble urokinase-type plasminogen activator receptor (SuPAR), and/or Alpha-1-antitrypsin (A1AT), preferably LTB4.

2. The method according to claim 1 wherein determining the levels of the at least 3 markers of eosinophil levels further comprises determining the levels of at least 1 or 2 further markers selected from Human neutrophil elastase (HNE), Soluble urokinase-type plasminogen activator receptor (SuPAR), and/or Calprotectin.

3. The method according to claim 1 or 2 wherein determining the levels of the at least 3 markers of eosinophil levels further comprises determining the levels of Soluble urokinase-type plasminogen activator receptor (SuPAR), and HNE.

4. The method according to any one of claims 1 to 3 wherein determining the levels of the at least 3 markers of neutrophil levels comprises determining the levels of at least MMP9, EDN and LTB4.

5. The method according to any one of claims 1 to 4 wherein determining the levels of the at least 3 markers of neutrophil levels further comprises determining the levels of MBP.

6. The method according to any one of claims 1 to 5 wherein determining the levels of the at least 3 markers of neutrophil levels further comprises determining the levels of at least 1 or 2 further (different) marker(s) selected from CRP, SuPAR, A1AT and/or LTB4.

7. The method according to any one of claims 1 to 6 wherein the levels of at least 5 markers of eosinophil levels and at least 5 markers of neutrophil levels are determined in the blood sample,
wherein the markers of eosinophil levels are preferably EDN, RNASE3, SuPAR, HNE and MPO;
and wherein the markers of neutrophil levels are preferably
(i) MMP9 and EDN; and
(ii) at least 3 markers selected from LTB4, A1AT, SuPAR and/or CRP;
for example wherein the markers of neutrophil levels are MMP9, EDN, SuPAR, LTB4 and A1AT; or MMP9, C-reactive protein (CRP), EDN, A1AT and LTB4; or MMP9, EDN, CRP, SuPAR and A1AT.

8. A method for selecting corticosteroids to be administered as a treatment to a patient suffering from an exacerbation of inflammation of a respiratory condition, the method comprising determining the levels of at least 3 markers of eosinophil levels in a blood sample which has been taken from the patient suffering from an exacerbation of inflammation of a respiratory condition, wherein perturbed levels of the at least 3 markers of eosinophil levels results in selection of corticosteroids to be administered as the treatment for the exacerbation of inflammation of a respiratory condition,
wherein determining the levels of the at least 3 markers of eosinophil levels comprises determining the levels of EDN, MPO and RNASE3; optionally wherein determining the levels of the at least 3 markers of eosinophil levels further comprises determining the levels of HNE, SuPAR, and/or Calprotectin, preferably HNE and SuPAR.

9. A method for selecting antibiotics to be administered as a treatment to a patient suffering from an exacerbation of inflammation of a respiratory condition, the method comprising determining the levels of at least 3 markers of neutrophil levels in a blood sample which has been taken from the patient suffering from an exacerbation of inflammation of a respiratory condition wherein perturbed levels of at least 3 markers of neutrophil levels results in selection of antibiotics to be administered as the treatment for the exacerbation of inflammation,
wherein determining the levels of the at least 3 markers of neutrophil levels comprises determining the levels of at least (i) Matrix metallopeptidase 9 (MMP9) and Eosinophil-derived neurotoxin (EDN); and (ii) at least one of leukotriene B4 (LTB4), C-reactive protein (CRP), Soluble urokinase-type plasminogen activator receptor (SuPAR), and/or Alpha-1-antitrypsin (A1AT), preferably LTB4, optionally at least 2 or 3 of these markers.

10. A method for selecting and monitoring treatment of a patient suffering from an exacerbation of inflammation of a respiratory condition, the method comprising:
(i) selecting a treatment to be administered to the patient using a method as defined in any one of claims 1-9; and
(ii) with respect to the at least 3 markers for which levels were perturbed when determining the treatment to be administered of step (i), determining the levels of said at least 3 markers in a further blood sample which has been taken from the patient at a later time point wherein:
(a) perturbed levels of the at least 3 markers in the further sample indicate that the treatment should continue or be altered; or
(b) a return to non-perturbed levels of the at least 3 markers in the further sample indicate or predict successful treatment of the exacerbation of inflammation;

11. A system or test kit for selecting a treatment to be administered to a patient suffering from an exacerbation of inflammation of a respiratory condition, comprising:
a. one or more testing devices for determining the levels of at least 3 markers of eosinophil levels and at least 3 markers of neutrophil levels in a blood sample which has been taken from the patient suffering from an exacerbation of inflammation of a respiratory condition;
b. a processor; and
c. a storage medium comprising a computer application program that, when executed by the processor, is configured to:
i. Access and/or calculate the determined levels of the at least 3 markers of eosinophil levels and the at least 3 markers of neutrophil levels in a blood sample on the one or more testing devices;
ii. Calculate whether there is a perturbed level of the at least 3 markers of eosinophil levels and the at least 3 markers of neutrophil levels in the blood sample; and
iii. Output from the processor the treatment to be administered to the patient suffering from an exacerbation of inflammation, wherein:
perturbed levels of the at least 3 markers of eosinophil levels and no perturbation in the levels of the at least 3 markers of neutrophil levels result in selection of corticosteroids to be administered as the treatment for the exacerbation of inflammation; or
perturbed levels of the at least 3 markers of neutrophil levels and no perturbation in the levels of the at least 3 markers of eosinophil levels result in selection of antibiotics to be administered as the treatment for the exacerbation of inflammation; or
perturbed levels of the at least 3 markers of eosinophil levels and the at least 3 markers of neutrophil levels result in selection of corticosteroids and antibiotics to be co-administered as the treatment for the exacerbation of inflammation;
wherein the at least 3 markers of eosinophil levels comprise at least EDN, MPO and RNASE3;
and wherein the at least 3 markers of neutrophil levels comprise at least (i) MMP9 and EDN; and (ii) at least one of leukotriene B4 (LTB4), C-reactive protein (CRP), Soluble urokinase-type plasminogen activator receptor (SuPAR), and/or Alpha-1-antitrypsin (A1AT), preferably LTB4.

12. A system or test kit for selecting corticosteroids to be administered as a treatment to a patient suffering from an exacerbation of inflammation of a respiratory condition, comprising:
a. one or more testing devices for determining the levels of at least 3 markers of eosinophil levels in a blood sample which has been taken from the patient suffering from an exacerbation of inflammation of a respiratory condition;
b. a processor; and
c. a storage medium comprising a computer application program that, when executed by the processor, is configured to:
i. Access and/or calculate the determined levels of the at least one marker of eosinophil levels in a blood sample on the one or more testing devices;
ii. Calculate whether there is a perturbed level of the at least one marker of eosinophil levels in the blood sample; and
iii. Output from the processor that corticosteroids are selected to be administered as the treatment for the exacerbation of inflammation if there is a perturbed level of the at least one marker of eosinophil levels in the blood sample,
wherein the at least 3 markers of eosinophil levels comprise at least Eosinophil-derived neurotoxin (EDN), Myeloperoxidase (MPO) and Eosinophil cationic protein (RNASE3), and optionally the levels of HNE, SuPAR and/or Calprotecin are also determined.

13. A system or test kit for selecting antibiotics to be administered as a treatment to a patient suffering from an exacerbation of inflammation, comprising:
a. one or more testing devices for determining the levels of at least 3 markers of neutrophil levels in a blood sample which has been taken from the patient suffering from an exacerbation of inflammation;
b. a processor; and
c. a storage medium comprising a computer application program that, when executed by the processor, is configured to:
i. Access and/or calculate the determined levels of the at least one marker of neutrophil levels in a blood sample on the one or more testing devices;
ii. Calculate whether there is a perturbed level of the at least one marker of neutrophil levels in the blood sample; and
iii. Output from the processor that antibiotics are selected to be administered as the treatment for the exacerbation of inflammation if there is a perturbed level of the at least one marker of neutrophil levels in the blood sample;
wherein the at least 3 markers of neutrophil levels comprise at least (i) MMP9 and EDN;
and (ii) at least one of leukotriene B4 (LTB4), C-reactive protein (CRP), Soluble urokinase-type plasminogen activator receptor (SuPAR), and/or Alpha-1-antitrypsin (A1AT), preferably LTB4.

14. The system or test kit according to any one of claims 11-13 further comprising a display for the output from the processor and/or wherein the one or more testing devices are disposable single use devices and/or wherein the one or more testing devices comprise lateral flow test strips, optionally comprising a lateral flow test strip for each marker that is determined.

15. A method for selecting initial treatment of a patient suffering from an exacerbation of inflammation of a respiratory condition as defined in any one of claims 1-9, or a method for selecting and monitoring initial treatment of a patient suffering from an exacerbation of inflammation of a respiratory condition as defined in claim 10, said method comprising
(i) using antibodies to detect one or more or all of the markers;
(ii) using a lateral flow assay to detect one or more or all of the markers; and/or
(iii) using a system or test kit as defined in one of claims 11-14.

16. The method according to any one of claims 1-10 or 15 or the system or test kit according to any one of claims 11-15 wherein:
(i) the treatment will be the first treatment to be administered to the patient suffering from an exacerbation of inflammation; and/or
(iii) the subject is suffering from a respiratory disorder, optionally wherein the respiratory disorder is chronic obstructive pulmonary disease (COPD), cystic fibrosis (CF) or asthma, preferably COPD.

17. A computer application program as defined in any one of claims 11-15.

## Patentansprüche

1. Verfahren zum Auswählen einer Behandlung zur Verabreichung an einen Patienten, der an einer Verschlimmerung einer Entzündung der Atemwege leidet, wobei das Verfahren die Bestimmung der Spiegel von mindestens 3 Markern für Eosinophilenspiegel und mindestens 3 Markern für Neutrophilenspiegel in einer Blutprobe umfasst, die dem Patienten entnommen wurde, der an einer Verschlimmerung einer Entzündung der Atemwege leidet, wobei:
(i) gestörte Spiegel der mindestens 3 Marker für Eosinophilenspiegel und keine Störung in den Spiegeln der mindestens 3 Marker für Neutrophilenspiegel zur Auswahl von Kortikosteroiden führen, die als Behandlung für die Verschlimmerung der Entzündung zu verabreichen sind;
(ii) gestörte Spiegel der mindestens 3 Marker für Neutrophilenspiegel und keine Störung der Spiegel der mindestens 3 Marker für Eosinophilenspiegel zur Auswahl von Antibiotika führen, die als Behandlung für die Verschlimmerung der Entzündung zu verabreichen sind; oder
(iii) gestörte Spiegel der mindestens 3 Marker für Eosinophilenspiegel und der mindestens 3 Marker für Neutrophilenspiegel zur Auswahl von Kortikosteroiden und Antibiotika führen, die als die Behandlung für die Verschlimmerung der Entzündung gemeinsam zu verabreichen sind;
wobei die Bestimmung der Spiegel der mindestens 3 Marker für Eosinophilenspiegel die Bestimmung der Spiegel von mindestens eosinophil-abgeleitetem Neurotoxin (EDN), Myeloperoxidase (MPO) und eosinophilem kationischem Protein (RNASE3) umfasst;
und wobei die Bestimmung der Spiegel der mindestens 3 Marker für Neutrophilenspiegel die Bestimmung der Spiegel von mindestens (i) Matrix-Metallopeptidase 9 (MMP9) und eosinophil-abgeleitetem Neurotoxin (EDN); und (ii) mindestens einem von Leukotrien B4 (LTB4), C-reaktivem Protein (CRP), löslichem Urokinase-Typ-Plasminogen-Aktivator-Rezeptor (SuPAR) und/oder Alpha-1-Antitrypsin (A1AT), vorzugsweise LTB4, umfasst.

2. Verfahren nach Anspruch 1, bei dem das Bestimmen der Spiegel der mindestens 3 Marker für Eosinophilenspiegel ferner das Bestimmen der Spiegel von mindestens 1 oder 2 weiteren Markern umfasst, die unter humaner neutrophiler Elastase (HNE), löslichem Urokinase-Typ-Plasminogen-Aktivator-Rezeptor (SuPAR) und/oder Calprotectin ausgewählt sind.

3. Verfahren nach Anspruch 1 oder 2, bei dem das Bestimmen der Spiegel der mindestens drei Marker für Eosinophilenspiegel ferner das Bestimmen der Spiegel des löslichen Urokinase-Typ-Plasminogen-Aktivator-Rezeptors (SuPAR) und von HNE umfasst.

4. Verfahren nach einem der Ansprüche 1 bis 3, bei dem das Bestimmen der Spiegel der mindestens 3 Marker für Neutrophilenspiegel das Bestimmen der Spiegel von mindestens MMP9, EDN und LTB4 umfasst.

5. Verfahren nach einem der Ansprüche 1 bis 4, bei dem das Bestimmen der Spiegel der mindestens 3 Marker für Neutrophilenspiegel ferner das Bestimmen der Spiegel von MBP umfasst.

6. Verfahren nach einem der Ansprüche 1 bis 5, bei dem das Bestimmen der Spiegel der mindestens 3 Marker für die Neutrophilenspiegel ferner das Bestimmen der Spiegel von mindestens 1 oder 2 weiteren (unterschiedlichen) unter CRP, SuPAR, A1AT und/oder LTB4 ausgewählten Markern umfasst.

7. Verfahren nach einem der Ansprüche 1 bis 6, bei dem die Spiegel von mindestens 5 Markern für Eosinophilen-Spiegel und von mindestens 5 Markern für Neutrophilenspiegel in der Blutprobe bestimmt werden,
wobei die Marker für Eosinophilenspiegel vorzugsweise EDN, RNASE3, SuPAR, HNE und MPO sind;
und wobei die Marker für Neutrophilenspiegel vorzugsweise sind:
(i) MMP9 und EDN; und
(ii) mindestens 3 Marker, ausgewählt unter LTB4, A1AT, SuPAR und/oder CRP;
wobei die Marker für Neutrophilenspiegel beispielsweise MMP9, EDN, SuPAR, LTB4 und A1AT; oder MMP9, C-reaktives Protein (CRP), EDN, A1AT und LTB4; oder MMP9, EDN, CRP, SuPAR und A1AT sind.

8. Verfahren zum Auswählen von Kortikosteroiden zur Verabreichung als Behandlung an einen Patienten, der an einer Verschlimmerung einer Entzündung der Atemwege leidet, wobei das Verfahren die Bestimmung der Spiegel von mindestens 3 Markern für Eosinophilenspiegel in einer Blutprobe umfasst, die dem Patienten, der an einer Verschlimmerung einer Entzündung der Atemwege leidet, entnommen wurde,
wobei gestörte Spiegel der mindestens 3 Marker für Eosinophilenspiegel zur Auswahl von als Behandlung für die Verschlimmerung der Entzündung der Atemwege zu verabreichenden Kortikosteroiden führen,
wobei das Bestimmen der Spiegel der mindestens 3 Marker für Eosinophilenspiegel das Bestimmen der Spiegel von EDN, MPO und RNASE3 umfasst; optional, wobei das Bestimmen der Spiegel der mindestens 3 Marker für Eosinophilenspiegel ferner das Bestimmen der Spiegel von HNE, SuPAR und/oder Calprotectin, vorzugsweise von HNE und SuPAR, umfasst.

9. Verfahren zum Auswählen von Antibiotika zur Verabreichung als Behandlung an einen Patienten, der an einer Verschlimmerung einer Entzündung der Atemwege leidet, wobei das Verfahren das Bestimmen der Spiegel von mindestens 3 Markern für Neutrophilenspiegel in einer Blutprobe umfasst, die dem Patienten, der an einer Verschlimmerung einer Entzündung einer Atemwegserkrankung leidet, entnommen wurde,
wobei gestörte Spiegel der mindestens 3 Marker für Neutrophilenspiegel zu einer Auswahl von als Behandlung für die Verschlimmerung der Entzündung zu verabreichenden Antibiotika führen,
wobei das Bestimmen der Spiegel der mindestens 3 Marker für Neutrophilenspiegel das Bestimmen der Spiegel von mindestens (i) Matrix-Metallopeptidase 9 (MMP9) und eosinophil-abgeleitetem Neurotoxin (EDN) umfasst; und (ii) mindestens eines von Leukotrien B4 (LTB4), C-reaktivem Protein (CRP), löslichem Urokinase-Typ-Plasminogenaktivator-Rezeptor (SuPAR) und/oder Alpha-1-Antitrypsin (A1AT), vorzugsweise LTB4, optional mindestens 2 oder 3 dieser Marker, umfasst.

10. Verfahren zum Auswählen und Überwachen der Behandlung eines Patienten, der an einer Verschlimmerung einer Entzündung der Atemwege leidet, wobei das Verfahren umfasst:
(i) Auswählen einer dem Patienten zu verabreichenden Behandlung unter Verwendung eines Verfahrens, wie in einem der Ansprüche 1-9 definiert; und
(ii) Bestimmen der Spiegel der mindestens drei Marker, deren Spiegel bei der Bestimmung der zu verabreichenden Behandlung aus Schritt (i) gestört waren, in einer weiteren, dem Patienten zu einem späteren Zeitpunkt entnommenen Blutprobe, wobei
(a) gestörte Spiegel der mindestens 3 Marker in der weiteren Probe anzeigen, dass die Behandlung fortgesetzt oder geändert werden sollte; oder
(b) eine Rückkehr zu nicht gestörten Spiegeln der mindestens 3 Marker in der weiteren Probe eine erfolgreiche Behandlung der Verschlimmerung der Entzündung anzeigt oder vorhersagt.

11. System oder Testkit zur Auswahl einer Behandlung zur Verabreichung an einen Patienten, der an einer Verschlimmerung einer Entzündung der Atemwege leidet, umfassend:
a. eine oder mehrere Testvorrichtungen zum Bestimmen der Spiegel von mindestens 3 Markern für Eosinophilenspiegel und mindestens 3 Markern für Neutrophilenspiegel in einer Blutprobe, die dem Patienten, der an einer Verschlimmerung der Entzündung der Atemwege leidet, entnommen wurde;
b. einen Prozessor; und
c. ein Speichermedium, das ein Computeranwendungsprogramm umfasst, das, wenn es von dem Prozessor ausgeführt wird, konfiguriert ist zum:
i. Zugreifen auf und/ oder Berechnen der ermittelten Werte der mindestens 3 Marker für Eosinophilenspiegel und der mindestens 3 Marker für Neutrophilenspiegel in einer Blutprobe auf dem einen oder mehreren Testgeräten;
ii. Berechnen, ob eine gestörte Konzentration der mindestens 3 Marker für Eosinophilenspiegel und der mindestens 3 Marker für Neutrophilenspiegel in der Blutprobe vorliegt; und
iii. Ausgeben der dem an einer Verschlimmerung der Entzündung leidenden Patienten zu verabreichenden Behandlung durch den Prozessor, wobei:
gestörte Spiegel der mindestens 3 Marker für Eosinophilenspiegel und keine Störung in den Spiegeln der mindestens 3 Marker für Neutrophilenspiegel zur Auswahl von Kortikosteroiden als für die Verschlimmerung der Entzündung zu verabreichende Behandlung führen, die sind; oder
gestörte Spiegel der mindestens 3 Marker für Neutrophilenspiegel und keine Störung in den Spiegeln der mindestens 3 Marker für Eosinophilenspiegel zur Auswahl von Antibiotika als für die Verschlimmerung der Entzündung zu verabreichende Behandlung führen; oder
gestörte Spiegel der mindestens 3 Marker für Eosinophilenspiegel und der mindestens 3 Marker für Neutrophilenspiegel zur Auswahl von Kortikosteroiden und Antibiotika als für die Verschlimmerung der Entzündung zu verabreichende Behandlung führen;
wobei die mindestens 3 Marker für Eosinophilenspiegel mindestens EDN, MPO und RNASE3 umfassen;
und wobei die mindestens 3 Marker für Neutrophilenspiegel mindestens (i) MMP9 und EDN umfassen; und (ii) mindestens eines von Leukotrien B4 (LTB4), C-reaktivem Protein (CRP), löslichem Urokinase-Typ-Plasminogenaktivator-Rezeptor (SuPAR) und/oder Alpha-1-Antitrypsin (A1AT), vorzugsweise LTB4 umfassen.

12. System oder Testkit zur Auswahl von Kortikosteroiden zur Verabreichung als Behandlung an einen Patienten, der an einer Verschlimmerung einer Entzündung der Atemwege leidet, umfassend:
a. ein oder mehrere Testgeräte zur Bestimmung der Spiegel von mindestens 3 Markern für Eosinophilenspiegel in einer Blutprobe, die dem Patienten, der an einer Verschlimmerung der Entzündung der Atemwege leidet, entnommen wurde;
b. einen Prozessor; und
c. ein Speichermedium, das ein Computeranwendungsprogramm umfasst, das, wenn es von dem Prozessor ausgeführt wird, konfiguriert ist zum:
i. Zugreifen auf und/ oder Berechnen der ermittelten Werte des mindestens einen Markers für Eosinophilenspiegel in einer Blutprobe auf den ein oder mehreren Testgeräten;
ii. Berechnen, ob ein gestörter Spiegel des mindestens einen Markers für Eosinophilenspiegel in der Blutprobe vorhanden ist; und
iii. Ausgeben, durch den Prozessor, dass Kortikosteroide ausgewählt werden, um als Behandlung für die Verschlimmerung der Entzündung verabreicht zu werden, wenn ein gestörter Spiegel des mindestens einen Markers für Eosinophilenspiegel in der Blutprobe vorhanden ist,
wobei die mindestens 3 Marker für Eosinophilenspiegel mindestens eosinophil-abgeleitetes Neurotoxin (EDN), Myeloperoxidase (MPO) und eosinophiles kationisches Protein (RNASE3) umfassen, und optional auch die Spiegel von HNE, SuPAR und/oder Calprotecin bestimmt werden.

13. System oder Testkit zur Auswahl von Antibiotika zur Verabreichung als Behandlung an einen Patienten, der an einer Verschlimmerung einer Entzündung leidet, umfassend:
a. ein oder mehrere Testgeräte zur Bestimmung der Spiegel von mindestens 3 Markern für Neutrophilenspiegel in einer Blutprobe, die dem Patienten, der an einer Verschlimmerung der Entzündung leidet, entnommen wurde;
b. einen Prozessor; und
c. ein Speichermedium, das ein Computeranwendungsprogramm umfasst, das, wenn es von dem Prozessor ausgeführt wird, konfiguriert ist zum:
i. Zugreifen auf und/ oder Berechnen der ermittelten Werte des mindestens einen Markers für Neutrophilenspiegel in einer Blutprobe auf dem einen oder mehreren Testgeräten;
ii. Berechnen, ob ein gestörter Spiegel des mindestens einen Markers für Neutrophilenspiegel in der Blutprobe vorhanden ist; und
iii. Ausgeben, durch den Prozessor, dass Antibiotika ausgewählt werden, um als Behandlung für die Verschlimmerung der Entzündung verabreicht zu werden, wenn ein gestörter Spiegel des mindestens einen Markers der Neutrophilenspiegel in der Blutprobe vorhanden ist;
wobei die mindestens 3 Marker für Neutrophilenspiegel mindestens (i) MMP9 und EDN; und (ii) mindestens eines von Leukotrien B4 (LTB4), C-reaktivem Protein (CRP), löslichem Urokinase-Typ-Plasminogenaktivator-Rezeptor (SuPAR) und/oder Alpha-1-Antitrypsin (A1AT), vorzugsweise LTB4 umfassen.

14. System oder Testkit nach einem der Ansprüche 11 bis 13, ferner umfassend eine Anzeige für die Ausgabe des Prozessors und/oder bei dem die ein oder mehreren Testgeräte Einweggeräte zum einmaligen Gebrauch sind und/oder bei dem die ein oder mehreren Testgeräte Lateral-Flow-Teststreifen umfassen, optional umfassend einen Lateral-Flow-Teststreifen für jeden bestimmten Marker.

15. Verfahren zum Auswählen der Erstbehandlung eines Patienten, der an einer Verschlimmerung einer Entzündung der Atemwege leidet, gemäß einem der Ansprüche 1 bis 9, oder Verfahren zum Auswählen und Überwachen der Erstbehandlung eines Patienten, der an einer Verschlimmerung einer Entzündung der Atemwege leidet, gemäß Anspruch 10, wobei das Verfahren umfasst:
(i) Verwenden von Antikörpern zum Nachweis eines oder mehrerer oder aller Marker;
(ii) Verwenden eines Lateral-Flow-Assays zum Nachweis eines oder mehrerer oder aller der Marker; und/oder
(iii) Verwenden eines Systems oder Testkits, wie in einem der Ansprüche 11-14 definiert.

16. Verfahren nach einem der Ansprüche 1-10 oder 15 oder System oder Testkit nach einem der Ansprüche 11-15, bei dem:
(i) die Behandlung die erste Behandlung ist, die dem Patienten verabreicht wird, der an einer Verschlimmerung der Entzündung leidet; und/oder
(iii) der Patient an einer Atemwegserkrankung leidet, wobei optional es sich bei der Atemwegserkrankung um eine chronisch obstruktive Lungenerkrankung (COPD), zystische Fibrose (CF) oder Asthma, vorzugsweise COPD, handelt.

17. Computeranwendungsprogramm nach einem der Ansprüche 11-15.

## Revendications

1. Procédé de sélection d'un traitement à administrer à un patient souffrant d'une exacerbation de l'inflammation d'une affection respiratoire, le procédé comprenant l'étape consistant à déterminer les niveaux d'au moins 3 marqueurs de niveaux d'éosinophiles et d'au moins 3 marqueurs de niveaux de neutrophiles dans un échantillon de sang qui a été prélevé sur le patient souffrant d'une exacerbation de l'inflammation d'une affection respiratoire dans lequel :
(i) des niveaux perturbés des au moins 3 marqueurs de niveaux d'éosinophiles et aucune perturbation des niveaux des au moins 3 marqueurs de niveaux de neutrophiles entraînent la sélection de corticostéroïdes pour être administrés comme traitement pour l'exacerbation de l'inflammation ;
(ii) des niveaux perturbés des au moins 3 marqueurs de niveaux de neutrophiles et aucune perturbation des niveaux des au moins 3 marqueurs de niveaux d'éosinophiles entraînent la sélection d'antibiotiques pour être administrés comme traitement pour l'exacerbation de l'inflammation ; ou
(iii) des niveaux perturbés des au moins 3 marqueurs de niveaux d'éosinophiles et des au moins 3 marqueurs de niveaux de neutrophiles entraînent la sélection de corticostéroïdes et d'antibiotiques pour être co-administrés comme traitement pour l'exacerbation de l'inflammation ;
dans lequel la détermination des niveaux des au moins 3 marqueurs de niveaux d'éosinophiles comprend la détermination des niveaux d'au moins la neurotoxine dérivée des éosinophiles (EDN), la myéloperoxydase (MPO) et la protéine cationique des éosinophiles (RNASE3) ;
et dans lequel la détermination des niveaux des au moins 3 marqueurs de niveaux de neutrophiles comprend la détermination des niveaux d'au moins (i) la métallopeptidase matricielle 9 (MMP9) et la neurotoxine dérivée des éosinophiles (EDN) ; et (ii) d'au moins l'un(e) du leucotriène B4 (LTB4), de la protéine C-réactive (CRP), du récepteur de l'activateur du plasminogène de type urokinase soluble (SuPAR) et/ou de l'alpha-1-antitrypsine (A1AT), de préférence LTB4.

2. Procédé selon la revendication 1, dans lequel la détermination des niveaux des au moins 3 marqueurs de niveaux d'éosinophiles comprend en outre la détermination des niveaux d'au moins 1 ou 2 marqueur(s) supplémentaire(s) choisi(s) entre l'élastase des neutrophiles humains (HNE), le récepteur de l'activateur du plasminogène de type urokinase soluble (SuPAR) et/ou la calprotectine.

3. Procédé selon la revendication 1 ou 2, dans lequel la détermination des niveaux des au moins 3 marqueurs de niveaux d'éosinophiles comprend en outre la détermination des niveaux de récepteur de l'activateur du plasminogène de type urokinase soluble (SuPAR) et de HNE.

4. Procédé selon l'une quelconque des revendications 1 à 3, dans lequel la détermination des niveaux des au moins 3 marqueurs de niveaux de neutrophiles comprend la détermination des niveaux d'au moins MMP9, EDN et LTB4.

5. Procédé selon l'une quelconque des revendications 1 à 4, dans lequel la détermination des niveaux des au moins 3 marqueurs de niveaux de neutrophiles comprend en outre la détermination des niveaux de MBP.

6. Procédé selon l'une quelconque des revendications 1 à 5, dans lequel la détermination des niveaux des au moins 3 marqueurs de niveaux de neutrophiles comprend en outre la détermination des niveaux d'au moins 1 ou 2 marqueur(s) (différent(s)) supplémentaire(s) choisi(s) entre CRP, SuPAR, A1AT et/ou LTB4.

7. Procédé selon l'une quelconque des revendications 1 à 6 dans lequel les niveaux d'au moins 5 marqueurs de niveaux d'éosinophiles et d'au moins 5 marqueurs de niveaux de neutrophiles sont déterminés dans l'échantillon de sang,
dans lequel les marqueurs de niveaux d'éosinophiles sont de préférence EDN, RNASE3, SuPAR, HNE et MPO ;
et dans lequel les marqueurs de niveaux de neutrophiles sont de préférence
(i) MMP9 et EDN ; et
(ii) au moins 3 marqueurs choisis entre LTB4, A1AT, SuPAR et/ou CRP ;
par exemple dans lequel les marqueurs de niveaux de neutrophiles sont MMP9, EDN, SuPAR, LTB4 et A1AT ; ou MMP9, la protéine C-réactive (CRP), EDN, A1AT et LTB4 ; ou MMP9, EDN, CRP, SuPAR et A1AT.

8. Procédé de sélection de corticostéroïdes à administrer comme traitement à un patient souffrant d'une exacerbation de l'inflammation d'une affection respiratoire, le procédé comprenant l'étape consistant à déterminer les niveaux d'au moins 3 marqueurs de niveaux d'éosinophiles dans un échantillon de sang qui a été prélevé sur le patient souffrant d'une exacerbation de l'inflammation d'une affection respiratoire,
dans lequel des niveaux perturbés des au moins 3 marqueurs de niveaux d'éosinophiles entraînent la sélection de corticostéroïdes pour être administrés comme traitement pour l'exacerbation de l'inflammation d'une affection respiratoire,
dans lequel la détermination des niveaux des au moins 3 marqueurs de niveaux d'éosinophiles comprend la détermination des niveaux d'EDN, de MPO et de RNASE3 ; facultativement dans lequel la détermination des niveaux des au moins 3 marqueurs de niveaux d'éosinophiles comprend en outre la détermination des niveaux de HNE, SuPAR et/ou de la calprotectine, de préférence de HNE et de SuPAR.

9. Procédé de sélection d'antibiotiques à administrer comme traitement à un patient souffrant d'une exacerbation de l'inflammation d'une affection respiratoire, le procédé comprenant l'étape consistant à déterminer les niveaux d'au moins 3 marqueurs de niveaux de neutrophiles dans un échantillon de sang qui a été prélevé sur le patient souffrant d'une exacerbation de l'inflammation d'une affection respiratoire dans lequel des niveaux perturbés d'au moins 3 marqueurs de niveaux de neutrophiles entraînent la sélection d'antibiotiques pour être administrés comme traitement pour l'exacerbation de l'inflammation,
dans lequel la détermination des niveaux des au moins 3 marqueurs de niveaux de neutrophiles comprend la détermination des niveaux d'au moins (i) la métallopeptidase matricielle 9 (MMP9) et la neurotoxine dérivée des éosinophiles (EDN) ; et (ii) d'au moins l'un(e) du leucotriène B4 (LTB4), de la protéine C-réactive (CRP), du récepteur de l'activateur du plasminogène de type urokinase soluble (SuPAR) et/ou de l'alpha-1-antitrypsine (A1AT), de préférence LTB4, facultativement d'au moins 2 ou 3 de ces marqueurs.

10. Procédé de sélection et de surveillance de traitement d'un patient souffrant d'une exacerbation de l'inflammation d'une affection respiratoire, le procédé comprenant les étapes consistant à :
(i) sélectionner un traitement à administrer au patient en utilisant un procédé tel que défini dans l'une quelconque des revendications 1 à 9 ; et
(ii) en ce qui concerne les au moins 3 marqueurs pour lesquels les niveaux ont été perturbés lors de la détermination du traitement à administrer de l'étape (i), déterminer les niveaux desdits au moins 3 marqueurs dans un échantillon de sang supplémentaire qui a été prélevé sur le patient à un moment ultérieur dans lequel :
(a) des niveaux perturbés des au moins 3 marqueurs dans l'échantillon supplémentaire indiquent que le traitement doit continuer ou être modifié ; ou
(b) un retour à des niveaux non perturbés des au moins 3 marqueurs dans l'échantillon supplémentaire indique ou prédit un traitement réussi de l'exacerbation de l'inflammation.

11. Système ou kit de test pour sélectionner un traitement à administrer à un patient souffrant d'une exacerbation de l'inflammation d'une affection respiratoire, comprenant :
a. un ou plusieurs dispositif (s) de test pour déterminer les niveaux d'au moins 3 marqueurs de niveaux d'éosinophiles et d'au moins 3 marqueurs de niveaux de neutrophiles dans un échantillon de sang qui a été prélevé sur le patient souffrant d'une exacerbation de l'inflammation d'une affection respiratoire ;
b. un processeur ; et
c. un support de stockage comprenant un programme d'application informatique qui, lorsqu'il est exécuté par le processeur, est configuré pour :
i. accéder aux et/ou calculer les niveaux déterminés des au moins 3 marqueurs de niveaux d'éosinophiles et des au moins 3 marqueurs de niveaux de neutrophiles dans un échantillon de sang sur le ou les plusieurs dispositif(s) de test ;
ii. calculer s'il existe un niveau perturbé des au moins 3 marqueurs de niveaux d'éosinophiles et des au moins 3 marqueurs de niveaux de neutrophiles dans l'échantillon de sang ; et
iii. délivrer en sortie à partir du processeur le traitement à administrer au patient souffrant d'une exacerbation de l'inflammation, dans lequel :
des niveaux perturbés des au moins 3 marqueurs de niveaux d'éosinophiles et aucune perturbation des niveaux des au moins 3 marqueurs de niveaux de neutrophiles entraînent la sélection de corticostéroïdes pour être administrés comme traitement pour l'exacerbation de l'inflammation ; ou
des niveaux perturbés des au moins 3 marqueurs de niveaux de neutrophiles et aucune perturbation des niveaux des au moins 3 marqueurs de niveaux d'éosinophiles entraînent la sélection d'antibiotiques pour être administrés comme traitement pour l'exacerbation de l'inflammation ; ou
des niveaux perturbés des au moins 3 marqueurs de niveaux d'éosinophiles et des au moins 3 marqueurs de niveaux de neutrophiles entraînent la sélection de corticostéroïdes et d'antibiotiques pour être co-administrés comme traitement pour l'exacerbation de l'inflammation ;
dans lequel les au moins 3 marqueurs de niveaux d'éosinophiles comprennent au moins EDN, MPO et RNASE3 ;
et dans lequel les au moins 3 marqueurs de niveaux de neutrophiles comprennent au moins (i) MMP9 et EDN ; et (ii) au moins l'un(e) du leucotriène B4 (LTB4), de la protéine C-réactive (CRP), du récepteur de l'activateur du plasminogène de type urokinase soluble (SuPAR) et/ou de l'alpha-1-antitrypsine (A1AT), de préférence LTB4.

12. Système ou kit de test pour sélectionner des corticostéroïdes à administrer comme traitement à un patient souffrant d'une exacerbation de l'inflammation d'une affection respiratoire, comprenant :
a. un ou plusieurs dispositif (s) de test pour déterminer les niveaux d'au moins 3 marqueurs de niveaux d'éosinophiles dans un échantillon de sang qui a été prélevé sur le patient souffrant d'une exacerbation de l'inflammation d'une affection respiratoire ;
b. un processeur, et
c. un support de stockage comprenant un programme d'application informatique qui, lorsqu'il est exécuté par le processeur, est configuré pour :
i. accéder aux et/ou calculer les niveaux déterminés de l'au moins un marqueur de niveaux d'éosinophiles dans un échantillon de sang sur le ou les plusieurs dispositif(s) de test ;
ii. calculer s'il existe un niveau perturbé de l'au moins un marqueur de niveaux d'éosinophiles dans l'échantillon de sang ; et
iii. délivrer en sortie à partir du processeur que des corticostéroïdes sont sélectionnés pour être administrés comme traitement pour l'exacerbation de l'inflammation s'il existe un niveau perturbé de l'au moins un marqueur de niveaux d'éosinophiles dans l'échantillon de sang,
dans lequel les au moins 3 marqueurs de niveaux d'éosinophiles comprennent au moins la neurotoxine dérivée des éosinophiles (EDN), la myéloperoxydase (MPO) et la protéine cationique des éosinophiles (RNASE3), et facultativement, les niveaux de HNE, de SuPAR et/ou de la calprotectine sont également déterminés.

13. Système ou kit de test pour sélectionner des antibiotiques à administrer comme traitement à un patient souffrant d'une exacerbation de l'inflammation, comprenant :
a. un ou plusieurs dispositif (s) de test pour déterminer les niveaux d'au moins 3 marqueurs de niveaux de neutrophiles dans un échantillon de sang qui a été prélevé sur le patient souffrant d'une exacerbation de l'inflammation ;
b. un processeur ; et
c. un support de stockage comprenant un programme d'application informatique qui, lorsqu'il est exécuté par le processeur, est configuré pour :
i. accéder aux et/ou calculer les niveaux déterminés de l'au moins un marqueur de niveaux de neutrophiles dans un échantillon de sang sur le ou les plusieurs dispositif(s) de test ;
ii. calculer s'il existe un niveau perturbé de l'au moins un marqueur de niveaux de neutrophiles dans l'échantillon de sang ; et
iii. délivrer en sortie à partir du processeur que des antibiotiques sont sélectionnés pour être administrés comme traitement pour l'exacerbation de l'inflammation s'il existe un niveau perturbé de l'au moins un marqueur de niveaux de neutrophiles dans l'échantillon de sang ;
dans lequel les au moins 3 marqueurs de niveaux de neutrophiles comprennent au moins (i) MMP9 et EDN ; et (ii) au moins l'un(e) du leucotriène B4 (LTB4), de la protéine C-réactive (CRP), du récepteur de l'activateur du plasminogène de type urokinase soluble (SuPAR) et/ou de l'alpha-1-antitrypsine (A1AT), de préférence LTB4.

14. Système ou kit de test selon l'une quelconque des revendications 11 à 13, comprenant en outre un dispositif d'affichage pour la sortie du processeur et/ou dans lequel le ou les plusieurs dispositif(s) de test est/sont un/des dispositif(s) à usage unique jetable(s) et/ou dans lequel le ou les plusieurs dispositif(s) de test comprend/comprennent des bandelettes réactive à flux latéral, comprenant facultativement une bandelette réactive à flux latéral pour chaque marqueur qui est déterminé.

15. Procédé de sélection d'un traitement initial d'un patient souffrant d'une exacerbation de l'inflammation d'une affection respiratoire tel que défini dans l'une quelconque des revendications 1 à 9, ou procédé de sélection et de surveillance d'un traitement initial d'un patient souffrant d'une exacerbation de l'inflammation d'une affection respiratoire tel que défini dans la revendication 10, ledit procédé comprenant les étapes consistant à
(i) utiliser des anticorps pour détecter un ou plusieurs ou tous les marqueur(s) ;
(ii) utiliser un essai à flux latéral pour détecter un ou plusieurs ou tous les marqueur(s) ; et/ou
(iii) utiliser un système ou kit de test tel que défini dans l'une des revendications 11 à 14.

16. Procédé selon l'une quelconque des revendications 1 à 10 ou 15 ou système ou kit de test selon l'une quelconque des revendications 11 à 15, dans lequel :
(i) le traitement sera le premier traitement à administrer au patient souffrant d'une exacerbation de l'inflammation ; et/ou
(iii) le sujet souffre d'un trouble respiratoire, facultativement dans lequel le trouble respiratoire est la bronchopneumopathie chronique obstructive (BPCO), la fibrose kystique (FK) ou l'asthme, de préférence BPCO.

17. Programme d'application informatique tel que défini dans l'une quelconque des revendications 11 à 15.
